(19) **Europäisches Patentamt**
**European Patent Office**
**Office européen des brevets**

(11) **EP 3 131 460 B1**

(12) **EUROPEAN PATENT SPECIFICATION**

(45) Date of publication and mention
of the grant of the patent:
**02.09.2020 Bulletin 2020/36**

(21) Application number: **15780530.0**

(22) Date of filing: **06.01.2015**

(51) Int Cl.:
**A61B 5/026** (2006.01)

(86) International application number:
**PCT/US2015/000001**

(87) International publication number:
**WO 2015/160390 (22.10.2015 Gazette 2015/42)**

(54) **FLOW RATE SENSOR SYSTEM FOR MEASURING THE FLOW RATE OF A BODILY FLUID**

DURCHFLUSSRATENSENSORSYSTEM ZUR MESSUNG DER DURCHFLUSSRATE EINER KÖRPERFLÜSSIGKEIT

SYSTÈME DÉTECTEUR DE MESURE DU DÉBIT D'UN LIQUIDE CORPOREL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.04.2014 US 201414120048**

(43) Date of publication of application:
**22.02.2017 Bulletin 2017/08**

(73) Proprietor: **Vivonics, Inc.**
**Bedford, MA 01730 (US)**

(72) Inventors:
• **GOLDIE, James H.**
**Lexington, MA 02421 (US)**
• **TRUONG, Thieu Q.**
**North Easton, MA 02356 (US)**
• **DUONG, Minh**
**Worcester, MA 01602 (US)**
• **RUSSELL, Thomas**
**New Providence, NJ 07974 (US)**

(74) Representative: **Jones, Graham Henry**
**Graham Jones & Company**
**77 Beaconsfield Road**
**Blackheath, London SE3 7LG (GB)**

(56) References cited:
WO-A1-2012/176194    WO-A1-2014/013062
US-A- 5 346 508    US-A- 5 598 847
US-A1- 2005 204 811    US-A1- 2005 204 811
US-A1- 2005 277 839    US-A1- 2009 143 673
US-A1- 2009 204 019    US-A1- 2011 054 333
US-A1- 2013 226 068    US-A1- 2013 245 404
US-B1- 6 533 733

**Description**

FIELD OF THE INVENTION

This invention relates to a flow rate sensor system for measuring the flow rate of a bodily fluid according to independent claim 1.

BACKGROUND OF THE INVENTION

Drainage of cerebrospinal fluid (CSF) is one major life-sustaining therapy which may be used for patients with congenital or acquired hydrocephalus or patients with serious head injuries. Ventriculo-peritoneal (VP) shunt placement for CSF drainage is a common procedure in neurosurgery. However, shunt failure is common and shunt revision surgery is even more common than initial placement. One study of shunt-related deaths from Jan 1990 to July 1996 found that children are dying of shunt failure and that early detection could prevent many of these deaths. Another study of all shunt procedures performed between January 1996 and December 2005, excluding temporary shunts such as external ventricular drains (EVDs), found that the median shunt survival life span was 398 days. These results are in good agreement yet another study that suggests failure rates of 25 to 40 percent in the first year and failure of seventy percent of shunts by five years. These findings point to the importance of the need for a non-invasive and convenient system and method for monitoring shunt function.

Invasive surgery allows direct observation of the shunt and its flow behavior when it is allowed to drain into a collection vessel. This is different than measuring the flow rate when the shunt is draining into the peritoneum, but it does allow the surgeon to check patency of the shunt and provide an indication of current flow.

The ShuntCheck by NeuroDX Development (Bensalem, PA 19020) has been shown in clinical studies to provide a means for assessing CSF flow through a VP Shunt. The ShuntCheck uses thermal techniques for determining that CSF flow is present in the shunt. The ShuntCheck relies on a disposable temperature sensor placed over the skin proximate the shunt tubing. An ice cube is placed over the shunt and the effect on the temperature of the skin close to the shunt downstream of the ice cube is monitored. The ShuntCheck has the advantage that it is not implanted, but it has the disadvantage that it is unable to provide a quantitative measure of flow rate.

Another conventional quantitative flow measuring device for measuring the flow of CSF in VP shunt tubing uses an implantable device that produces a bubble in the shunt tubing by electrolysis. The bubble is then detected by an electrode arrangement using electric impedance or ultrasonically with a Doppler probe. Extracorporeal high-frequency transmission supplies the energy for electrolysis and flow may be calculated based on the velocity of bubble flow in the tubing.

Another conventional VP shunt pressure sensor determines pressure from deflection of a capacitive membrane. The shunt flow sensor membrane has a vacuum on the side of the membrane that is not in contact with fluid. Thus, the device is measuring pressure relative to vacuum.

Yet another conventional implanted intracranial pressure (ICP) sensor relies on the deflection of a membrane and the resultant change in the resonance of an LC-circuit.

However, none of the devices discussed above have yet to be demonstrated in humans to provide sufficiently reliable and accurate quantitative information on shunt function to be adopted for clinical use.

Current techniques to evaluate implanted shunts may include cranial imaging techniques, such as ultrasonography, computer assisted tomography (CAT), magnetic resonance imaging (MRI), and the like. Such techniques require relatively expensive equipment typically only available in hospitals. Intracranial pressure monitoring is currently available through implanted catheters and transducers, typically in an intensive care unit. Thus, clinicians rely on reports of symptoms from the patient, imaging of the ventricles, or the use of invasive devices to treat diseases related to CSF flow through VP shunts. Additionally, the progression of disease and injuries cannot be studied extensively because of the lack of shunt flow data.

Therefore, there is a need for an implanted sensor system capable of determining and reporting flow rate of a bodily fluid, such as CSF, that can be queried transcutaneously to allow the clinician to noninvasively assess shunt function.

U.S. Patent Publication No. 2009/0204019 A1 to Giuggen et al. discloses a combined pressure and flow rate sensor integrated in a shunt system for implantation into a patient for delivering excess fluid from the lateral ventricles of the patient's brain to a diversion site. The shunt includes a pressure transducer for measuring ICP, a rate flow transducer for measuring flow rate of the CSF flowing within the shunt, and a telemetry circuit. The '019 Patent Application teaches the amount of heat transferred to the heating element is directly correlated to the fluid flow and fluid flow is determined by measuring a temperature gradient across sensors.

SUMMARY OF THE INVENTION

This invention features a flow rate sensor system for measuring the flow rate of a bodily fluid according to independent claim 1. The system includes an encapsulated implant having a flow tube having an inlet and an outlet configured to receive a flow of a bodily fluid. A heating element externally coupled to the flow tube is configured to dissipate heat at a predetermined rate over a predetermined amount of time. A single thermistor externally and directly coupled to the heating element is configured to measure a temperature rise of the heating element over the predetermined amount of time. An implant microcontroller coupled to the single thermistor is configured to determine the flow rate of the bodily fluid in the flow tube from the measured temperature rise of the heating element over the predetermined amount of time and from a curve fit applied to a stored set of previously obtained calibration measurements. An implant power and communication subsystem coupled to the implant microcontroller is configured to wirelessly receive power and wirelessly transmit and receive data.

The system also includes an external device having an external microcontroller and an external power and communication subsystem coupled to the external microcontroller configured to wirelessly deliver power to the implant power and communication subsystem and transmit and receive data to and from the implant power and communication subsystem.

The heating element may include a surface mount resistor. The heating element may include a coil of electrically conductive wire or a printed circuit heater. The heating element may be directly attached to the external surface of the flow tube. The system may include a thermal insulator configured to thermally isolate the heating element and the single thermistor from cooling paths other than the direct cooling path to the bodily fluid in the flow tube. The thermal insulator may include an insulation layer over the heating element and the single thermistor. The thermal insulator may include a sealed volume of air surrounding the heating element and the single thermistor. The flow through flow tube may be comprised of a thin wall of polymer material with low thermal conductivity configured to limit heat transfer along a length and a circumference of the tube while maintaining heat transfer in a radial direction to the fluid. The bodily fluid may include one or more of: cerebrospinal fluid (CSF), bile, blood, and urine. The encapsulated implant may be coupled to a shunt, tube, vessel or catheter implanted in a human body or an animal. The shunt may include one or more of: a ventriculo-peritoneal (VP) shunt, ventroarterial shunt, and lumboperitoneal shunt. The encapsulated implant may be coupled to a distal catheter of the shunt. The encapsulated implant may be coupled to a proximal catheter of the shunt. The heating element and the single thermistor may be located proximate the outlet. The heating element and the single thermistor may be located proximate the inlet. The heating element and the single thermistor may be located between the inlet and the outlet. The external power and communication subsystem includes an external coil coupled to the external microcontroller and the implant power and communication subsystem includes an implant coil coupled to the microcontroller. The implant coil of the encapsulated implant may be located using the magnitude of the induced voltage wirelessly sent from the implant coil to the external coil. The external coil may be positioned proximate and in alignment with the implant coil to achieve sufficient inductive coupling between the external coil and the implant coil. The implant coil may be integrated with the encapsulated implant. The implant coil may be remotely located from and tethered to the encapsulated implant. The external power and communication subsystem may include a resonant circuit comprised of the external coil and a capacitor, and a source of low-level voltage pulses, the external device resonant circuit configured to provide sinusoidal current in the external coil of sufficient amplitude to induce sufficient sinusoidal voltage in the implant coil. The implant power and communication subsystem may include an implant resonant circuit comprised of the implant coil and a capacitor having a resonance frequency closely matched to the resonance frequency of the external resonant circuit to maintain sufficient AC voltage amplitude to power the implant power and communication subsystem and to enable communication between the external power and communication subsystem and implant power and communication subsystem. The implant power and communication subsystem may be configured to convert induced sinusoidal voltages in the implant coil to a highly regulated DC voltage over the range of loading conditions to power the heating element, the temperature sensor, the microcontroller, and components of the implant power and communication subsystem. The external power communication subsystem may be configured to enable the external microcontroller to communicate data to the implant power and communication subsystem by changing the voltage supplied to the resonant circuit of the external power and communication subsystem to modulate the amplitude of the voltage induced in the implant coil and use that change in voltage to represent different binary states. The implant power and communication subsystem may transmit binary values serially to the external power and communication subsystem by sequentially applying and removing an electrical load from the implant coil to induce changes in voltage in the external coil that are decoded into data by the external microcontroller. The external power and communication subsystem may include a sense resistor configured to measure change in the amplitude of the current in external power and communication subsystem resulting from changes in the induced voltage in the external coil. The external microcontroller may be coupled to the series resistor and may be configured to decode changes in the current of the external power and communication subsystem into data. The implant microcontroller may be configured to store the set of previously obtained calibration measurements relating heating element temperature rise to flow rate. The implant microcontroller may be configured to determine the flow rate from the measured temperature rise when temperature of the heating element is determined to be no longer rising to minimize the length of time needed to determine the flow rate, the amount of heat generated by the heating device, and the amount of heat delivered to a patient. The implant microcontroller may be configured to store identification information associated with the encapsulated implant. The implant microcontroller may be configured to use the mean value of a set of temperature rise samples obtained over the predetermined amount of time as the temperature rises to determine the flow rate of the bodily fluid in order to increase the signal to noise ratio. The implant microcontroller may be configured to use a weighted average of a set of temperature rise samples obtained over a predetermined amount of time as the temperature rises to determine the flow rate of the bodily fluid in order to increase the signal to noise ratio. The encapsulated implant may be implanted in a human body. The external device may include a smart device including a flow sensor App and a tethered external coil. The external device may include a display for displaying one or more of: the measured flow rate, the predetermined amount of time, induced voltage on the implant coil, and identification information associated with the encapsulated implant.

In another aspect, a flow rate sensor system for measuring the flow rate of a bodily fluid is featured, according to claim 1.

The system includes an encapsulated implant having a flow tube having an inlet and an outlet configured to receive a flow of a bodily fluid. A heating element externally coupled to the flow tube is configured to dissipate heat at a predetermined rate over a predetermined temperature rise of the heating element. A single thermistor externally and directly coupled to the heating element is configured to measure a temperature drop of the heating element over a predetermined amount of time of cooling. An implant microcontroller coupled to the single thermistor is configured to determine the flow rate of the bodily fluid in the flow tube from the measured temperature drop of the heating element over the predetermined amount of cooling time and from a curve fit applied to a stored set of previously obtained calibration measurements. An implant power and communication subsystem coupled to the implant microcontroller is configured to wirelessly receive power and wirelessly transmit and receive data. The system also includes an external device having an external microcontroller, and an external power and communication subsystem coupled to the external microcontroller configured to wirelessly deliver power to the implant power and communication subsystem and transmit and receive data to and from the implant power and communication subsystem.

In another aspect, a flow rate sensor system for measuring the flow rate of a bodily fluid is featured according to claim 1.

The system includes an encapsulated implant having a heating element externally coupled to a shunt, catheter, tube, or vessel configured to receive a flow of a bodily fluid, the heating element configured to dissipate heat at a predetermined rate over a predetermined amount of time. A single thermistor externally and directly coupled to the heating element is configured to measure a temperature rise of the heating element over the predetermined amount of time. An implant microcontroller coupled to the single thermistor is configured to determine the flow rate of the bodily fluid in the shunt, catheter, tube or vessel from the measured temperature rise of the heating element over the predetermined amount of time and from a curve fit applied to a stored set of previously obtained calibration measurements. An implant power and communication subsystem coupled to the implant microcontroller is configured to wirelessly receive power and wirelessly transmit and receive data. The system also includes an external device having an external microcontroller and an external power and communication subsystem coupled to the external microcontroller configured to wirelessly deliver power to the implant power and communication subsystem and transmit and receive data to and from the implant power and communication subsystem.

In one embodiment, the encapsulated implant may be configured as a two-piece clamp externally coupled to the shunt, catheter, tube, or vessel.

In another aspect, a flow rate sensor system for measuring the flow rate of a bodily fluid is featured according to claim 1.

The system includes an encapsulated implant having a heating element externally coupled to the shunt, catheter, tube, or vessel configured to receive a flow of a bodily fluid, the heating element configured to dissipate heat at a predetermined rate over a predetermined temperature rise of heating element. A single thermistor externally and directly coupled to the heating element is configured to measure a temperature drop of the heating element over a predetermined amount of time of cooling. An implant microcontroller coupled to the single thermistor is configured to determine the flow rate of the bodily fluid in the shunt, catheter, tube or vessel from the measured temperature drop of the heating element over the predetermined amount of cooling time and from a curve fit applied to a stored set of previously obtained calibration measurements. An implant power and communication subsystem coupled to the implant microcontroller is configured to wirelessly receive power and wirelessly transmit and receive data. The system also includes an external device having an external microcontroller and an external power and communication subsystem coupled to the external microcontroller configured to wirelessly deliver power to the implant power and communication subsystem and transmit and receive data to and from the implant power and communication subsystem.

In one embodiment, the encapsulated implant may be configured as a two-piece clamp externally coupled to the shunt, catheter, tube, or vessel.

BRIEF DESCRIPTION OF THE SEVERAL VIEWS OF THE DRAWINGS

Other objects, features and advantages will occur to those skilled in the art from the following description of a preferred embodiment and the accompanying drawings, in which:

Fig. 1 is a perspective side view showing the primary components of one embodiment of the flow rate sensor system thereof for measuring the flow rate of a bodily fluid through a shunt implanted in a patient.

Fig. 2 is a top-view showing in further detail one embodiment of the encapsulated implant shown in Fig. 1;

Fig. 3 is a top-view of the encapsulated implant shown in Fig. 2 without the encapsulant thereon;

Fig. 4 is a schematic end-view showing in further detail one example of the placement of the heating element and temperature sensor about the flow tube shown in Figs. 2 and 3;

Fig. 5 is a graph depicting one example of a stored set of previously obtained calibration measurements showing a relationship between the temperature rise of the heating element and the flow rate of the bodily fluid used by the encapsulated implant 12 shown in one or more of Figs. 1-3;

Fig. 6 is a graph depicting one example of a curve fit to the previously obtained calibration measurements shown in Fig. 5;

Fig. 7 is a graph showing one example of the flow rate measured by the encapsulated implant shown in one or more of Figs. 1-3 compared to the actual flow rate imposed by a syringe pump;

Fig. 8 is a graph depicting another example of a stored set of previously obtained calibration measurements showing a relationship between the temperature drop of a heating element and the flow rate of the bodily fluid used by the encapsulated implant 12 shown in one or more of Figs. 1-3;

Fig. 9 is a graph depicting one example of a curve fit to the previously obtained calibration measurements shown in Fig. 8;

Fig. 10 is a schematic block diagram showing one embodiment of the primary components of the implant power and communication subsystem of the encapsulated implant shown in one or more of Figs. 1-3;

Fig. 11 is a schematic block diagram showing one embodiment of the primary components of the external power and communication subsystem of the external device shown in Fig. 1;

Fig. 12 shows an example of a resistance temperature detector (RTD) which may be used for the temperature sensor shown in at least Figs. 3, 4, and 10;

Fig. 13 shows an example of a thermocouple which may be used for the temperature sensor shown in at least Figs. 3, 4, and 10;

Fig. 14 shows an example of the heating element configured as a coil of electrically conductive wire;

Fig. 15 shows an example of the heating element configured as a printed circuit heater a resistor;

Figs. 16-17 show examples of the heating element configured as a resistor;

Fig. 18 is a schematic end-view showing one example of an insulation layer which may be placed about the heating element and temperature sensor shown in at least Figs. 3, 4, and 10;

Fig. 19 is a schematic end-view showing an example of an insulation layer of sealed air surrounding the heating element and temperature sensor shown in Figs. 3, 4, and 10;

Fig. 20 is a three-dimensional view showing in further detail the insulation layer of sealed air surrounding the heating element and temperature sensor shown in Fig. 19;

Fig. 21 is a front side-view showing one example of the primary components of a VP shunt and various locations of the encapsulated implant on a ventricular catheter or a distal catheter;

Fig. 22 is a side-view showing in further detail one example of the primary components of the flow rate sensor system shown in one or more of Figs. 1-19;

Fig. 23 is a front side-view showing in further detail one example of the alignment of the implant coil shown in at least Figs. 2, 3, and 10 with the external coil of the external power and communication subsystem shown in at least Figs. 1 and 22;

Fig. 24 is a schematic diagram showing one example of the implant coil separately located from the encapsulated implant; and

Fig. 25 is a three-dimensional front-view showing the primary components of another embodiment of the flow rate sensor system for measuring the flow rate of a bodily fluid through a shunt catheter, tube or vessel.

DETAILED DESCRIPTION OF THE INVENTION

Aside from the preferred embodiment or embodiments disclosed below, this invention is capable of other embodiments and of being practiced or being carried out in various ways. Thus, it is to be understood that the invention is not limited in its application to the details of construction and the arrangements of components set forth in the following description or illustrated in the drawings. If only one embodiment is described herein, the claims hereof are not to be limited to that embodiment.

The invention is defined solely by the appended claims.

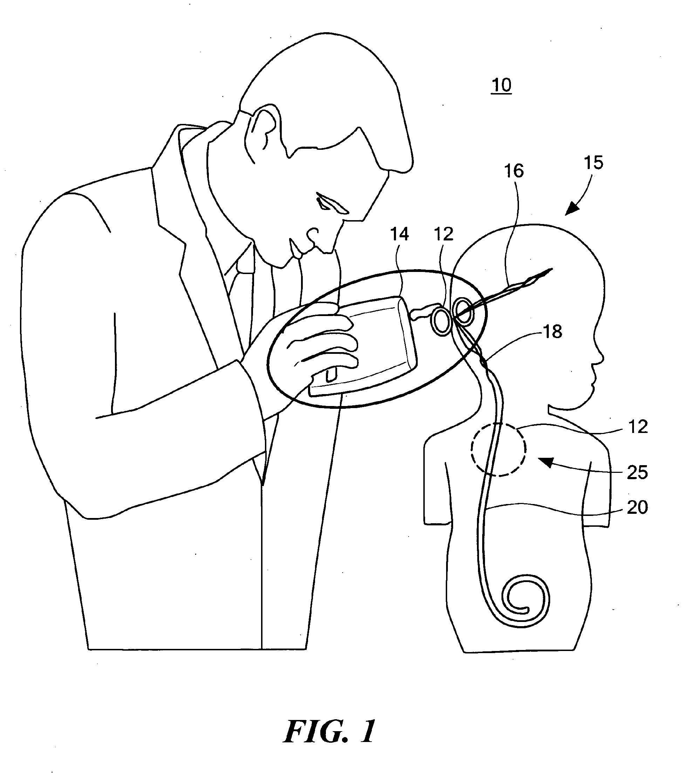

There is shown in Fig. 1 one embodiment of system 10 for measuring the flow rate of a bodily fluid. System 10 includes encapsulated implant 12 and external device 14. In the example shown in Fig. 1, encapsulated implant 12 is implanted into human body 15 and is coupled to ventricular or proximal catheter 16 of VP shunt 18. In other examples, encapsulated implant 12 (shown in phantom) may be coupled to distal catheter 20 of VP shunt 18, indicated at 25. However, encapsulated implant 12 need not necessarily be coupled in-line to VP shunt 18 as shown and may be externally coupled over any type catheter, shunt, tube, vessel and the like, which is implanted in the human body or the body of an animal and has a flow of bodily fluid there through, as discussed in further detail below.

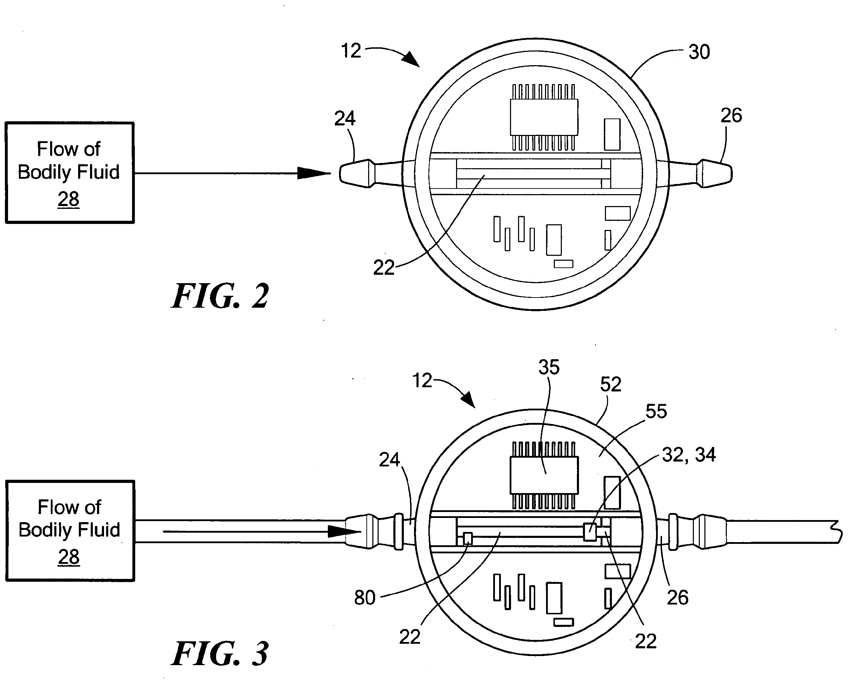

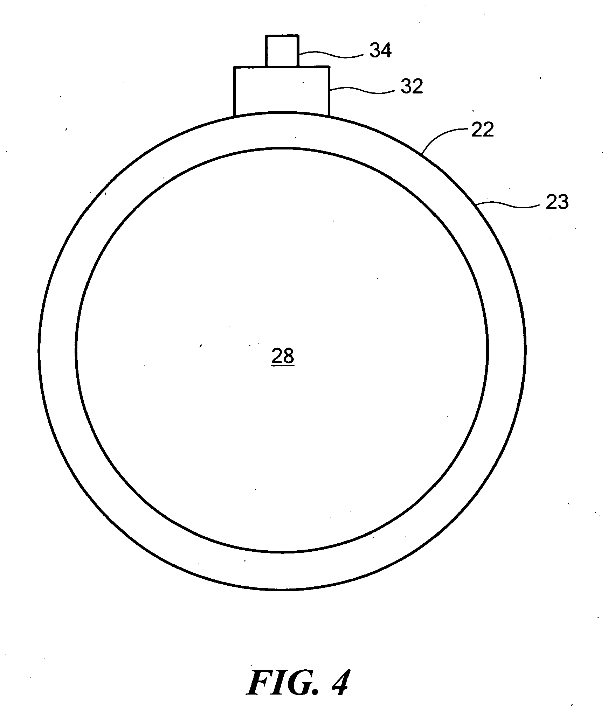

In this example, encapsulated implant 12, Fig. 2, includes flow tube 22 having inlet 24 and outlet 26 configured to receive flow of bodily fluid 28. Flow of bodily fluid 28 may include CSF, blood, bile, urine, or other bodily fluid. Encapsulated implant 12 includes encapsulant 30, e.g., a medical grade polyurethane such as Steralloy FDF 2380 (Hapco, Inc. Hanover, MA 02339), silicone, or other biocompatible materials. Encapsulated implant 12, Fig. 3, where like parts have been given like numbers, shown without encapsulant 30 for clarity, also includes heating element 32 externally coupled to flow tube 22 and temperature sensor 34 externally coupled to heating element 32. Fig. 4 shows in further detail one example of heating element 32 directly and externally coupled to external surface 23 of flow tube 22 and temperature sensor 34 externally coupled to heating element 32. In one embodiment, heating element 32, Figs. 3 and 4, is configured to dissipate heat at a predetermined rate over a predetermined amount of time. In this example, temperature sensor 34 is configured to measure the temperature rise of heating element 32 over a predetermined amount of time, e.g., 10 seconds, 20 seconds, 30 seconds, and the like, as discussed in further detail below.

Encapsulated implant 12, Fig. 3, also includes implant microcontroller 35, preferably coupled to printed circuit board (PCB) 55, configured to determine the flow rate of flow of bodily fluid 28 in flow tube 22 from the temperature rise of heating element 32 over the predetermined amount of time and a curve fit to a stored set of previously obtained calibration measurements. The stored set of previously obtained calibration measurements include measurements of the temperature rises of same heating element 32 and associated imposed flow rates over the same predetermined amount of time and level of heat dissipation. The store set of previously obtained calibration measurements are preferably stored by implant microcontroller 35.

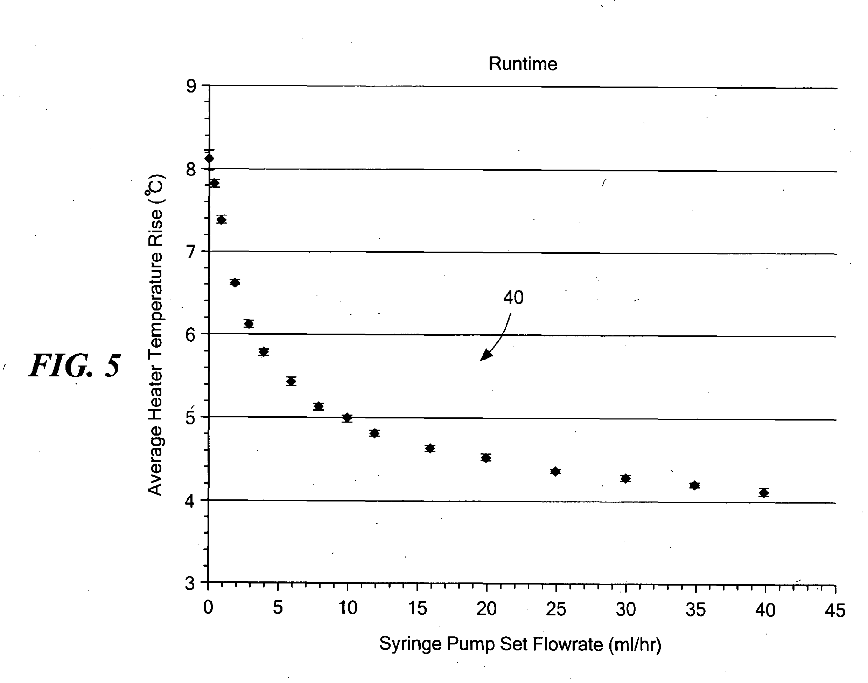

In one example, the stored set of previously obtained calibration measurements shown by data points 40, Fig. 5, may be created by a user request using external device 14, Fig. 1. The stored set of previously obtained measurements may be created by applying a regulated DC voltage to heating element 32, Figs. 3 and 4, that produces a repeatable heat dissipation level from the heating element 32 each time the regulated DC voltage is applied, discussed in further detail below. The resulting heat created within heating element 32 causes a rise in temperature of the heating element 32 over the same predetermined amount of time, e.g., 5, 10, 15, 20, 30, or 40 seconds, or similar time intervals, as that used for the flow rate measurement. The rise in temperature of heating element 32 is measured by temperature sensor 34 over the predetermined amount of time, while heating element 32 is turned on and dissipating heat. In order to accurately obtain the temperature rise of temperature sensor 34 and heating element 32, temperature measurements are recorded for a few seconds prior to turning on heating element 32. The average of these temperature measurements may serve as a baseline temperature that can be subtracted from the temperature measurements taken after heating element 32 is turned on.

To create the stored set of previously obtained calibration measurements, the resulting temperature rise over the predetermined amount of time is preferably matched to the imposed flow rate, and two values are stored in the memory of the implant microcontroller 35 as the stored set of calibration measurements. In one design, rather than store only one value of temperature rise for calibration, multiple values of temperature rise versus flow rate over the predetermined amount time and rate of heat dissipation may be stored.

In one example, a pump capable of accurately delivering a known desired flow rate, such as a well-calibrated syringe pump may be used to create the stored calibrated measurement. The stored set of calibrated flow rate measurements may be obtained in this manner at each of multiple flow rate settings over the known range of feasible bodily fluid flow rates through flow tube 22, e.g., from about 0 to about 40 mL/hr.

The number of calibration values for the stored set of calibrated flow rate measurements is preferably sufficient to characterize a curve of the rise in temperature of heating element 32 as a function of flow rate, e.g., data points 40, Fig. 5. The heat dissipation from heating element 32, Figs. 3 and 4, need not be precisely known, but is preferably repeatable each time heating element 32 and encapsulated implant 12, Figs. 1-3, is activated.

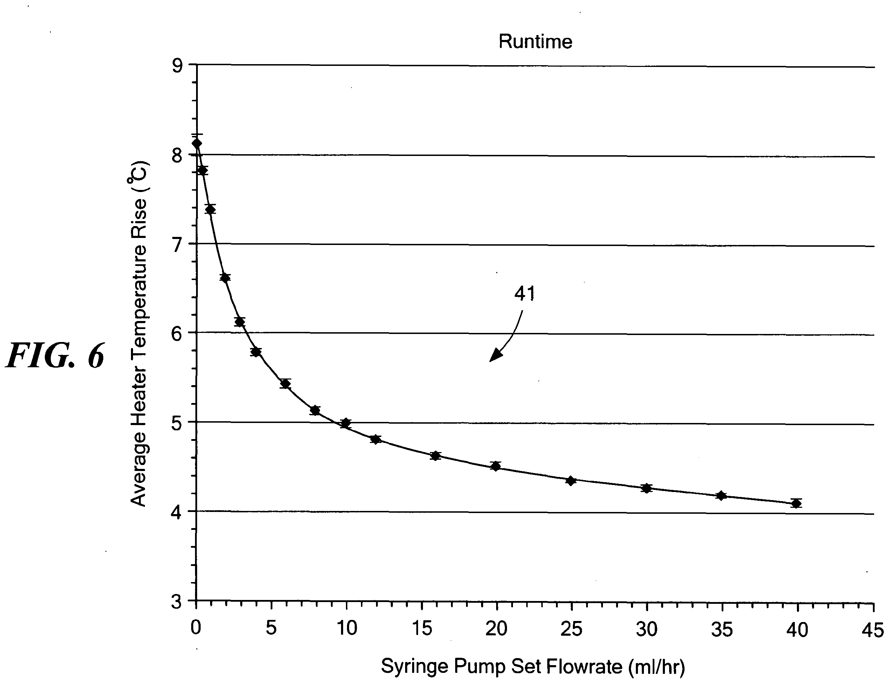

In operation, a regulated DC voltage is applied to heating element 32 and the temperature rise of heating element 32 is sensed by temperature sensor 34 and recorded by implant microcontroller 35. Curve fitting is preferably applied to the stored set preferably previously obtained calibration measurements of temperature rise versus flow rate to derive a continuous relationship between measured temperature rise and flow rate, as shown by curve 41, Fig. 6. From curve 41 and the measured temperature rise, the flow rate of flow of bodily fluid 28, Fig. 3, through flow tube 22 over the duration of the measurement is inferred.

Curve fitting is a well understood process of creating a curve or a continuous mathematical function that closely fits a series of data points. For determination of flow rate, curve fitting can involve either interpolation between calibration data points of measured temperature rises versus flow rates, or the determination of a smooth mathematical function that fits all the data points 40, Fig. 5, to a good approximation. Curve 41, Fig. 6, shows one example of curve fitting in which linear extrapolation is employed between each of the adjacent data points 40 shown in Fig. 5.

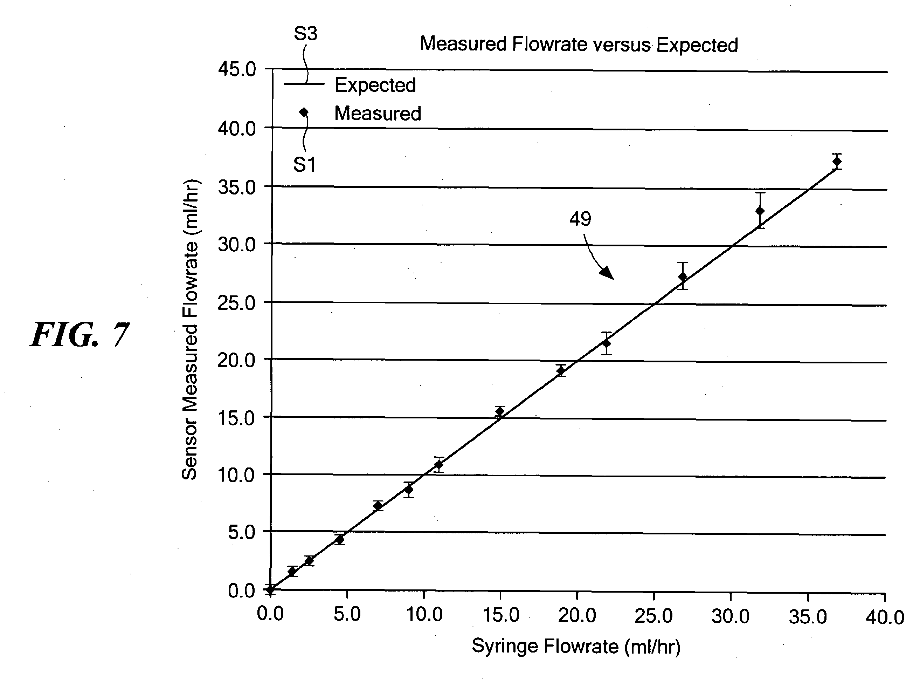

Plot 49, Fig. 7, shows one example of the flow rates measured by encapsulated implant 12, indicated at 51, compared to actual known flow rates imposed by a calibrated syringe pump, indicated at 53. As can be seen, encapsulated implant 12, Figs. 1-3, accurately determined the flow rate of flow of bodily fluid 28 in flow tube 22.

If the implant microcontroller 35 determines that the temperature of heating element 32 is no longer rising (i.e. that steady state has been reach), then implant microcontroller 35 can terminate the measurement since it has already acquired a sufficient number of temperature values from temperature sensor 34 to determine the flow rate. This can reduce the predetermined amount of time needed to determine the flow rate of flow of bodily fluid 28, e.g., to between about 5 to 10 seconds and minimize the amount of heat needed to be generated by heating element 32.

The dependence of the temperature rise of heating element 32 over the duration of a flow rate measurement by encapsulated implant 12 rate arises from the flow rate of flow of bodily fluid 28, Fig. 2, and the heat transfer coefficient for heat transfer from heating element 32 into flow of bodily fluid 28 inside tube 22 in accordance with formula:

$$h = Q/(A\Delta T) \hspace{4cm} (1)$$

where Q is the heat dissipated by the heating element 32, A is the area of heat transfer, and $\Delta T$ is the difference in temperatures between heating element 32 and bodily fluid 28 flowing in tube 22. The heat transfer coefficient h increases monotonically with flow rate, at least over the range of CSF flow rates possible within a shunt. Therefore, since the level of heat dissipation Q produced by the heating element is fixed for a given applied DC voltage, the temperature rise of heating element 32 during a flow rate measurement will decrease with increasing CSF flow rate.

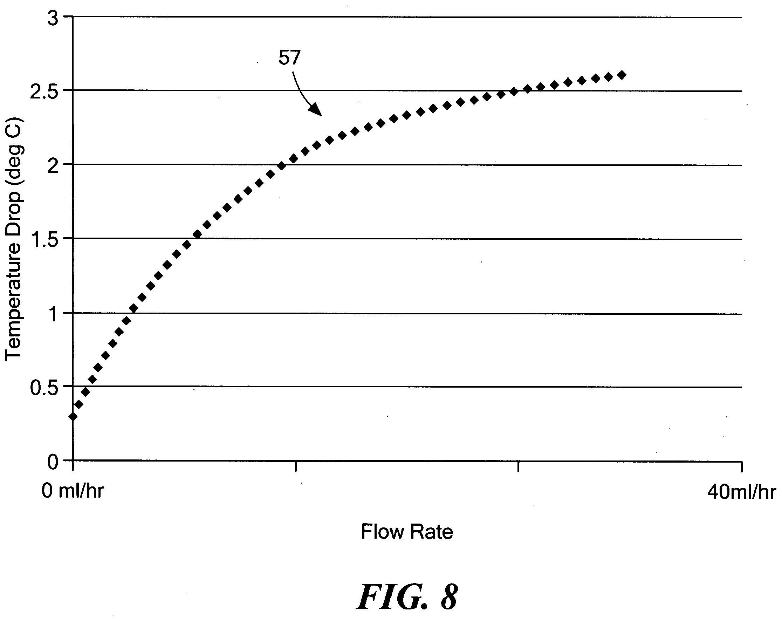

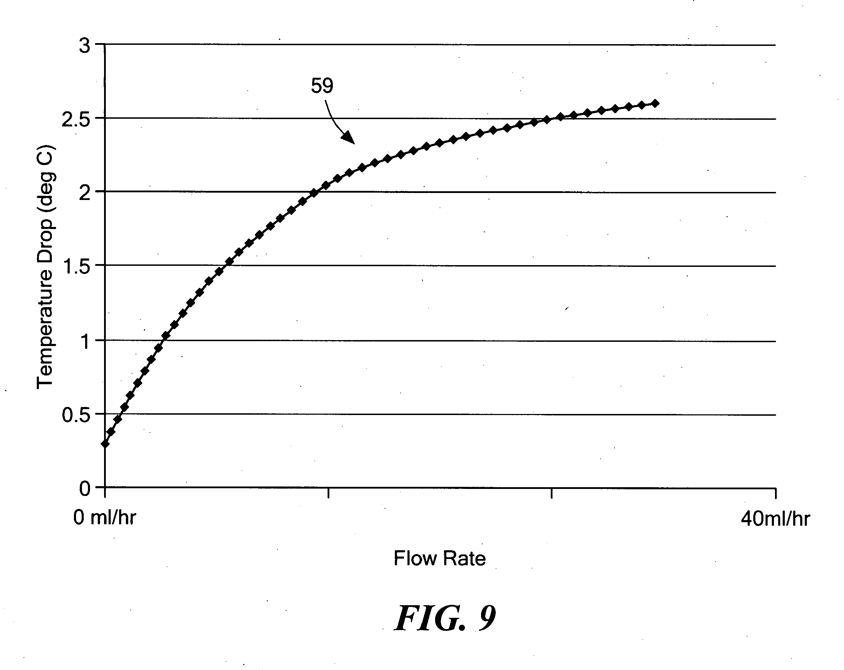

Flow rate sensor system 10, shown in one or more of Figs. 1-4, may also be used to determine flow rate by measuring the temperature drop of heating element 32 after it is allowed to cool for a predetermined amount of time, e.g., 5, 10, 20, 30 seconds, and the like, after the heating element 32 has been previously heated until a predetermined temperature rise is achieved, e.g., 2, 3, 4 °C, and the like. The measured temperature drop is then compared with a set of previously obtained calibration measurements in which temperature drops were measured and stored after heating element 32 was first heated to the same temperature rise and then turned off for the same predetermined amount of time, while precisely known flow rates were imposed. Similarly, as discussed above with reference to Figs. 5 and 6, a curve fit or interpolation may be used to estimate flow rate from the stored set of previously obtained calibration measurements. For example, a flow rate may be determined by first turning on heating element 32 and allowing it to continue to warm up until implant microcontroller 35, Fig. 3, detects a predetermined rise in temperature has been achieved, e.g., about 3°C. At this point heating element 32 is turned off and allowed to cool for a predetermined period of time e.g., 5, 10, 15 seconds, and the like. During the cooling of heating element 32, the temperature drop of heating element 32 over a predetermined amount of time will depend on the flow rate of bodily fluid 28 in tube 22, since the rate of cooling of heating element 32 depends on the flow rate of bodily fluid 28. In particular, the rate of cooling will increase as the flow rate of bodily fluid 28 increases, and, therefore, the temperature drop of heating element 32 over a predetermined amount of time of cooling will also increase as flow rate of bodily fluid 28 increases. In this example, implant microcontroller 35 is configured to determine the flow rate of the bodily fluid in the flow tube from the measured temperature drop of the heating element 32 and curve fit to a stored set of previously obtained calibration measurements having a relationship between temperature drop of heating element and flow rate of bodily fluid established similar as described above. In this example, data points 57, Fig. 8, is utilized instead of data points 40, Fig. 5 described above. Curve-fitting or interpolation is preferably applied to the set of previously obtained calibration measurements shown in Fig. 8 in order to infer the flow rate through tube 22 over the duration of the measurement, e.g. as shown by curve 59, Fig. 9.

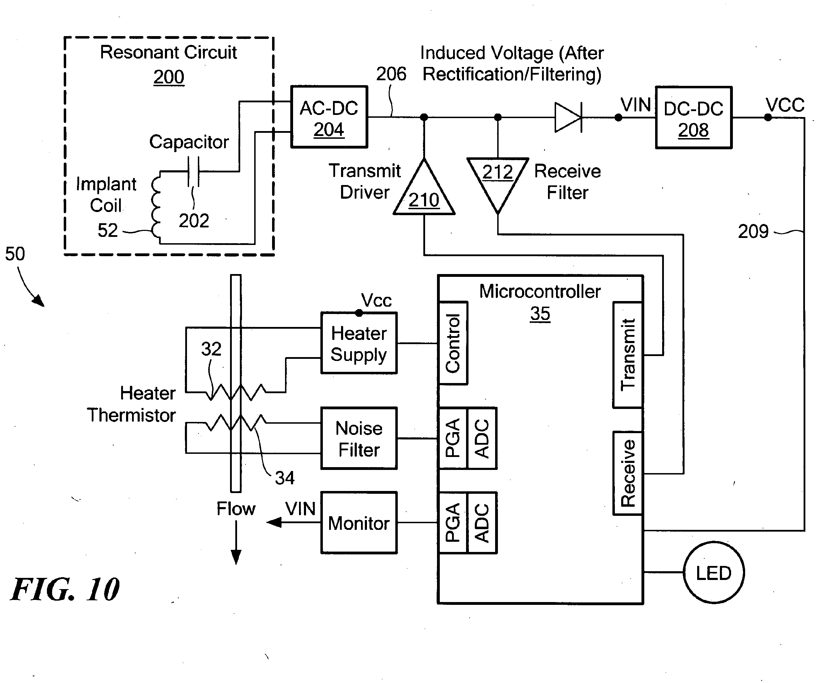

Encapsulated implant, Figs. 1-3, also includes implant power and communication subsystem 50, Fig. 10, preferably formed on printed circuit board 55, Fig. 3. Implant power and communication subsystem 50, Fig. 10, is configured to wirelessly receive power and transmit and receive data.

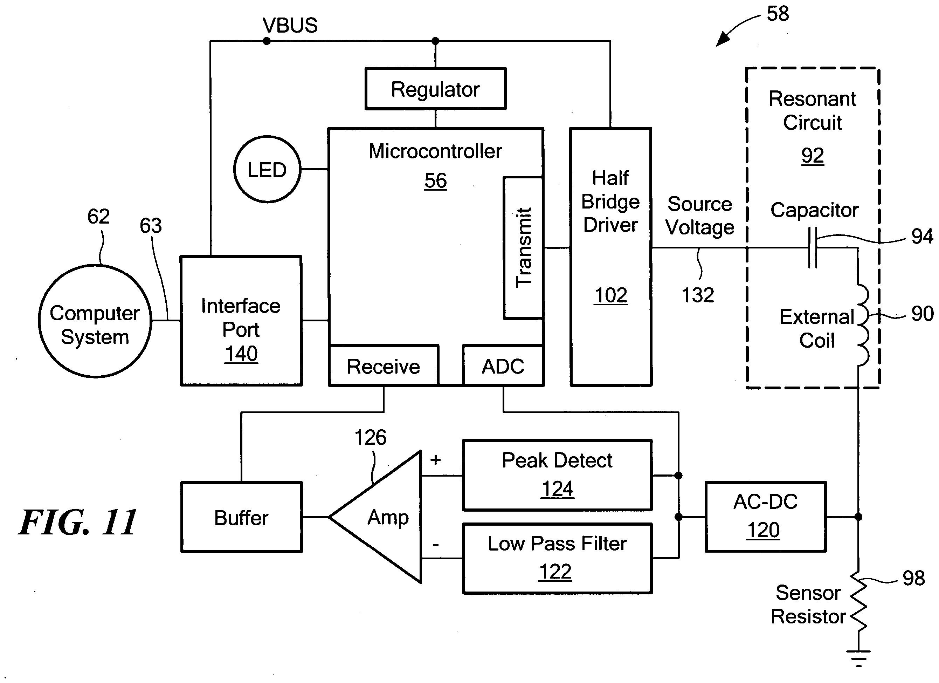

External device 14, Fig. 1, of system 10 also includes external microcontroller 56, Fig. 11, and external power and communication subsystem 58 coupled to external microcontroller 56 configured to wirelessly deliver power to implant power and communication subsystem 50, Fig. 10, and wirelessly transmit and receive data to and from implant power and communication subsystem 50 of encapsulated implant 12, Figs. 1-3, as discussed in further detail below.

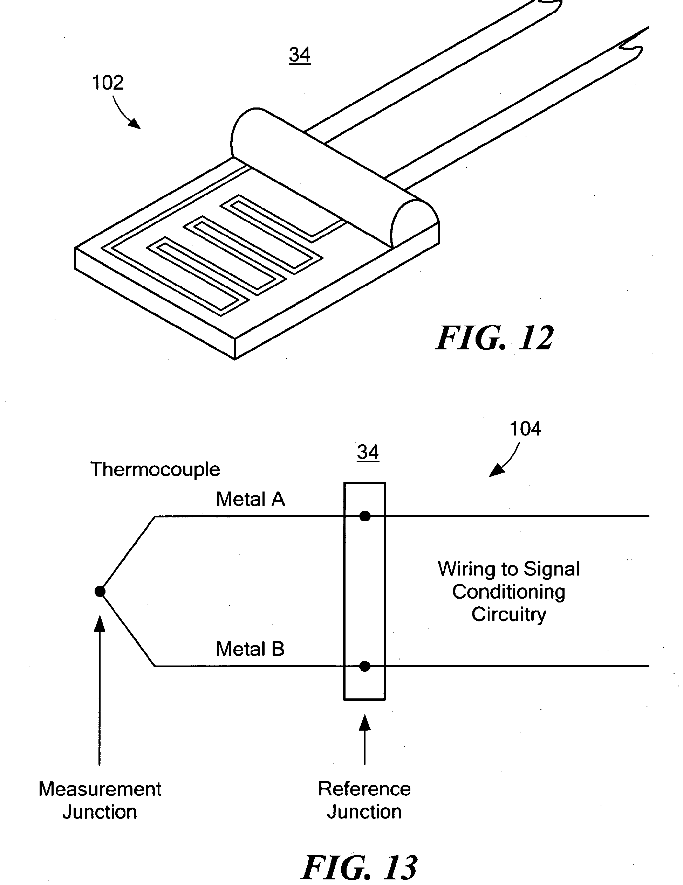

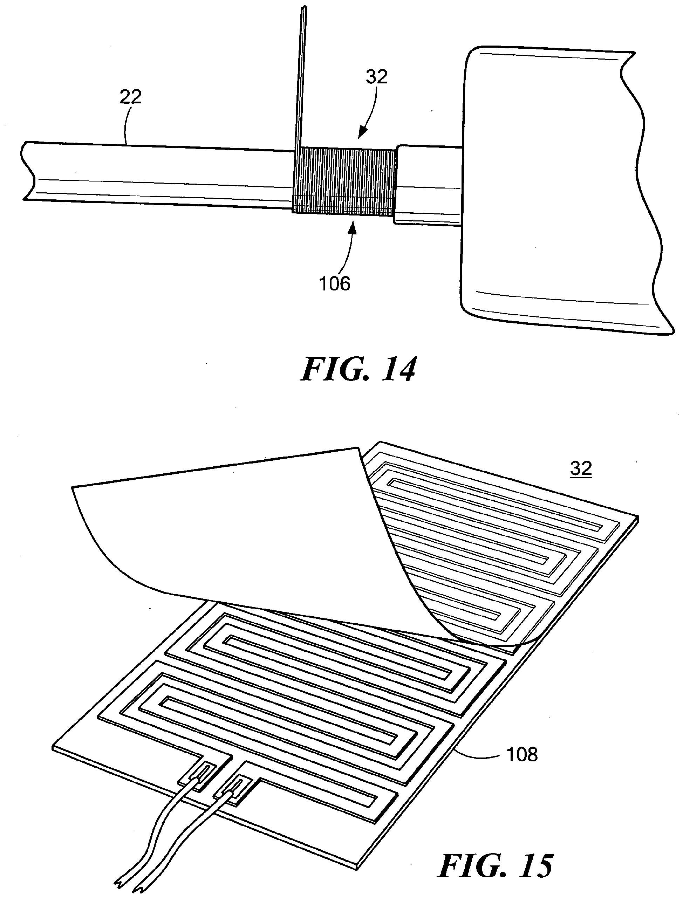

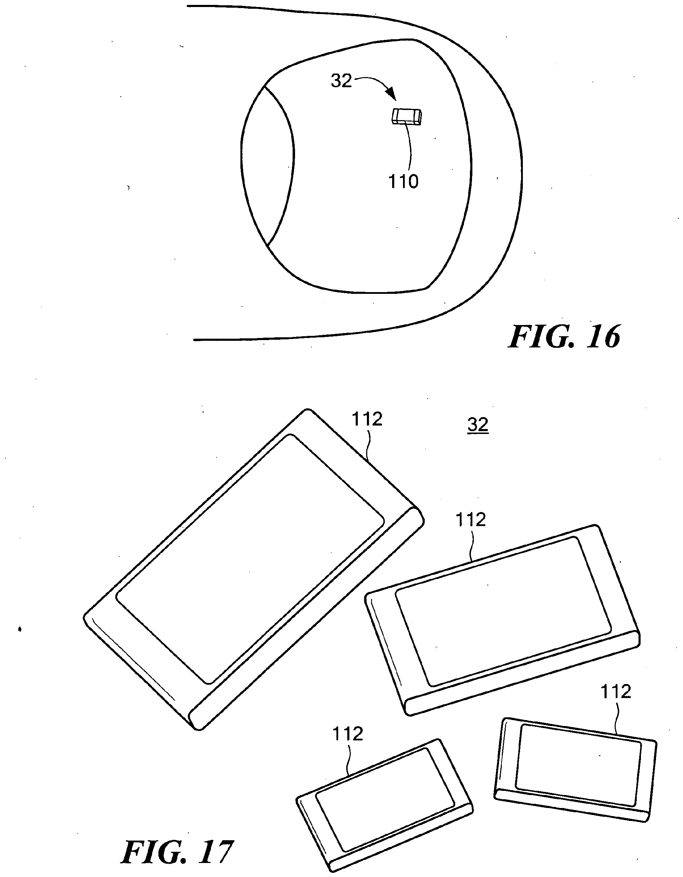

In one example, temperature sensor 34, Figs. 3 and 4, includes a thermistor, e.g., thermistor 34' as shown in Fig. 10. In other designs, temperature sensor 34 may be a resistance temperature detector (RTD), e.g. RTD 102, Fig. 12, or a thermocouple, e.g., thermocouple 104, Fig. 13. In other examples, heating element 32 may be a coil of electrically conductive wire wound around the flow tube 22, e.g., coil 106, Fig. 14, of electrically conductive wire. In another design, heating element 32 may be a printed circuit heater, e.g., printed circuit heater 108, Fig. 15. In yet another design, heating element 32 may be a resistor (either surface mount or leaded), e.g. resistor 110, Fig. 16, or any of resistors 112, Fig. 17.

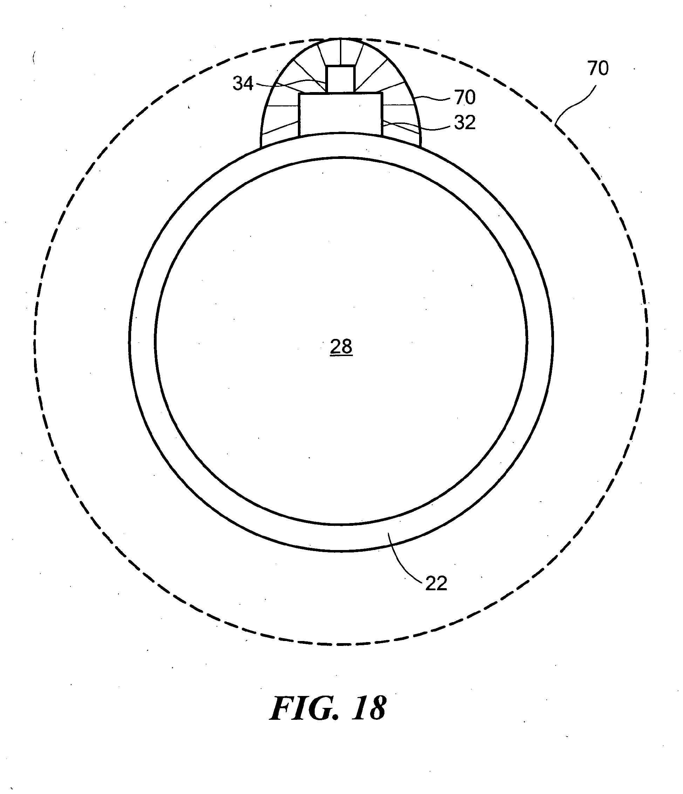

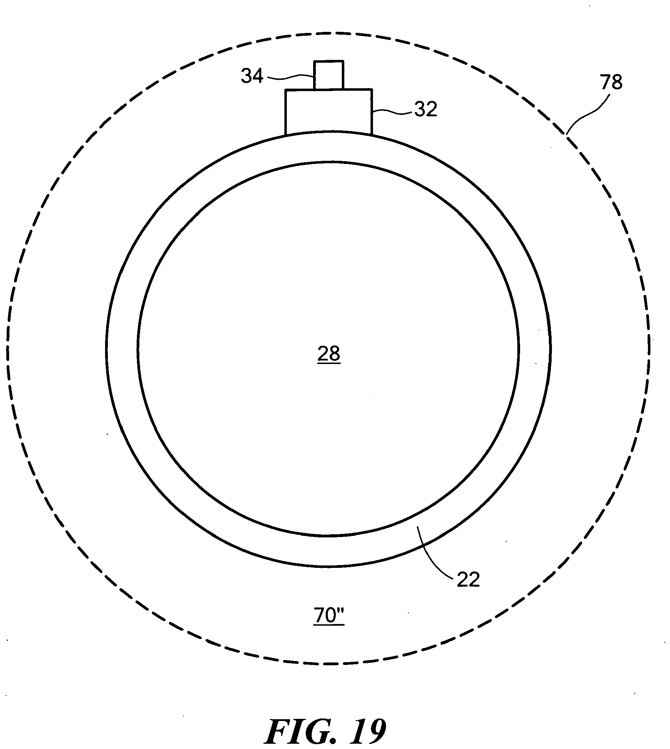

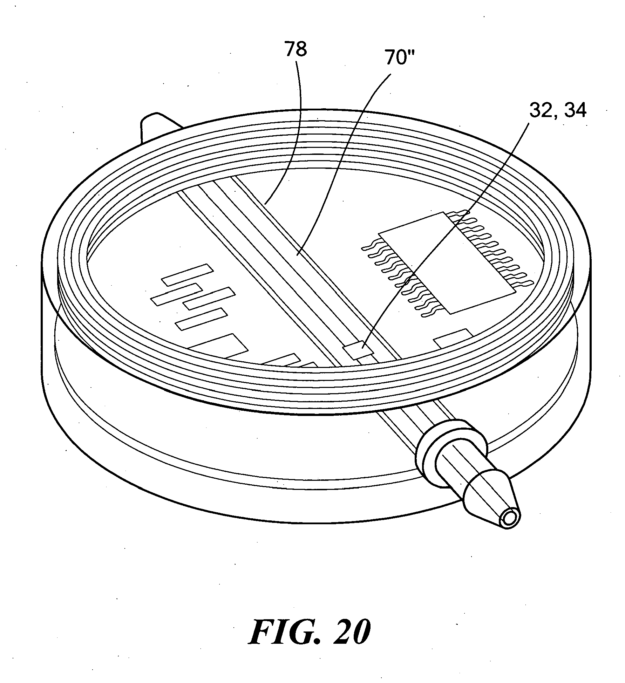

Preferably, heating element 32, Figs. 3 and 4, is directly attached to flow tube 22 as shown in Fig. 4. The rise in temperature of the heating element 32 over the predetermined amount of time or the temperature drop over the predetermined amount of time of cooling represents the 'signal' employed during a flow rate measurement to determine flow rate. The signal may be increased by thermally isolating the heating element 32 from heat transfer paths other than conduction/convection to the flow of bodily fluid 28, Fig. 3, flowing through the flow tube 22. In one design, encapsulated implant 12 includes thermal insulator 70, Fig. 18, which preferably covers heating element 32 and temperature sensor 34 as shown to thermally isolate heating element 32 and temperature sensor 34 from heat transfer paths other than conduction/convection to the flow of bodily fluid 28, Fig. 3. Thermal insulator 70 may also surround all of flow tube 22, as shown by thermal insulator 70' in phantom. In other examples, insulation layer 70", Fig. 19, may be a pocket of sealed air created by surrounding flow tube 22 with hollow tube 78. Fig. 20, where like parts have been given like numbers, shows in further detail insulation layer 70" of sealed air and hollow tube 78 surrounding heating element 32 and temperature sensor 34 and flow tube 22.

In one example, flow tube 22, Figs. 3, 4, 18-20, may be made of a thin walled polymer material with low thermal conductivity, such as polyimide or similar type material, to limit heat transfer along the length and circumference of flow tube 22 while maintaining heat transfer in the radial direction to the bodily fluid in tube 22 made viable by the thin wall thickness of the tube.

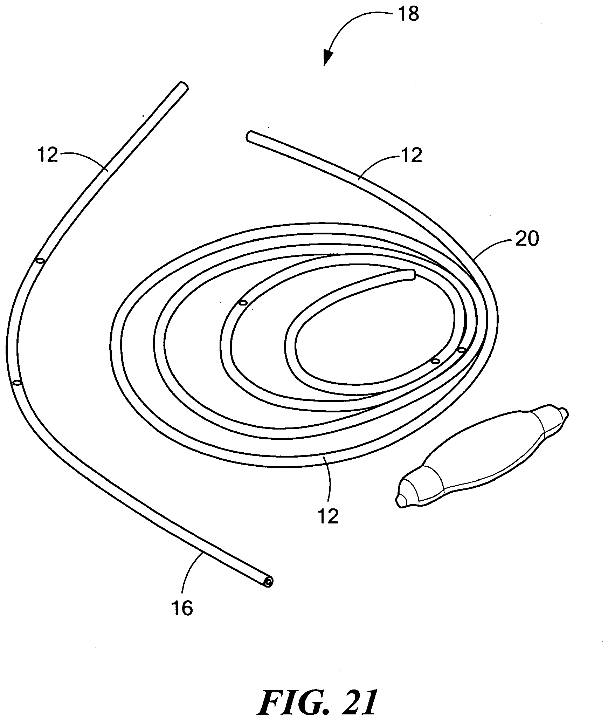

As discussed above, encapsulated implant 12, Fig. 1, may be coupled to VP shunt 18, e.g., to distal catheter 20 of VP shunt 18 or proximal catheter 16. Fig. 21 shows in further detail one example of the structure of VP shunt 18 with distal catheter 20 and proximal or ventricular catheter 16. Encapsulated implant 12 may be located at any position on ventricular catheter 16 or distal catheter 20 as shown. In other examples, encapsulated implant may be coupled to shunt, catheter, tube, or vessel implanted in the body, such as a ventroarterial shunt or a lumboperitoneal shunt.

In the example shown in Figs. 2 and 3, heating element 32 and temperature sensor 34 are shown located proximate outlet 26. In other examples, heating element 32 and temperature sensor 34 may be located proximate inlet 24 indicated at 80, Figs. 3, or between inlet 24, and outlet 26, as indicated at 82.

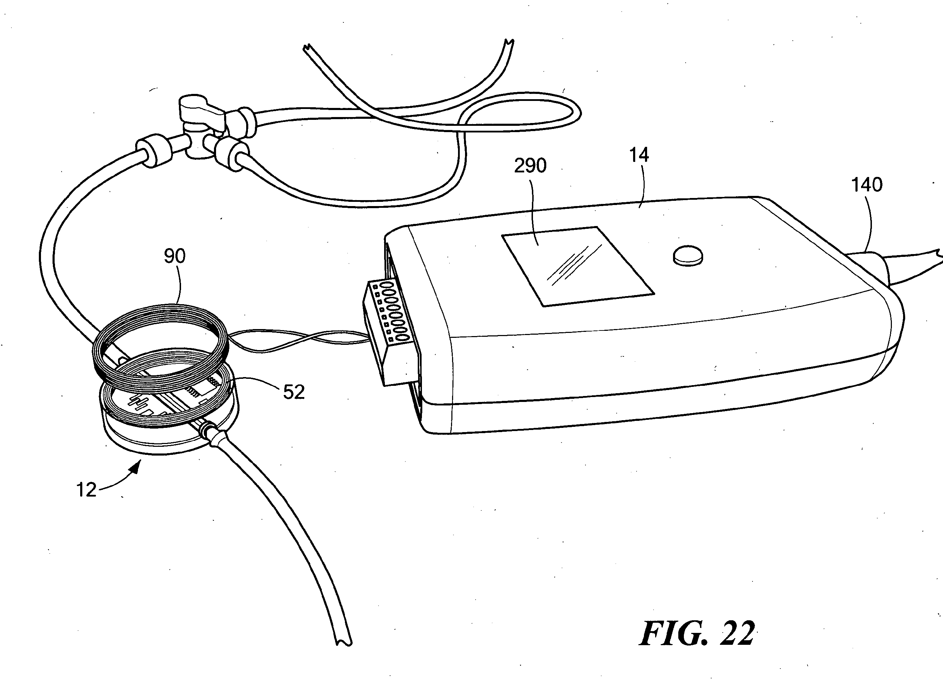

External power and communication subsystem 58, Fig. 11, of external device 14, Fig. 1, includes external coil 90, Fig. 11 coupled to microcontroller 56. Fig. 22, where like parts have been given like numbers, shows in further detail one example of external coil 90 of external device 14 shown placed in close proximity to implant coil 52 of encapsulated implant 12. As shown, implant coil 52 is integrated with encapsulated implant 12, as depicted in further detail in Fig. 3. External power and communication subsystem 58, Fig. 11, is configured to inductively transfer power from external coil 90, Figs. 11 and 22, to implant coil 52, Figs. 3 and 22, of implant power and communication subsystem 50, Fig. 10.

It is well known that the presence of a time-varying current in one coil will induce a voltage in a nearby second coil. This principle is employed by system 10 for measuring the flow rate of a bodily fluid to enable wireless power transfer and communication between external device 14 and encapsulated implant 12. The voltage ($V_2$) induced in the implant coil 52 by external coil 90 may be shown by the equation:

$$V_2\,(t) = M\,(dI_1/dt)$$

$$(2)$$

where M is the mutual inductance between the implant coil 52 and external coil 90 and $I_1$ (t) is the current in the external coil 90. If the current in the external coil 90 coil is sinusoidally-varying in time at a frequency $\omega = 2\pi f$, where $f$ is the frequency in Hertz, then:

$$\underline{V}_2 = \omega M \underline{I}_1, \qquad\qquad (3)$$

where $\underline{V}_2$ and $\underline{I}_1$ are the amplitude of the voltage induced in implant coil 52 and the amplitude of the current in the external coil 90, respectively. Likewise, if the current in the implant coil is time-varying, a voltage is induced in external coil 90 given by:

$$\underline{V}_1 = \omega M \underline{I}_2, \qquad\qquad (4)$$

where $\underline{V}_1$ and $\underline{I}_2$ are the amplitude of the voltage induced in the external coil 90 and the amplitude of the current in external coil 90, respectively

The mutual inductance depends both on the self-inductances of the coupled external coil 90 ($L_1$) and implant coil 52 ($L_2$) coils and the coupling coefficient ($K_C$) between them:

$$M = K_C (L_1 L_2)^{1/2}, \qquad\qquad (5)$$

where $K_C$ depends on relative orientation, lateral alignment and proximity of the external coil 90 and implant coil 52. The self-inductance of the external coil 90 ($L_1$) is preferably set such that the source voltage 132, Fig. 11, of the external power and communication subsystem 58 is at a convenient and safe level, whereas the self-inductance of implant coil 52 ($L_2$) and the coupling coefficient ($K_C$) are preferably sufficient such that the induced voltage (after rectification and filtering) on implant power and communication subsystem 50, Fig. 10, of encapsulated implant 12 is high enough to meet the input voltage specifications of the DC-DC converter 208, Fig. 10, that provides the regulated DC voltage necessary to operate the implant microcontroller 35, heating element 32, temperature sensor 34 and other various electronic components of encapsulated implant 12, Fig. 3, e.g., printed circuit board (PCB) 55 and the various electronics thereon.

External power and communication subsystem 58, Fig. 11, preferably includes external resonance circuit 92 comprised of external coil 90, and capacitor 94, and source voltage 132 generated by a half bridge driver 103 or by other equivalent device known to those skilled in the art. The external power and communication subsystem 58 is preferably configured to generate AC current flow at a predetermined resonance frequency in external coil 90, in order to induce sinusoidal voltage signals in implant coil 52, shown in at least Figs. 10 and 22.

In one example, resonant circuit 92, Fig. 11, and analog electronics 96 filter and amplify changes in the voltage drop across current sense resistor 98 in order to recover data communications bits transmitted from implant power and communication subsystem 50, Fig. 10, of encapsulated implant 12. Half-bridge driver circuit 103, Fig. 11, with a dedicated controller and two MOSFETs (not shown) may be used to drive external coil 90. External coil 90 in combination with series capacitor 94 preferably forms a resonant circuit with a predetermined resonant frequency, e.g., 100 kHz. In one example, a 100 kHz square wave generated by external microcontroller 56 may be applied to half-bridge driver circuit 103 to create a sinusoidal current flow through the external coil 90. The frequency may be adjusted to produce the closest match between resonant circuit 92 and resonant circuit 200, Fig. 10, of implant power and communication subsystem 50, as discussed below. The voltage drop across sense resistor 98, Fig. 11, may be used to monitor the current through external coil 90. The voltage across sense resistor 98 is preferably converted to DC by AC-DC rectifier 120 and filtered by filter 122 and peak detector 124 to remove the 100 kHz signal. The difference between the peak voltage and the filtered voltage is then amplified by amplifier 126, converted to digital signal levels and fed to external microcontroller 56 for decoding of the digital data transmitted by the implant power and communication subsystem 50, Fig. 10 of encapsulated implant 12. In order to provide communications as well as power, external power and communication subsystem 58, Fig. 11, of external device 14 can modulate the square wave signal delivered to half-bridge driver 103 to encode information. The implant power and communication system 50, Fig. 10, can decode the modulation in order to recover the data being transmitted.

The inductance of external coil 90, Fig. 11, and capacitor 94 create a resonant frequency in accordance with the formula:

$$f_n = 1/[2\pi(LC)^{1/2}] \qquad\qquad (6)$$

where L is the inductance of external coil 90 and C is the capacitance of capacitor 94. At this frequency, the reactive impedance of capacitor 94 cancels out the reactive impedance of external coil 90, and in the vicinity of this frequency, both the reactance and overall impedance of the external power and communication subsystem 58, Fig. 11, of external device 14 are greatly reduced.

The use of resonance frequency may be beneficial because square-wave pulses, which are conveniently produced by half bridge driver 103 or other AC voltage source known to those skilled in the art, give rise to sinusoidally-varying current in external power and communication subsystem 58. Further, the impedance of series resonant circuit 92 is a minimum at resonance, which maximizes the current for a given applied voltage, thereby lowering the voltages to levels as may be found in a common battery or USB interface, e.g., interface port 140. In addition, since the current through external coil 90 varies with the applied square wave frequency, the power delivered to the external coil 90 can be easily tuned by changing the square wave frequency. The value of capacitor 94 is preferably chosen such that the capacitor 94 and external coil 90 resonate at a desirable frequency. The choice of resonant frequency may include, *inter alia,* the available space for external coil 90, frequency-dependent coil losses, skin effect, FCC regulations, guidelines regarding patient exposure to electromagnetic fields, and the like. Preferably, resonant circuit 92 is driven by a square wave source voltage 132, with its frequency set at or near the resonant frequency of resonant circuit 92. This results in a sinusoidally-varying current in the external power and communication subsystem 58 at the frequency of the voltage source pulses. This current gives rise to a magnetic field in the space surrounding external coil 90. A fraction of the field lines of this magnetic field are inductively linked to implant coil 52, Fig. 10, thereby inducing sinusoidally-varying voltage in implant coil 52.

Implant power and communication subsystem 50, Fig. 10, of encapsulated implant 12 includes implant resonance circuit 200 comprised of implant coil 52 and capacitor 202. Implant resonance circuit 200 is preferably configured to have a resonance frequency matching the resonance frequency closely provided by resonance circuit 92, Fig. 11. The sinusoidally varying magnetic field generated by the sinusoidal current in external coil 90 links the implant coil 52 of resonant circuit 200. The resulting induced sinusoidally varying voltages in implant coil 52, Fig. 10, are then rectified by AC-DC rectifier 204 to create a DC voltage on line 206, which is applied to the input of DC-DC converter 208, which creates a constant regulated DC voltage on line 209. DC-DC power supply 208 provides power to implant microcontroller 35, heating element 32, e.g., a thermistor, in this example, acting as temperature sensor 34, and other components on PCB 55, Fig. 3, of encapsulated implant 12, which may require power.

In one example, external device 14, Figs. 1 and 22, encodes digital data for communication with encapsulated implant 12, shown in one or more of Figs 1-3, 10 and 22, by changing the magnitude of the source voltage 132, Fig. 11, which gives rise to a corresponding change in current in external power and communication subsystem 58, which, in turn, gives rise to a change in the amplitude of the voltage induced on implant coil 52. Implant microcontroller 35 preferably monitors the voltage at the output of the AC-DC rectifier 204 by receive filter 212, in order to decode digital data sent from external power and communication subsystem 58 of external device 14.

Implant power and communication subsystem 50, Fig. 10, of encapsulated implant 12, Figs. 1-3, 10 and 22, preferably communicates to external power and communication subsystem 58, Fig. 11, of external device 14, by modulating the electrical load on implant coil 52, by controlling the closure of a switch within the transmit driver 210, Fig. 10, coupled to the output of AC-DC rectifier 204. Closure of the transmit driver switch 210 gives rise to an abrupt increase in current in the implant coil 52 and AC-DC rectifier 204 of the implant power and communication subsystem 50, which, in turn, gives rise to a change in the induced voltage and current flow in external coil 90, and external power and communication subsystem 58. The voltage drop across the sense resistor 92 provides a means for monitoring the current flow in external power and communication subsystem 58 and external device 14 and thus provides a means for external microcontroller 56, onboard the external device 14, to decode the changes in current into digital data. The maximum rate of data transfer (i.e., baud rate) may be limited by the carrier frequency. In one example, at a carrier frequency of 100 kHz, a reasonable rate is 1200 baud.

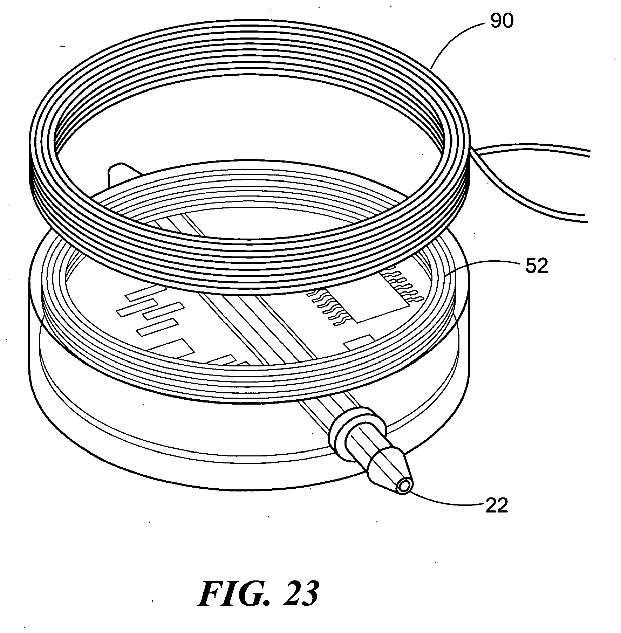

Preferably, implant coil 52, Figs. 3, 10, and 22, and external coil 90, Figs. 11 and 22, are preferably placed in close proximity to each other, e.g., as shown in Fig. 22 and in further detail in Fig. 23 to provide sufficient inductive coupling between implant coil 52 and external coil 90 such that external power and communication subsystem 58 can wirelessly provide power to implant power and communication subsystem 50 and data can be wirelessly communicated to and from external power and communication subsystem 58 and implant power and communication subsystem 50, as discussed above.

In one embodiment, external coil 90 of external device 14 may be located relative to implant coil 52 of encapsulated implant 12 in human body 15, Fig. 1, using data wirelessly sent from implant power and communication subsystem 50 to external power and communication subsystem 58. For example, data communicated from implant power and communication subsystem 50 to external power and communication subsystem 58 includes the magnitude of the induced voltage (after rectification and filtering) onboard implant power and communication subsystem 50 of encapsulated implant 12, which provides a means by which a user of system 10 can position external device 14 and external coil 90, e.g., as shown in Fig. 1, relative to implant coil 52 of encapsulated implant 12 in human body 15. Preferably, the induced voltage onboard implant power and communication subsystem 50 is sufficient to both enable wireless communication and power transfer and to power the implant power and communication subsystem 50. Thus, the value of the induced voltage of implant coil 52 of implant power and communication subsystem 50 can be the basis for an intuitive, graphical display by external device 14 (discussed below) that enables the user to readily find an acceptable location for the external device 14 and to verify that sufficient coupling between implant coil 52 of encapsulated implant 12 and external coil 90 of external device 14 has been achieved for a calibration or flow measurement. Proper placement and orientation of external coil 90 of external device 14 and implant coil 52 of encapsulated implant 12 over the course of the flow rate measurement can be maintained by, *inter alia,* positioning and securing the external device 14 with apparel or by hand, such that the external coil 90 is positioned over implant coil 52, affixing the external coil 90 temporarily to the skin directly over the implant coil 52, e.g., using medical grade tape or adhesive, or longer term affixation, e.g., suturing, adhesive, of the external device 14 and external coil 90 to the skin over the encapsulated implant 12 for a period over which regular flow measurements will be needed.

Preferably, implant microcontroller 35, Figs. 3 and 10, is configured to store the measured flow rate, the stored set of previously obtained calibration measurements, e.g. as shown in Figs. 5 and 8, and identification information associated with the encapsulated implant 12, e.g., the serial number, model number, and the like, in a non-volatile manner.

In one example, external device 14, Fig. 22, includes display 290 which may display the measured flow, the previously obtained calibration measurements, the value of induced voltage on implant coil 52 or similar type measurements or values, and the identification information associated with encapsulated implant 12.

External device 14, Figs. 1, 11, and 20, may include interface port 140 coupled to external microcontroller 56 configured to connect to computer subsystem 62, Fig. 11, by electrical cable 63. In another example, interface port 140 coupled to external microcontroller 56 may be configured to wirelessly connect computer subsystem 62. Interface port 140 coupled to external microcontroller 56 may also be configured to wirelessly connect to computer subsystem 62 configured as a smart device.

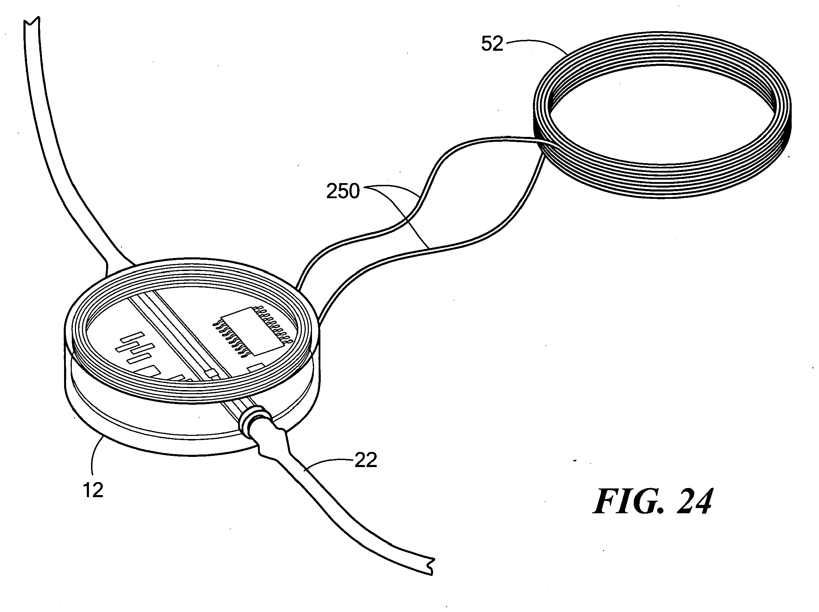

Although, as discussed thus far, implant coil 52, shown in one or more of Figs. 2, 3, 22 and 23, is shown integrated with encapsulated implant 12, this is not a necessary limitation of this invention. In other embodiments, implant coil 52, Fig. 24, where like parts have been given like numbers, may be located remotely from encapsulated implant 12 as shown and coupled to encapsulated implant 12 with wires 250.

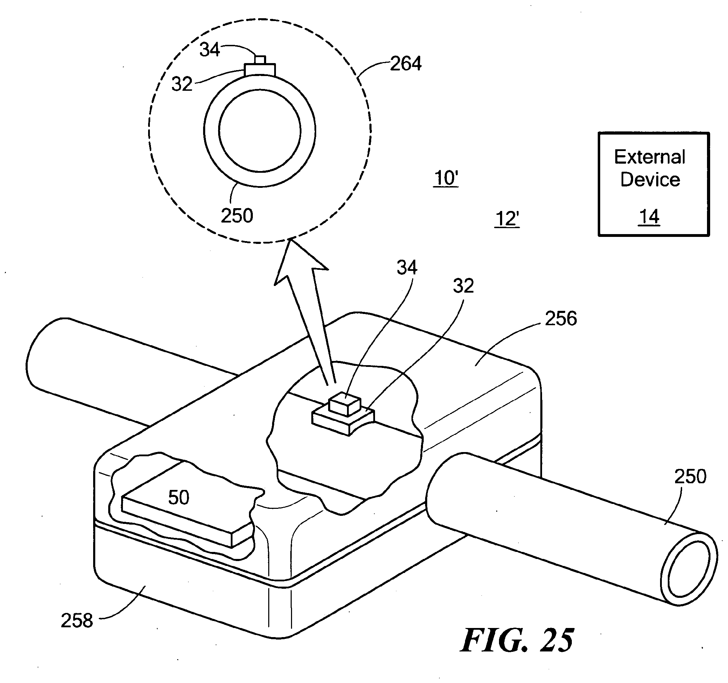

Although, as discussed above with reference to one or more of Figs. 1-24 encapsulated implant 12 is shown having flow tube 22, Fig. 3, with inlet 24 and outlet 26 which receive flow of bodily fluid 28 in-line and heating element 32 and temperature sensor 34 and implant power and communication subsystem 50 are integrated in part of encapsulated implant 12, this is not a necessary limitation of this invention. In another embodiment, flow rate sensor system 10', Fig. 25, where like parts have been given like numbers, for non-invasively measuring the flow rate of a bodily fluid includes external device 14 with external power and communication subsystem 58, having the same design as discussed above with reference to at least Figs. 1, 11, and 20. However, in this embodiment, system 10', Fig. 25, includes encapsulated implant 12' that is clamped over a shunt, tube, vessel or catheter 250 implanted in a human body or animal body. Encapsulated implant 12' clamps over shunt, tube, vessel or catheter 250 using clamshell device 252 with clamping members 256 and 258 as shown. Encapsulated implant 12' includes heating element 32 and temperature sensor 34 and implant power and communication subsystem 50 having a similar structure as discussed above with reference to one or more of Figs. 1-24. In this design, heating element 32, Fig. 25, externally and directly couples to shunt, tube, vessel or catheter 250, and temperature sensor 34 is directly externally coupled to heating element 32, e.g., as shown in blow-out caption 264. System 10' operates similar to system 10, discussed above, with reference to one or more of Figs. 1-24.

External device 14, Figs. 1, 22, and 25, may be a dedicated unit, designed for measuring the flow rate of a bodily fluid, or may be a smart device, such as a phone or tablet with an App and attached coil accessory similar to external coil 90 and wires connecting it to external device 14.

The result is flow rate sensor system 10, shown in one or more of Figs. 1-25, that accurately and measures the flow rate of a bodily fluid and provides a means of obtaining quantitative information on how a shunt, such as a VP shunt, or other similar type shunt, tube, vessel, or catheter, is functioning when implanted in a human or animal body. System 10 in some examples eliminate the need for obtaining cranial imaging using ultrasound, CT scanning, MRI, X-ray, and the like. Flow rate sensor system 10 can display or report the rate of flow of bodily fluids, such as CSF and other bodily fluids, and can be queried transcutaneously to allow the clinician to non-invasively assess shunt function during emergency room visits or during routine office visits. System 10 enables the primary care physician or specialist to see changes in the flow of bodily fluids over time and anticipate shunt failures prior to the development of symptoms. Thus, flow rate sensor system 10 enables timely intervention to maintain shunt function and reduce the likelihood of emergency shunt revision surgeries. Flow rate sensor system 10 can measure and monitor flow rate of bodily fluids in a patient with a shunt who arrives at the emergency room with symptoms possibly indicative of shunt failure and quickly and accurately provide the clinician with information regarding shunt function and, thus, can avoid unnecessary diagnostic or surgical procedures. The result is better care, reduced risk of death or injury from shunt failure, and reduced cost of care for those whose lives depend on continuous and proper function of their shunts.

External device 14 enables the clinician to obtain and store a "snapshot" of flow rate of CSF or other bodily fluids whenever needed. Because patient posture and orientation can affect flow through a shunt, the clinician can choose to place patient in various orientations and then take a flow rate measurement at selected orientations. The external device or external coil can be affixed to the patient to enable automatically-initiated, periodic measurements and storage of the flow rates of bodily fluids, such as CSF, over an extended time period. This allows the clinician to see any trends in the flow characteristics of the shunt over a desired period of time. For example, CSF flow rate measurements could be automatically obtained every half hour to monitor shunt function, both in the hospital and after discharge, for the critical days following a shunt placement or a shunt revision surgery. In a second example, a CSF flow rate measurement could be taken every 5 minutes on a shunted patient who arrives at the emergency room with symptoms possibly indicative of shunt failure. This would give the clinician complete knowledge of the flow characteristics of the shunt, possibly preventing unnecessary diagnostic or surgical procedures, including MRI or CT imaging and shunt revisions.

For enablement purposes only, the following code portions are provided which can be executed on implant microcontroller 35 and external microcontroller 56 to carry out the primary steps and/or functions of flow rate sensor system 10 shown in one or more of Figs. 1-25 and recited in the claims hereof. Other equivalent algorithms and code can be designed by a software engineer and/or programmer skilled in the art, using the information provided herein.

IMPLANT CODE

```
/*
 * comm.c
 *
 *  Created on: Feb 1, 2013
 *      Author: Tom
 */

#include <stdio.h>
#include <stdlib.h>
#include <string.h>

#include "main.h"
#include "comm.h"
#include "measure.h"
#include "uart1.h"

// List of all commands with a short description
const struct cmd_table commands[] =
{
            {"cal", "calibrate flow (ml/hr)", do_calibrate},
            {"del", "delete cal table flow (ml/hr)", do_delCal},
            {"meas", "measure flow", do_meas},
            {"adc",  "display ADC <loop count>", do_adc},
            {"zero", "zero ADCs", do_zero},
            {"exp", "set exp averaging", do_exp},
            {"up", "y = use upstream sensor", do_upstream},
            {"time", "set scan time <time> <interval>", do_time},
            {"heat", "0 = off, 1 = on", do_heat},
            {"com", "comm test off time (ms)", do_comm},
            {"term", "comm echo test", do_term},
//          {"echo", "echo chars to remote", do_echo},
            {"info", "display settings", do_info},
            {"help", "this help screen", do_help},
            {"", "", NULL}
};

// UART selector  0 = UART0, 1 = UART1
uint8_t uartSelect = 0;

// I/O buffer is global so it can be shared and strtok can be called
// from called function
char line[STRLEN];
// global buffer for dec to str conversions
char decstr[VSTRLEN];

const char delim[] = " ,\t";
const char null_str[] = "";

//----------------------------------------------------------------------------
// cmdHandler:  Processes commands received from USB
//----------------------------------------------------------------------------
void cmdHandler(void)
{
        char *cmd;
        int i;

        uartSelect = 0;                                      // reset to
uart 0
        if (kbhit1()) uartSelect = 1;
```

```
            else if (!kbhit()) return;

            getInput(line, STRLEN);
            cmd = strtok(line, delim);
//          parse(line,cmd);
            if (cmd != NULL)
            {
                    for (i = 0; commands[i].func != NULL; ++i)
                    {
                            if (strcmp(commands[i].cmd, cmd) == 0)
                            {
                                    // pass the first parameter to called function
                                    // additional parameters can be fetched by calling
strtok
                                    (commands[i].func)(strtok(NULL,delim));
                                    break;
                            }
                    }
                    if (commands[i].func == NULL && cmd[0] != ';') printf("Invalid
command\r\n");
            }
            printf("\r\nI> ");
    }

    //------------------------------------------------------------------------
    // do_term:  Send and receive characters from UART0 to UART1
    //------------------------------------------------------------------------
    void do_term(char *word)
    {
            char ch;

            while (1)
            {
                    if (kbhit1())
                    {
                            ch = getchar1();
                            putchar(ch);
                    }
                    if (kbhit())
                    {
                            ch = getchar();
                            if (ch == 0x1b) break;
                            putchar1(ch);
                            putchar(ch);
                    }
            }
            // flush buffers
            if (kbhit1()) getchar1();
            if (kbhit()) getchar();
    }

    //------------------------------------------------------------------------
    // do_comm:  Comm test
    //------------------------------------------------------------------------
    void do_comm(char *word)
    {
            uint16_t offtime = 0,ontime = 0;

            if (word != NULL)
            {
                    ontime = atoi(word);
```

```
                {
                        if (ontime == 0)
                        {
                                while (!kbhit0() && !kbhit1())
                                {
                                        putchar1(word[0]);
                                        usdelay(offtime);
                                }
                                return;
                        }
                }
                word = strtok(NULL,delim);
                if (word != NULL) offtime = atoi(word);

                __bic_SR_register(GIE);                          // Disable all
        interrupts
                while (!kbhit0() && !kbhit1())
                {
                        TX2_ON();
                        usdelay(ontime);
                        TX2_OFF();
                        usdelay(offtime);
                }
                __bis_SR_register(GIE);                          // Enable all
        interrupts
                }
                getchar();
        }

//----------------------------------------------------------------------------
// do_heat:  Turns heater on or off
//----------------------------------------------------------------------------
        void do_heat(char *word)
        {
                if (word != NULL)
                {
                        if (atoi(word) == 1) HEAT_ON();
                        else HEAT_OFF();
                }

        }

        //----------------------------------------------------------------------------
        // do_upstream:  Enable or disable the upstream temperature sensor
        //   'y' = upstream sensor is present
        //   'n' = no upstream sensor
        //----------------------------------------------------------------------------
        void do_upstream(char *word)
        {
                if (word != NULL)
                {
                        if (strchr(word,'y') != NULL) fset.mode |= UPSTREAM;
                        else fset.mode &= ~UPSTREAM;
                        printf("Sensor must be re-calibrated after changing mode\r\n");
                }
        }
//----------------------------------------------------------------------------
// do_calibrate:  Measure a flow and record calibration values
//   Format: cal <flow rate> [<heater rise> <downstream rise]
//   flow rate is in ml/hr, heater and downstream rise in degrees C
//----------------------------------------------------------------------------
```

```
void do_calibrate(char *word)
{
        int i;
        flowCal_t new;

        // If there are no arguments, list the cal table
        if (word == NULL)
        {
                printf("\tFLOW\tHEAT\r\n");
                for (i = 0; i < fset.calEntries; ++i)
                {
                        printf("cal\t%s\t", decToStr(fset.cal[i].flow));
                        printf("%s\r\n", decToStr(fset.cal[i].heat));
        //              printf("%s\r\n", decToStr(fset.cal[i].down));
                }
                return;
        }
        // Check to make sure there is room in the cal table
        if (fset.calEntries >= MAX_CAL_TABLE)
        {
                printf("Cal table is full - delete first\r\n");
                return;
        }

        // Parse the flow rate and check that it is valid
        new.flow = strToDec(word);
        if (new.flow < 0 || new.flow > 9900)
        {
                printf("Invalid flow rate\r\n");
                return;
        }

        // Parse for user entry of heat param of cal table
        // If present, enter into table without performing a temp scan
        // This allows the cal table to be restored from a saved file on the PC
        word = strtok(NULL,delim);
        if (word != NULL)
        {
                new.heat = strToDec(word);
                addCal(new);
        }
        // Perform a calibration by doing a temperature scan and then entering
the
        // results into the calibration table
        else
        {
                scanReady();
                printf("Set flow to %s ml/hr and press enter...",
decToStr(new.flow));
                if (getchar() == 0x1b) return;                // escape
                printf("\r\n");
                calibrate(new.flow);
        }
        writeSettings();
}

//-----------------------------------------------------------------------
// do_delCal:  Delete an entry in the calibration table
//   Format: del <flow rate> (ml/hr)
//-----------------------------------------------------------------------
        void do_delCal(char *word)
```

```
        {
                int16_t flowRate;

                if (word != NULL)
                flowRate = strToDec(word);
                {
        //      if (flowRate == 0 && strchr(line,'0') == NULL) return;
                        delCal(flowRate);
                        writeSettings();
                }
        }
}

//--------------------------------------------------------------------------
// do_meas:  Measure flow rate by doing a temperature scan and then computing
        flow
//  Format: meas
//--------------------------------------------------------------------------
        void do_meas(char *word)
        {
                int16_t flow;

                scanReady();
                printf("Start flow and press enter...");
                if (getchar() == 0x1b) return;
                printf("\r\n");
                flow = measureFlow(1);
                if (flow >= 0) printf("\r\nFlow = %s ml/hr\r\n", decToStr(flow));
        }

//--------------------------------------------------------------------------
        // do_zero:  Zero the ADCs
        //  Format: zero
        //--------------------------------------------------------------------------
        void do_zero(char *word)
        {
                zeroADC();
                printf("ADC0 = %d  ADC1 = %d  ADC2 = %d\r\n",
                                (int16_t)(fset.zero[0]/fset.exp),
                                (int16_t)(fset.zero[1]/fset.exp),
                                (int16_t)(fset.zero[2]/fset.exp));
        }

//--------------------------------------------------------------------------
        // do_adc:  Display current ADC readings until key press
        //  if a parameter of c is specified, show ADC counts instead of temperature
        //  Format: adc [c]
//--------------------------------------------------------------------------
        void do_adc(char *word)
        {
                int i, cnt = 0;
                uint16_t now, timeStamp = 0;

                if (word != NULL) cnt = atoi(word);
                if (cnt == 0) cnt = 32000;

                if (cnt != 1) printf("TIME\tUP\tHEAT\tVIN\r\n");
                now = ms10;
                while (!kbhit() && cnt != 0)
                {
                        for (; cnt != 0 && !kbhit(); --cnt)
                        {
```

```
                              idle = tempEven();
                              if (cnt != 1 || timeStamp != 0) printf("%s\t",
        dec1ToStr(timeStamp));

                              for (i = 0; i < 2; ++i)
                              {
                                      if (strchr(word, 'c') == NULL) printf("%s",
        decToStr(chanTemp(i)));

                                      else printf("%d",chanADC(i));
                                      putchar('\t');
                              }
                              printf("%s\r\n",decToStr(inVoltage()));
                              while (now - ms10 < fset.interval*10);     // Wait for
        interval
                              timeStamp += fset.interval;                 // Increment
        time stamp
                              if (timeStamp > 30000) timeStamp = 0;      // in case of roll
        over
                              now += fset.interval*10;                        //
        increment
        for next interval
                      }
                }
                if (kbhit()) getchar();
        }
        /*
        //--------------------------------------------------------------------------------
        // do_chipTemp:  Display the microcontroller chip temperature
        //   If a parameter is given, calibrate to that temperature
        //   Format:  chip [<cal temperature>] (deg C)
        //--------------------------------------------------------------------------------
        void do_chipTemp(char *word)
        {
                printf("Chip Temp = %s\r\n", decToStr(chipTemp(strToDec(line))));
        }
        */
        //--------------------------------------------------------------------------------
        // do_exp:  Set the exponential moving average.
        //   Do not allow an exponent of zero or more than 1000
        //   Format: exp <exp factor>
        //   Note that 1/<exp factor> is added into the moving average each sample
        //--------------------------------------------------------------------------------
        void do_exp(char *word)
        {
                int16_t new = 0;

                if (word != NULL)
                {
                        new = atoi(word);
                        if (new > 0 && new <= 1000)
                        {
                                fset.zero[0] = (fset.zero[0] * new) / fset.exp;
                                fset.zero[1] = (fset.zero[1] * new) / fset.exp;
                                fset.zero[2] = (fset.zero[2] * new) / fset.exp;
                                fset.exp = new;
                                writeSettings();
                        }
                        else printf("Invalid exp factor\r\n");
                }
        }

        //---------------------------------------------------------------------------------
```

```
// do_time:  Set the scan time from 1 to 3000 seconds.  Optionally set
//   the scan interval for 0.1 to 100.0 seconds.
//   Format: time <scan time> [<scan interval>] (seconds)
//-----------------------------------------------------------------------
void do_time(char *word)
{
        int16_t value;

        if (word != NULL)
        {
                value = atoi(word);
                if (value > 0 && value < 3000) fset.scanTime = value;
                word = strtok(NULL,delim);
                if (word != NULL)
                {
                        value = strToDec(word)/10;
                        if (value > 0 && value <= 100) fset.interval = value;
                }
                writeSettings();
        }
}


//-----------------------------------------------------------------------
// do_info:  Display settings and calibration constants
//   Format: info
//-----------------------------------------------------------------------
void do_info(char *word)
{
        printf("Scan time = %d sec\r\n", fset.scanTime);
        printf("Scan interval = %s\r\n", declToStr(fset.interval));
        printf("Exponential averaging = %d\r\n", fset.exp);
        printf("Upstream sensor enabled = %c\r\n", (fset.mode&UPSTREAM) ? 'y' :
'n');
//      printf("Low flow threshold <= %s ml/hr\r\n", decToStr(fset.calLo));
}


//-----------------------------------------------------------------------
// do_help: Prints a list of commands
//   Format: help
//-----------------------------------------------------------------------
void do_help(char *word)
{
        int i;

        for (i = 0; commands[i].func != NULL; ++i)
        {
                printf("%s\t%s\r\n", commands[i].cmd, commands[i].desc);
        }
}


//-----------------------------------------------------------------------
// decToStr:  Converts decimal number with two places to a string
//   Returns ptr to STATIC string
//-----------------------------------------------------------------------
char *decToStr(int16_t value)
{
        char istr[VSTRLEN];

        if (value < 0) snprintf(istr, VSTRLEN,"-%d", abs(value/100));
        else snprintf(istr, VSTRLEN, "%d", value/100);
        value = abs(value % 100);
```

```
        if (value < 10) snprintf(decstr,VSTRLEN,"%s.0%d", istr, value);
        else snprintf(decstr,VSTRLEN,"%s.%d", istr, value);
        return decstr;
}

//------------------------------------------------------------------------
// decToStr:  Converts decimal number with one place to a string
//  Returns ptr to STATIC string
//------------------------------------------------------------------------
char *dec1ToStr(int16_t value)
{
        char istr[VSTRLEN];

        if (value < 0) snprintf(istr, VSTRLEN,"-%d", abs(value/10));
        else snprintf(istr, VSTRLEN, "%d", value/10);
        snprintf(decstr,VSTRLEN,"%s.%d", istr, abs(value % 10));
        return decstr;
}


//------------------------------------------------------------------------
// strToDec:  Converts a decimal string with two decimal places to an
//  integer of the form value*100.
//------------------------------------------------------------------------
int16_t strToDec(char *str)
{
        int16_t intg = 0;
        uint16_t frac = 0;
        char sdec[3] = "00";
        char *decptr;

        intg = atoi(str) * 100;
        decptr = strchr(str,'.');
        if (decptr != NULL)
        {
                if (decptr[1] != '\0')
                {
                        sdec[0] = decptr[1];
                        if (decptr[2] != '\0') sdec[1] = decptr[2];
                        frac = atoi(sdec);
                }
        }
        return (intg+frac);
}

//------------------------------------------------------------------------
// getInput0:  Reads a line from UART0 and fills a string
//  Implements backspace correctly.  Adds a CRLF to output.
//------------------------------------------------------------------------
void getInput0(char *line, int maxlen)
{
  char ch;
  int i = 0;

  line[0] = '\0';
   while (1)
  {
        ch = getchar();
     if (ch == '\r' || ch == '\n' || ch == 0x1b || ch == '\0' )
     {
                putchar ('\r');
                putchar ('\n');
```

```
               line[i] = '\0';
               return;
          }
          if (ch == '\b')
          {
            if (i > 0)
            {
                putchar('\b');
                putchar(' ');
                putchar('\b');
                --i;
            }
          }
          else if (i < maxlen-3)
          {
            putchar(ch);
            line[i] = ch;
            ++i;
          }
        }
    }


    //------------------------------------------------------------------------
    // getInput:  Reads a line from UART0 or UART1 and fills a string
    //    Implements backspace correctly.  Adds a CRLF to output.
    //------------------------------------------------------------------------
    void getInput(char *line, int maxlen)
    {
          if (uartSelect == 1) getInput1(line, maxlen);
          else getInput0(line, maxlen);
    }


    //----------------------------------------------------------------------
    // getchar0: Inputs a single character from UART0
    //----------------------------------------------------------------------
    int getchar0(void)
    {
          while (!(IFG1 & URXIFG0))/* if (sleepTimer == 0) return '\0'*/;
          return RXBUF0;
    }


    //----------------------------------------------------------------------
    // getchar: Inputs a single character from UART0 or UART1
    //----------------------------------------------------------------------
    int getchar(void)
    {
          if (uartSelect == 1) return getchar1();
          else return getchar0();
    }


    //----------------------------------------------------------------------
    // putchar0: Output a single character on UART0
    //----------------------------------------------------------------------
    int putchar0(int ch)
    {
          while (!(IFG1 & UTXIFG0));          // USART TX buffer ready?
          TXBUF0 = ch;                        // TX character
          return 0;
    }

    //----------------------------------------------------------------------
```

```
// putchar: Output a single character on UART0 or UART1
//---------------------------------------------------------------
int putchar(int ch)
{
        if (uartSelect == 1)
        {
                putchar1(ch);
                putchar0(ch);
        }
        else putchar0(ch);
        return 0;
}


//---------------------------------------------------------------
// kbhit0:  Returns 1 if character waiting at UART0
//---------------------------------------------------------------
int kbhit0(void)
{
        return (IFG1 & URXIFG0);
}


//---------------------------------------------------------------
// kbhit:  Returns 1 if character waiting at UART0 or UART1
//---------------------------------------------------------------
int kbhit(void)
{
        if (uartSelect == 1) return kbhit1();
        return (IFG1 & URXIFG0);
}


//---------------------------------------------------------------
// printf: redirected to UART0
//   The library version of printf() does not use putchar() so it is
//   not enough to just redefine putchar().
//---------------------------------------------------------------
int printf(const char *fmt, ...)
{
        char buf[60], *p;
        va_list ap;

        va_start(ap, fmt);
        vsnprintf(buf, sizeof(buf), fmt, ap);
        for (p = buf; *p; ++p)
        {
                putchar(*p);
        }
        va_end(ap);
        return 0;
}
/*
//---------------------------------------------------------------
// parse:  Copy first word of a string to a new string and then
//   remove it from the original string.
//   word must be same length as line to avoid overwriting
//---------------------------------------------------------------
void parse(char *line, char *word)
{
        int i, j;

        word[0] = '\0';
```

```c
        // strip off leading blanks and tabs and commas
        for (i = 0; line[i] != '\0' && (line[i] == ' '
                    || line[i] == '\t' || line[i] == ',' || line[i] == '\n');
++i);
        if (line[i] == '\0') return;

        // copy non-blank chars to word
        for (j = 0; line[i] != '\0' && line[i] != ' ' && line[i] != '\t'
                && line[i] != ','; ++i, ++j) word[j] = line[i];
        word[j] = '\0';

        // move up remaining chars
        for (j = 0; line[j] != '\0'; ++j, ++i) line[j] = line[i];
        line[j] = '\0';
}
*/
/*
 * comm.h
 *
 *  Created on: Feb 1, 2013
 *      Author: Tom
 */

#ifndef COMM_H_
#define COMM_H_

#define STRLEN  40                              // maximum string length
#define VSTRLEN 10                              // maximum number string length

struct cmd_table
{
        char *cmd;                              // command name
        char *desc;                             // command description for help
        void (*func)(char *);                   // pointer to command handler
};


// Function prototypes

void cmdHandler(void);
uint32_t inpNum(char *prompt, uint32_t min, uint32_t max);
void getInput(char *line, int maxlen);
int kbhit(void);
void do_calibrate(char *word);
void do_meas(char *word);
void do_scan(char *word);
void do_help(char *word);
void do_info(char *word);
void printDecimal(int16_t value);
void do_exp(char *word);
void do_time(char *word);
void do_delCal(char *word);
void do_zero(char *word);
void do_adc(char *word);
void do_chipTemp(char *word);
void do_disp(char *word);
void do_calLo(char *word);
void do_upstream(char *word);
char *decToStr(int16_t value);
char *dec1ToStr(int16_t value);
void printADC(char chan);
```

```
//void parse(char *line, char *word);
void do_heat(char *word);
void do_comm(char *word);
void do_term(char *word);
int putchar0(int ch);
int getchar0(void);
int kbhit0(void);

#endif /* COMM_H_ */

//----------------------------------------------------------------------
// main.c
//   Vivonics Thermal Flow Sensor Implant
//   Version: 2.2  - October 2013
//   Author:  Tom Russell
//
//   Control program for flow sensor.
//   v2.1 - add feature for single thermistor operation
//   v2.12 - correct strToDec function
//   v2.13 - really corrected strToDec function
//   v2.20 - removed parse function and use strtok to save stack space
//          made dec to str functions return same global str
//          printf needs at least 120 bytes - allocate 200 bytes for stack
//----------------------------------------------------------------------


#include <stdio.h>
#include <stdlib.h>
#include "main.h"
#include "comm.h"
#include "measure.h"
#include "uart1.h"

// Settings to be saved and read from flash
// exp = 20, time = 30, interval = 0.5s
fset_t fset = {0x55, 1, 20, 30, 5, -26975, 0, 0, 0, 0,/* 200, */{0}};

// ADC variables set in interrupts
volatile int32_t avg[3];                        // averaged adc counts
volatile int16_t adc[3];                        // adc counts
volatile uint16_t ms10 = 0;                     // 10 ms up counter
volatile bool enableAveraging = 1;          // 1 = exponential averaging
enabled
//volatile int16_t sleepTimer = SLEEP_TIME;     // sleep timer for keypad
timeout

bool idle = 0;                                     // 1 = all
temperatures the same

int main(void)

{
    initialize();
    _stack = STACKCHECK;
    LED_ON();
    msdelay(1000);
    LED_OFF();
    readSettings();
    msdelay(50);
    autoBaud();          // send character for auto baud adjustment

    // Print welcome message to each of the UART ports
```

```
uartSelect = 1;
printf ("\r\nVivonics Thermal Flow Implant v2.20\r\n");
printf("I> ");

// ADCs are sampled in background so loop here waiting for a user command
RX_Ready();          // start software UART
while (1)
{
  if (_stack != STACKCHECK)
  {
          printf("*STACK OVERFLOW*\r\n");
          abort();
  }
  cmdHandler();
}
}

//----------------------------------------------------------------------------
// initialize:  Initialize ports, USART and ADC
//----------------------------------------------------------------------------
void initialize(void)
{
        int i;

        // Set DCO for calibrated 8MHz
        WDTCTL = WDTPW + WDTHOLD;                    // Stop WDT
        if (CALBC1_8MHZ ==0xFF || CALDCO_8MHZ == 0xFF)
        {
                while(1);                           // If calibration
constants erased
                                                    // do not load, trap CPU!!
        }
        // MCLK = 1MHz
        BCSCTL1 = CALBC1_8MHZ + DIVA_1;             // Set DCO and DIVA to
divided ACLK by 2....
        DCOCTL = CALDCO_8MHZ;
        BCSCTL2 |= DIVS_3 + DIVM_3;                 // MCLK  = SMCLK =
DCOCLK/8 = 1MHz
//      BCSCTL2 |= DIVS_3 + DIVM_2;                 // SMCLK = DCOCLK/8 =
1MHz, MCLK = 2 MHz

        // Wait for DCO to settle
        do
        {
                IFG1 &= ~OFIFG;                     // Clear OSCFault flag
                for (i = 0x47FF; i > 0; i--);       // Time for flag to set
        }
        while ((IFG1 & OFIFG));                     // OSCFault flag still set?

        // Setup Port 1 and 2
        P1_INIT();
        P2_INIT();

        // Setup flash memory timing generator for flash write
        FCTL2 = FWKEY + FSSEL0 + FN4+FN3;           // MCLK/24 = 333kHz (MCLK =
8MHz)

        // Setup USART0 for 115k baud
        ME1 |= UTXE0 + URXE0;                       // Enable USART0 TXD/RXD
        U0CTL |= CHAR;                              // 8-bit character
        U0TCTL |= SSEL1;                            // UCLK= SMCLK
```

```
        UOBR0 = 0x08;                               // 1MHz 115200
        UOBR1 = 0x00;                               // 1MHz 115200
        UOMCTL = 0x6d;                              // 1MHz 115200 modulation
        UOCTL &= ~SWRST;                            // Initialize USART state
machine
//      IE1 |= URXIE0;                              // Enable USART0 RX interrupt


        // Setup Timer A0 to count us and interrupt every 10ms
        TACTL = TASSEL_2 + MC_2;                    // SMCLK, contmode
        CCTL0 = CCIE;                               // CCR0 interrupt enabled


        // Initialize SD24 for conversion of all 3 channels
        // Two's complement output, interrupt on done, 1024 oversampling
        // Clock speed = 250kHz based on maximum source resistance of 40k
        // See datasheet settling time.  Sample rate is 4ms
        SD24CTL = SD24REFON+SD24SSEL_1+SD24DIV_2;       // 1.2V ref, SMCLK/4
        SD24CCTL0 |= SD24GRP+SD24DF+SD24OSR_1024;   // Group with CH1
        SD24CCTL1 |= SD24GRP+SD24DF+SD24OSR_1024;   // Group with CH2
        SD24CCTL2 |= SD24IE+SD24DF+SD24OSR_1024;    // Enable interrupt


#ifdef THERMISTOR                                   // Thermistors do
not use PGA
        SD24CTL |= SD24VMIDON;                       // Turn on external
VREF
#else
        SD24INCTL0 = SD24GAIN_32;                    // PGA Gain 32
        SD24INCTL1 = SD24GAIN_32;                    // PGA Gain 32
        SD24INCTL2 = SD24GAIN_32;                    // PGA Gain 32
#endif
        msdelay(10);                                 // Delay for
1.2V ref startup
        SD24CCTL2 |= SD24SC;                         // Set bit to start
conversion

        // Enable interrupts
        TAR = 0;                                     // Initialize
timer A
        CCR0 = 10000;                                //
        __bis_SR_register(GIE);                      // Enable all
interrupts
}

//------------------------------------------------------------------------
// usdelay:  Delays for given number of us (max = 65000)
//------------------------------------------------------------------------
void usdelay(uint16_t us)
{
        uint16_t start = TAR;
        while (TAR - start < us);
}

//------------------------------------------------------------------------
// msdelay:  Delays for given number of ms (max = 65000)
//------------------------------------------------------------------------
void msdelay(uint16_t ms)
{
        uint16_t start = TAR;
        while (ms > 0)
        {
                while (TAR - start < 1000);
                start += 1000;
```

```
            --ms;
        }
    }

    //-----------------------------------------------------------------------
    // writeSettings:  Write values to flash.
    //   Flash INFO segments are only 64 bytes long so split the data into 2
    segments.
    //-----------------------------------------------------------------------
    void writeSettings(void)
    {
        writeSegment((char *)0x1040, (char *)&fset, 64);
        writeSegment((char *)0x1080, ((char *)&fset)+64, sizeof(fset)-64);
    }

    //-----------------------------------------------------------------------
    // writeSegment:  Write values to flash segment.
    //   Length should not be longer than 64 bytes for info segments
    //-----------------------------------------------------------------------
    void writeSegment(char *Flash_ptr, char *data, int length)
    {
    //   char *Flash_ptr;                            // Flash pointer

    //   Flash_ptr = (char *)0x1040;                 // Initialize Flash pointer
      FCTL3 = FWKEY;                                 // Clear Lock bit
      FCTL1 = FWKEY + ERASE;                         // Set Erase bit
      *Flash_ptr = 0;                                // Dummy write to erase segment

      FCTL1 = FWKEY + WRT;                           // Set WRT bit for write operation
      for (; length > 0; --length)
          *Flash_ptr++ = *data++;

      FCTL1 = FWKEY;                                 // Clear WRT bit
      FCTL3 = FWKEY + LOCK;                          // Set LOCK bit
    }

    //-----------------------------------------------------------------------
    // readSettings:  Read values from flash in segment C & D into RAM
    //-----------------------------------------------------------------------
    void readSettings(void)
    {
        fset_t *Flash_ptr = (fset_t *)0x1040;                       // location
in flash
        if (Flash_ptr->flag != 0x55) writeSettings();
        fset = *Flash_ptr;
    }

    //-----------------------------------------------------------------------
    // SD24AISR:  Interrupt for SD24 configured for continuous conversion
    //   Read conversion results and save in global variables.
    //-----------------------------------------------------------------------
    #pragma vector=SD24_VECTOR
    __interrupt void SD24AISR(void)
    {
        switch (SD24IV)
        {
          case 2:                                    // SD24MEM Overflow
            break;
          case 4:                                    // SD24MEM0 IFG
            break;
          case 6:                                    // SD24MEM1 IFG
```

```
            break;
         case 8:                                            // SD24MEM2 IFG
            adc[0] = SD24MEM0;
            adc[1] = SD24MEM1;
            adc[2] = SD24MEM2;
            break;
    //            P1OUT ^= BIT0;                             // debug
      }
    }


    //-------------------------------------------------------------------------
    // TIMERA0ISR:  Interrupt for Timer A0
    //   Interrupt every 10 ms and increment counters
    //   Add to the exponential moving average for each channel and control LED
    //   Allow software UART to interrupt to avoid timing errors
    //-------------------------------------------------------------------------
    #pragma vector=TIMERA0_VECTOR
    __interrupt void TIMERA0ISR(void)
    {
         int i;
    //       static uint8_t heat = 0;
         static uint8_t ledcnt = 0;

         // Disable SD24 interrupts and re-enable global interrupt
         // so that only software UART can interrupt
         SD24CCTL2 &= ~SD24IE;
         __bis_SR_register(GIE);                            // Enable all interrupts

         CCR0 += 10000;                                     // Add 10ms offset
    to CCR0
         ++ms10;                                            // Increment
    10 ms counter
    //       if (sleepTimer != 0) --sleepTimer;
    /*
         // Heater control
         if (heaterPower != 0 && heat % heaterPower == 0) HEAT_ON();
         else HEAT_OFF();
         ++heat;
    */
         if (enableAveraging)
         {
              for (i = 0; i < 3; ++i)
              {
                   avg[i] += ((int32_t)fset.exp*adc[i] - avg[i])/fset.exp;
              }
         }

         // Flash LED every second
         LED_OFF();
         if (++ledcnt >= 100)
         {
              LED_ON();
              ledcnt = 0;
         }

         // disable GIE again and re-enable SD24
         __bic_SR_register(GIE);                            // Disable all interrupts
         SD24CCTL2 |= SD24IE;
    }

    // Unused interrupts
```

```
#pragma vector=USART0RX_VECTOR
__interrupt void USART0_RX (void)
{
}

/* defined in msp430 software uart
#pragma vector=TIMERA1_VECTOR
__interrupt void TIMERA1ISR(void)
{
}
*/

#pragma vector=USART0TX_VECTOR
__interrupt void USART0TXISR(void)
{
}

#pragma vector=PORT1_VECTOR
__interrupt void PORT1ISR(void)
{
}


#pragma vector=PORT2_VECTOR
__interrupt void PORT2ISR(void)
{
}

#pragma vector=WDT_VECTOR
__interrupt void WDTISR(void)
{
        while(1);
}


#pragma vector=NMI_VECTOR
__interrupt void NMIISR(void)
{
        while(1);
}
/*
 * main.h
 *
 *  Created on: Jan 31, 2013
 *       Author: Tom
 */

#include <msp430.h>

#ifndef MAIN_H_
#define MAIN_H_

struct bits8 {
        unsigned int b0 : 1;
        unsigned int b1 : 1;
        unsigned int b2 : 1;
        unsigned int b3 : 1;
        unsigned int b4 : 1;
        unsigned int b5 : 1;
        unsigned int b6 : 1;
```

```c
        unsigned int b7 : 1;
};
#define SFR8BIT(x) ((volatile struct bits8 *)(&x))

// Implement a bool type extension
#define false 0
#define true 1
#define bool char


// Variable types for MSP430
typedef unsigned long uint32_t;
typedef long int32_t;
typedef unsigned int uint16_t;
typedef int int16_t;
typedef unsigned short uint8_t;
typedef short int8_t;

typedef struct flowCal_s {
        int16_t flow;                             // flow rate in
ml/hr * 100
        int16_t heat;                             // heater delta t
in deg C * 100
//      int16_t down;                             // downstream delta
t in deg C * 100
} flowCal_t;

#define MAX_CAL_TABLE 20        // max entries in flow cal table

typedef struct fset_s
{
        char flag;                                // set to 0x55 to
indicate flash written
        char mode;                                // 0x01 = use
upstream sensor
        int16_t exp;                              // exponential
averaging factor
        int16_t scanTime;                         // default scan time in
sec
        int16_t interval;                         // scan time interval in
sec * 10
        int16_t chipTempOff;                      // chip temp calibration
offset
        int32_t zero[3];                          // zero channel offsets
        int16_t calEntries;                       // number of
entries in cal table
//      int16_t calLo;                            // threshold for
low flow regime ml/hr*100
        flowCal_t cal[MAX_CAL_TABLE];        // flow calibration table
} fset_t;

#define UPSTREAM 0x01                             // bit 0 in mode

#define SLEEP_TIME            3000

// PORT 1
#define HEAT     0x01                             // P1.0 = heat on
#define RX2      0x02                             // P1.1 = RX2
#define RX2A     0x04                             // P1.2 = RX2
alternate
#define TX       0x08                             // P1.3 = TX
#define RX       0x10                             // P1.4 = RX
```

```
#define P1NC        0x60                              // P1.5,P1.6 = no
connect
#define TX2         0x80                              // P1.7 = TX2

// select UART for TX and RX, select CCI1B for RX2, TA1 OUT for TX2
// enable pullup resistor on RX so not floating when disconnected
#define P1_INIT() { P1OUT = RX; P1REN = RX; P1DIR = HEAT+P1NC+TX+TX2; P1SEL =
TX+RX+TX2+RX2; P1SEL2 = TX2; }
//#define P1_INIT() { P1OUT = 0; P1DIR = HEAT+P1NC+TX+TX2; P1SEL = TX+RX; }

#define     HEAT_ON()   (P1OUT |= 0x01)              // turn heat on
#define HEAT_OFF()      (P1OUT &= ~0x01)          // turn heat off
#define IS_HEAT_ON() (P1OUT & 0x01)

// Port 2
// Note P2.6, P2.7 default to crystal so need to change P2SEL bit
#define TX2A       0x01                             // P2.0 = TX2
alternate
#define LED        0x40                             // P2.6 = LED

#define P2_INIT() { P2OUT = 0; P2DIR = ~TX2A; P2SEL = 0; }

#define LED_OFF() (P2OUT &= ~LED)
#define LED_ON() (P2OUT |= LED)
#define LED_TOG() (P2OUT ^= LED)

#define TX2_ON() (TACCTL1 = OUT);                   // TX2 is TA1 OUT
#define TX2_OFF() (TACCTL1 = 0);                    //

// Globals

extern fset_t fset;
extern volatile uint16_t ms10;                      // 10 ms counter
extern volatile int16_t adc[3];                     // adc counts
extern volatile int32_t avg[3];                     // average adc with
exp fact
extern volatile bool enableAveraging;               // user abort
extern bool idle;
extern uint8_t uartSelect;                          // uart port number

extern long long _stack;
#define STACKCHECK 0x5555555555555555

// Function prototypes

void initialize(void);
void msdelay(uint16_t ms);
void usdelay(uint16_t ms);
int16_t strToDec(char *str);
void readSettings(void);
void writeSettings(void);
void writeSegment(char *Flash_ptr, char *data, int length);
void adcPower(bool enable);

// Compile flags
// uncomment next line for thermistor inputs
#define THERMISTOR

#ifdef REMOTE_IO
#define putchar putchar1
#define getchar getchar1
```

```
#define kbhit kbhit1
#define printf printf1
#endif


#endif /* MAIN_H_ */
/*
 * measure.c
 *
 *  Created on: Feb 1, 2013
 *      Author: Tom
 */

#include <stdio.h>
#include <stdlib.h>
#include "main.h"
#include "measure.h"
#include "comm.h"

//#define MAX_CAL_TABLE 10
//struct flowCal calTable[MAX_CAL_TABLE];

//uint16_t calEntries = 0;                        // number of entries in
cal table
//uint16_t calTime = 100;                         // calibrated scans are
100 sec
int16_t coldComp = 0;                            // cold junction
compensation in counts
const int16_t steady = 10;                       // steady state is less
than 1 deg/sec
const int16_t chipTempGain = 72;                 // counts per 100 deg C
(.00132/(0.6V/32768))
const int16_t countsPerDegC = 64;                // type T counts per deg C at
gain 32


    //--------------------------------------------------------------------
    // chanADC:  Return the ADC counts for the channel
    //  <chan> = 0, 1 or 2 with no test for out of range.
    //--------------------------------------------------------------------
    int16_t chanADC(char chan)
    {
        // counts was scaled up by expFact to avoid truncation errors during
averaging
        return (int16_t)((avg[chan] - fset.zero[chan])/fset.exp);
    }

    //--------------------------------------------------------------------
    // chanTemp:  Convert ADC counts to temperature in degrees C * 100
    //  VREF---40k---THERM---GND
    //               |       |
    //               V+      V-
    //--------------------------------------------------------------------
    int16_t chanTemp(char chan)
    {
        const int16_t mintemp = 1100;            // 11C

        // Thermistor lookup table based on 1.2V ref
        // SEMITEC 223Fu3122 + 40k resistor
        // measured across thermistor which decreases resistance with
temperature
```

```
        // 11C - 59C
        const int16_t tempTable[] =
        {

            32268,31570,30878,30192,29514,28843,28180,27525,26879,26242,25614,24996,2
388,23790,23203,

            22626,22059,21503,20959,20425,19902,19390,18889,18399,17920,17452,16994,1
548,16112,15686,15271,

            14866,14472,14087,13712,13347,12991,12645,12308,11979,11660,11349,11047,
                10753,10467,10188,9918,9655,9399,
                -32768
        };

        int i;
        int16_t temp;
        int16_t counts;

        // counts was scaled up by expFact to avoid truncation errors during
averaging
            counts = (avg[chan] - fset.zero[chan])/fset.exp;

        // Check for counts out of range
        if (counts >= tempTable[0]) return mintemp;

        // Loop through table of counts to find the temperature
        // Temperature is given in degC * 100
        for (i = 0; counts < tempTable[i]; ++i);
        temp = (int32_t)100*(tempTable[i-1] - counts)/(tempTable[i-1] -
tempTable[i]);
        temp += (i - 1)*100 + mintemp;

        return temp;
    }


    //----------------------------------------------------------------------
    // scanTemp:  Scans for given time and prints temperatures.
    //   Start at -10 seconds and when 0 seconds is reached, turn on heat and
    //   zero the delta temperatures.  When done, return the avg delta temperatures
    //   scanReady() should be called before calling scanTemp().
    //----------------------------------------------------------------------
    flowCal_t scanTemp(int16_t sec, bool silent)
    {
//      int i;
        uint16_t now;                               // interval timer
        int16_t timeStamp = -50;            // time stamp in 0.1 sec units
        int16_t heatZero = 0/*, downZero = 0*/;
        int32_t heatAvg = 0;                // summing register for heat
//      int32_t downAvg = 0;                // summing register for down
        flowCal_t cal;                          // flow calibrate structure

        cal.flow = 0;
        printf("TIME\tUP\tHEAT\r\n");
        now = ms10;
        sec *= 10;
        // Print the temperature every scan interval seconds
        while (!kbhit())
        {
```

```
                idle = tempEven();
                if (fset.mode&UPSTREAM)  cal.heat = chanTemp(1) - chanTemp(0) -
        heatZero;
                else cal.heat = chanTemp(1) - heatZero;
                // At time stamp 0 zero the temperature differences and turn on
        heat
                if (timeStamp == 0)
                {
                        heatZero = cal.heat;
        //              downZero = cal.down;
                        cal.heat/* = cal.down*/ = 0;
        //              heaterPower = 1;
                        HEAT_ON();
                        LED_ON();
                }
        //      else if (timeStamp >= sec / 2) heaterPower = 0;
                // Keep track of sum of the temperature differences for averaging
                // Only average second half of time interval because starting is
                // similar
                if (timeStamp > 0)
                {
                        heatAvg += cal.heat;
        //              downAvg += cal.down;
        //              if (heaterPower > 1 && cal.heat < 150)
        //              {
        //                      printf("Full power\r\n");
        //                      heaterPower = 1;
        //              }

                }
                printf("%s\t", dec1ToStr(timeStamp));
                printf("%s\t", decToStr(chanTemp(0)));
                printf("%s\r\n", decToStr(cal.heat));
        //      printf("%s\r\n", decToStr(cal.down));
                if (timeStamp >= sec) break;

                while (now - ms10 < fset.interval*10);   // Wait for interval
                timeStamp += fset.interval;              // Increment time stamp
                now += fset.interval*10;                      // increment for
        next interval
            }
            // Compute averages and turn off the heat
            cal.heat = heatAvg * fset.interval / timeStamp;
        //  cal.down = downAvg * fset.interval / timeStamp;
        //  heaterPower = 0;
            HEAT_OFF();
            LED_OFF();
            if (kbhit())
            {
                    getchar();
                    cal.flow = -1;
                    printf("Aborted\r\n");
            }
            else
            {
                    if (fset.mode&UPSTREAM) printf("\r\nAverage Heater - Upstream");
                    else printf("\r\nAverage Heater Rise");
                    printf(" = %s\r\n", decToStr(cal.heat));
            }
            return cal;
        }
```

```
//-----------------------------------------------------------------------
// scanReady:  Returns false if not ready to start scanning due to
 // temperature difference in sensors.  Also update cold junction comp.
//-----------------------------------------------------------------------
bool scanReady(void)
{
//      chipTemp(0);
        msdelay(500);
        if (!tempEven())
        {
                printf("Warning - temperature sensors not within +/- 0.5C\r\n");
                return 0;
        }
        return 1;
}


//-----------------------------------------------------------------------
// tempEven:  Return 1 if both sensors are within 0.5C
//-----------------------------------------------------------------------
bool tempEven(void)
{
        return (chanTemp(1) - chanTemp(0)) <= 50;
}


//-----------------------------------------------------------------------
// addCal:  Adds a calibration point to the calibration table.
//   Entries are sorted by flow rate.
//-----------------------------------------------------------------------
void addCal(flowCal_t calpt)
{
        int i, j;

        if (fset.calEntries >= MAX_CAL_TABLE-1) return;

        // Locate the insertion point in the table
        for (i = 0; i < fset.calEntries; ++i)
        {
                if (calpt.flow <= fset.cal[i].flow) break;
        }
        // If rate already exists or at end of table, just write it
        if (i >= fset.calEntries)
        {
                ++fset.calEntries;
        }
        else if (calpt.flow < fset.cal[i].flow)
        {
                if (i >= MAX_CAL_TABLE-1)
                {
                        printf("Calibration table is full\r\n");
                        return;
                }
                // Move entries up to make room for new entry
                for (j = fset.calEntries; j >= i; --j)
                {
                        fset.cal[j+1] = fset.cal[j];
                }
                ++fset.calEntries;
        }
        fset.cal[i] = calpt;
```

```
        if (fset.calEntries == 0) fset.calEntries = 1;
}


//------------------------------------------------------------------------
// delCal:  Delete a calibration entry
//  If flowRate < 0 delete the whole table
//------------------------------------------------------------------------
void delCal(int16_t flowRate)
{
        int i, j;

        // If flow rate < 0 delete the whole table
        if (flowRate < 0) fset.calEntries = 0;
        else
        {
                // Locate the entry to delete
                for (i = 0; i < fset.calEntries; ++i)
                {
                        if (flowRate == fset.cal[i].flow) break;
                }
                if (i >= fset.calEntries)
                {
                        printf("Entry not found\r\n");
                        return;
                }
                // move up the entries to fill the gap
                for (j = i; j < fset.calEntries-1; ++j)
                {
                        fset.cal[j] = fset.cal[j+1];
                }
                --fset.calEntries;
        }
}


//------------------------------------------------------------------------
// calibrate: Perform a flow calibration
//  flowrate = ml/hr * 100
//------------------------------------------------------------------------
void calibrate(int16_t flowRate)
{
        flowCal_t new;

        new = scanTemp(fset.scanTime, 1);
        if (new.flow != -1)
        {
                new.flow = flowRate;
                addCal(new);
        }
}


//------------------------------------------------------------------------
// measureFlow: Make a flow rate measurement
//  Return:  flow rate in ml/hr * 100
//------------------------------------------------------------------------
int16_t measureFlow(bool silent)
{
        flowCal_t meas;
        int i;
        int32_t interp;

        // check to make sure cal table has an entry for no flow
```

```
if (fset.cal[0].flow != 0 || fset.calEntries == 0)
{
        printf("Calibration table has no 0 ml/hr entry\r\n");
        return -1;
}

// perform a temperature scan and abort if it was interrupted
meas = scanTemp(fset.scanTime, silent);
if (meas.flow == -1) return -1;

// Start at the highest flow rate and search down until the measured temp
// rise is greater than in the table.  Interpolate between values.

printf("Flow search:");
for (i = fset.calEntries-1; i >= 0; --i)
{
        printf(" %d", i);
        if (meas.heat <= fset.cal[i].heat)
        {
                if (i == fset.calEntries-1) return fset.cal[i].flow;
                else
                {
                        interp = 1000*(int32_t)(meas.heat - fset.cal[i+1].heat)
                                / (fset.cal[i].heat - fset.cal[i+1].heat);
                        return fset.cal[i+1].flow
                                - (interp * (fset.cal[i+1].flow -
fset.cal[i].flow)/1000);
                }
        }
}
return 0;

/*
// Start at the highest flow rate and search down until the measured temp
// rise is greater than in the table.  Interpolate between values.
// Do not exceed the temperature rise of 0 flow as this may produce
// ambiguous results.
if (fset.calLo != 0)
{
        if (meas.heat + 50 < fset.cal[0].heat)
        {
                printf("Flow search:");
                for (i = fset.calEntries-1; i >= 0; --i)
                {
                        printf(" %d", i);
                        if (meas.heat <= fset.cal[i].heat)
                        {
                                if (i == fset.calEntries-1) return
fset.cal[i].flow;
                                else
                                {
                                        interp = 1000*(int32_t)(meas.heat -
fset.cal[i+1].heat)
                                                / (fset.cal[i].heat -
fset.cal[i+1].heat);
                                        return fset.cal[i+1].flow
                                                - (interp * (fset.cal[i+1].flow -
fset.cal[i].flow)/1000);
                                }
                        }
                }
```

```
                }
                printf("\r\nlo flow search:");
                // Flow was not found so search using downstream temp starting from
no flow
                // Do not exceed a flow rate of 2 ml/hr (geometry dependent)
                for (j = 0; j <= fset.calEntries-1 && fset.cal[j].flow <=
fset.calLo; ++j)
                {
                        printf(" %d", j);
        //              if (j > 1 && fset.cal[j-1].heat > meas.heat) break;
                        if (meas.down <= fset.cal[j].down)
                        {
                                if (j == 0) return 0;
                                else
                                {
                                        interp = 1000*(int32_t)(fset.cal[j].down -
meas.down)
                                                / (fset.cal[j].down - fset.cal[j-1].down);
                                        return fset.cal[j].flow
                                                - (interp * (fset.cal[j].flow - fset.cal[j-
1].flow)/1000);
                                }
                        }
                }
        printf("\r\nhi flow search:");
        // Go back to searching from high to low flow rates using the heater temp
        for (i = fset.calEntries-1; i >= 0; --i)
        {
                printf(" %d", i);
                if (meas.heat <= fset.cal[i].heat)
                {
                        if (i == fset.calEntries-1) return fset.cal[i].flow;
                        else
                        {
                                interp = 1000*(int32_t)(meas.heat - fset.cal[i+1].heat)
                                        / (fset.cal[i].heat - fset.cal[i+1].heat);
                                return fset.cal[i+1].flow
                                        - (interp * (fset.cal[i+1].flow -
fset.cal[i].flow)/1000);
                        }
                }
        }
        printf("\r\nCan't find flow rate in table\r\n");
        return -1;
*/
}


//---------------------------------------------------------------------------
// zeroADC:  Record the ADC values while both thermistors are at the
//   same temperature and save the offset
//---------------------------------------------------------------------------
void zeroADC(void)
{
        fset.zero[0] = fset.zero[1] = fset.zero[2] = 0;

        printf("Make sure both thermistors are at the\r\n");
        printf("same temperature and press enter...");
        if (getchar() == 0x1b) return;
        printf("\r\n");
```

```
        msdelay(10000);
        fset.zero[1] = avg[1] - avg[0];
//      fset.zero[2] = avg[2] - avg[0];
        writeSettings();
    }


    //----------------------------------------------------------------------
    // inVoltage:  measure the input voltage and return as dec2 number
    //   assumes adc is on
    //----------------------------------------------------------------------
    uint16_t inVoltage(void)
    {
        uint32_t temp;

        temp = 1313L * fset.exp;
        temp = avg[2] * 100L / temp;
        return (uint16_t)temp;
    }
    /*
    //----------------------------------------------------------------------
    // chipTemp:  Returns the internal temperature of the microcontroller.
    //  Format is degrees C * 100 (2315 = 23.15C)
    //----------------------------------------------------------------------
    int16_t chipTemp(int16_t cal)
    {
        uint8_t saveChan;
        int32_t chipT = 0;
        int i;

        // Turn off exponential averaging of ADC channels
        enableAveraging = 0;
        // Switch channel 2 to internal temperature sensor
        saveChan = SD24INCTL2;
        SD24INCTL2 = SD24INCH_6;
        for (i = 0; i < 100; ++i)
        {
            msdelay(5);
            chipT += adc[2];
        }
        SD24INCTL2 = saveChan;
        msdelay(100);
        enableAveraging = 1;
        // If a value is given, compute the offset to calibrated the internal
sensor
        if (cal != 0)
        {
            fset.chipTempOff = cal - chipT/chipTempGain;
            writeSettings();
        }
        chipT = chipT/chipTempGain + fset.chipTempOff;
        // Set the cold junction compensation for the thermocouples
        coldComp = chipT;
        return (int16_t)chipT;
    }
    */



    /*
     * measure.h
     *
```

```
 *   Created on: Feb 1, 2013
 *       Author: Tom
 */

#ifndef MEASURE_H_
#define MEASURE_H_

// Public globals
extern const int16_t countsPerDegC;
extern uint8_t heaterPower;

// Function prototypes

flowCal_t scanTemp(int16_t sec, bool silent);
int16_t countsToTemp(int32_t counts);
int16_t flowCalIndex(int16_t flow);
int16_t measureFlow(bool silent);
void calibrate(int16_t flowRate);
int calComp(const void *a, const void *b);
void delCal(int16_t flowRate);
void zeroADC(void);
int16_t chipTemp(int16_t cal);
int16_t chanTemp(char chan);
int16_t chanADC(char chan);
bool scanReady(void);
void addCal(flowCal_t calpt);
bool tempEven(void);
uint16_t inVoltage(void);

#endif /* MEASURE_H_ */
```

```
************************************************************************
          *
          *                    MSP430 CODE EXAMPLE DISCLAIMER
          *
          * MSP430 code examples are self-contained low-level programs that typically
          * demonstrate a single peripheral function or device feature in a highly
          * concise manner. For this the code may rely on the device's power-on default
          * register values and settings such as the clock configuration and care must
          * be taken when combining code from several examples to avoid potential side
          * effects. Also see www.ti.com/grace for a GUI- and www.ti.com/msp430ware
          * for an API functional library-approach to peripheral configuration.
          *
          * --/COPYRIGHT--*/
//****************************************************************************
//   MSP430x21x1 Demo - Timer_A UART 9600, 1MHz DCO SMCLK
//
//   Description: This program demonstrates a full-duplex 9600-baud UART using
//   Timer_A3 and the DCO. A character is echoed on the Hyperterminal of a
//   a PC. The DCO frequency settings are stored in INFOA flash segment.
//   ACLK = n/a, MCLK = SMCLK = saved DCO 1Mhz
//   //* External watch crystal installed on XIN XOUT is required for ACLK *//
//
//                  MSP430F21x1
//                 -----------------
//         /|\|                XIN|-
//          | |                   |
//          --|RST           XOUT|-
//            |                   |
//            |           P1.3|--------> Power for MAX3221
//            |   CCI0A/TXD/P1.1|-------->
//            |                 | 9600 8N1
//            |   CCI0B/RXD/P2.2|<--------
//
//   L. Westlund / A. Dannenberg
//   Texas Instruments, Inc
//   July 2005
//   Built with CCE Version: 3.2.0 and IAR Embedded Workbench Version: 3.30A
//****************************************************************************

// Modified for mps430afe253 by Tom Russell for thermal sensor implant only
// Note that this is a half duplex UART where send and receive are on the same
line
// Transmit polarity is inverted because of transistor output
// Extra time added between transmit bytes to allow for capacitor recharge

// 2400 BAUD
// TX on P1.7 (OUT1)
// RX on P1.1 (CCI1B)
// Timer A1 interrupt


#include <msp430.h>
#include <stdio.h>
#include <stdlib.h>
#include <string.h>

#include "main.h"
#include "uart1.h"
#include "comm.h"
```

```
// Define TX and RX in main.h  must select alternate functions for
capture/compare
// Timer A must be configured for 1MHz


// Uncomment next line for IRDA inverted transmit and receive
// In IRDA each bit is divided into a pulse and then return to "zero"
// This avoids the implant losing power for too long which can cause
// it to reboot, but more likely causes rounding of the bits as current
// needs to flow to recharge the DC input capacitor
// Transmitter should select 1/4 bit time pulse width


#define IRDA
/*
//    Conditions for 2400 Baud SW UART, SMCLK = 1MHz
#define Bitime_recv_offset   208                        // ~ 0.5 bit length
#define Bitime_phase_0 208                              // ~0.5 bit time
#define Bitime_phase_1 209                              // remainder of bit time
#define Bitime     417                      // ~ 2398 baud
*/


// 1200 baud
// transmit from remote to implant = 1/4 bit time
//  (longest bit time available on the SI32 controller)
// transmit from implant to remote = 1/2 bit time (still works)
#ifdef IRDA
#define Bitime_recv_offset   105               // ~ 1/8 bit length
#else
#define Bitime_recv_offset   417               // ~ 0.5 bit length
#endif
#define Bitime_phase_0   417                           // 1/2 bit time
#define Bitime_phase_1 417                             // remainder of bit time
#define Bitime     834                     // ~ 1200 baud

volatile uint16_t RXTXData;                       // RXTX shift register
volatile uint8_t BitCnt;                          // RXTX bit counter
volatile char RXbuf = 0;                          // received character
volatile uint8_t TXphase = 0;               // bit phase for TX RZ
volatile bool RXbusy = 0;                         // 1 = receiving a char


//-------------------------------------------------------------------------
// autoBaud:  Sends a series of 0x00 for IRDA or 0x55 for non-IRDA
//   so remote can fine tune baud rate
//-------------------------------------------------------------------------
void autoBaud(void)
{
        int i;

        for (i = 0; i < 5; ++i)
        {
#ifdef IRDA
                putchar1(0);
#else
                putchar1(0x55);
#endif
                msdelay(10);
        }
}


//-------------------------------------------------------------------------
// getchar1: Inputs a single character from software UART
```

```
//  Once a character is read, buffer is cleared and interrupts
//  enabled to receive next character.
//------------------------------------------------------------
int getchar1(void)
{
      int ch;

      while (RXbuf == 0);                           // wait for a character
      ch = RXbuf;
      RXbuf = 0;
      RX_Ready();
      return ch;
}


//------------------------------------------------------------
// putchar1: Output a single character on software UART1
//------------------------------------------------------------
int putchar1(int ch)
{
      .RXTXData = ch;
      TX_Byte();                                    // returns when complete
//    putchar0(ch);                                 // debug echo to UART0
      return 0;
}


//------------------------------------------------------------
// kbhit1:  Returns waiting character or 0 but does not empty buffer
//------------------------------------------------------------
int kbhit1(void)
{
      return RXbuf;            // return non-zero if character in buffer
}
/*
//------------------------------------------------------------
// printf: redirected to UART0
// The library version of printf() does not use putchar() so it is
//  not enough to just redefine putchar().
//------------------------------------------------------------
int printf1(const char *fmt, ...)
{
      char buf[60], *p;
      va_list ap;

      va_start(ap, fmt);
      vsnprintf(buf, sizeof(buf), fmt, ap);
      for (p = buf; *p; ++p)
      {
            putchar1(*p);
      }
      va_end(ap);
      return 0;
}
*/
//------------------------------------------------------------
// getInput1:  Reads a line from UART1 and fills a string
//    Implements backspace correctly.  Adds a CRLF to output.
//    ECHO characters to UART0 since UART1 may be busy
//------------------------------------------------------------
void getInput1(char *line, int maxlen)
{
    char ch;
```

```
    int i = 0;

    line[0] = '\0';
     while (1)
    {
        ch = getchar1();
        if (ch == '\r' || ch == '\n' || ch == 0x1b || ch == '\0' )
        {
        putchar0('\r');
        putchar0('\n');
        putchar1('\r');
            putchar1('\n');
            line[i] = '\0';
            return;
        }
        if (ch == '\b')
        {
          if (i > 0)
          {
                putchar0('\b');
                putchar0(' ');
                putchar0('\b');
                putchar1('\b');
                putchar1(' ');
                putchar1('\b');
                --i;
          }
        }
        else if (i < maxlen-3)
        {
          putchar0(ch);
          putchar1(ch);
          line[i] = ch;
          ++i;
        }
    }
}

//-------------------------------------------------------------------------------
// TX_Byte:  Function Transmits Character from RXTXData Buffer
// On each compare match in interrupt, output a single bit
// Modified from sample code to invert bit sense
// Added a little extra delay for first bit
//-------------------------------------------------------------------------------
void TX_Byte (void)
{
        // Provide a mechanism for interrupt
        // If remote turns off power for > 20ms treat that as an escape received
/*
        if ((P1IN & RX2) == 0)
        {
                msdelay(10);
                if ((P1IN & RX2) == 0)
                {
                        RXbuf = 0x1b;
                        return;
                }
        }
*/
        while (RXbusy);
        TACCTL0 &= ~CCIE;                        // disable other interrupts
```

```
        BitCnt = 10;                         // Load Bit counter, 8data + ST/SP
        TACCR1 = TAR;                         // Current state of TA counter
        TACCR1 += Bitime*2;                   // Some time till first bit
        TXphase = 0;                                   // Reset phase for RZ
format
        RXTXData |= 0x100;                    // Add mark stop bit to RXTXData
        RXTXData = RXTXData << 1;             // Add space start bit
        TACCTL1 = OUTMOD0 + OUTMOD2 + CCIE;   // TXD = mark = idle (inverted)
        while ( TACCTL1 & CCIE);              // Wait for TX completion
        if (RXbuf == 0) RX_Ready();                // Ready for RX if last char
read
}


//------------------------------------------------------------------------------
// RX_Ready: Readies UART to Receive Character into RXTXData Buffer
// Sync capture not possible as DCO=TACLK=SMCLK can be off !!
// Do not set OUTMOD0 because need to leave output as low, not high
//------------------------------------------------------------------------------
void RX_Ready (void)
{
        BitCnt = 0x8;                                         // Load Bit counter
        TACCTL1 = CM1 + CCIS0/* + OUTMOD0*/ + CAP + CCIE;    // Neg Edge, Cap
}



//------------------------------------------------------------------------------
// Timer A1 interrupt:  Transmit and receive is done in this interrupt
//   Bit sense has been inverted from sample code
//------------------------------------------------------------------------------
// Timer A1 interrupt service routine
#pragma vector=TIMERA1_VECTOR
__interrupt void Timer_A (void)
{
        if (TAIV == 2)                                            // need to
check to reset interrupt flag
        {
                TACCTL0 &= ~CCIE;                                // disable other
interrupts
//              TACCR1 += Bitime;                       // Add Offset to CCR1

                // RX:  On first falling edge capture, switch from capture to
compare
                //      and then sample on each compare match.  Note the the SCCI
bit
                //      contains the input value latched on compare match
                if (TACCTL1 & CCIS0)                            // RX on CCI0B?
                {
                        RXbusy = 1;
                        TACCR1 += Bitime;                               // Add Offset to CCR1
                        if( TACCTL1 & CAP )                     // Capture mode
start bit edge
                        {
                                TACCTL1 &= ~ CAP;                       // Switch from
capture to compare mode
                                TACCR1 += Bitime_recv_offset;
                                //      _BIC_SR_IRQ(SCG1 + SCG0);               // DCO
reamins on after reti
                        }
                        else
                        {
```

```
                        RXTXData = RXTXData >> 1;
                        if (TACCTL1 & SCCI)                  // Get bit waiting
in receive latch
                                RXTXData |= 0x80;
                        BitCnt --;                           // All bits RXed?
                        if (BitCnt == 0)
                                //>>>>>>>>>> Decode of Received Byte Here
<<<<<<<<<<<<<<<<<<<<<<<<<<<<<<<<<
                                {
                                        RXbuf = RXTXData;
//
Save received char
                                        TACCTL1 = 0;
//                              .       TACCTL1 &= ~ CCIE;                   // All
bits RXed, disable interrupt
                                        TACCTL0 |= CCIE;
//
Enable timer A0 interrupt
                                        RXbusy = 0;
                                }
                                //>>>>>>>>>> Decode of Received Byte Here
<<<<<<<<<<<<<<<<<<<<<<<<<<<<<<<<
                        }
                }
                // TX:  Transmit next bit on each compare
                else
                {
                        if (BitCnt == 0)
                        {
                                TACCTL1 = 0;

        // leave in off state (same as stop bit)
//                              TACCTL1 &= ~ CCIE;                   // All bits
TXed, disable interrupt
                                TACCTL0 |= CCIE;
//
Enable timer A0 interrupt
                        }
                        else
                        {
#ifdef IRDA
                                if (TXphase == 0)

            // after phase 0, return to RESET
                                {
                                        TACCR1 += Bitime_phase_1;
//
Add Offset to CCR1
                                        if (RXTXData & 0x01) TACCTL1 |= OUTMOD2;
//
inverted from sample code
                                        else TACCTL1 &= ~OUTMOD2;
                                        TXphase = 1;
                                }
                                else
                                {
                                        TACCR1 += Bitime_phase_0;

            // after phase 1 is next bit
                                        TACCTL1 |= OUTMOD2;
                                        RXTXData = RXTXData >> 1;
```

```
                                        BitCnt --;
                                        TXphase = 0;
                        }
        #else
                        TACCR1 += Bitime;
        //
        Add Offset to CCR1
                        if (RXTXData & 0x01) TACCTL1 |= OUTMOD2;          //
        inverted from sample code
                        else TACCTL1 &= ~OUTMOD2;
                        RXTXData = RXTXData >> 1;
                        BitCnt --;
        #endif
                }
            }
        }
    }
// uart1.h

void TX_Byte(void);
void RX_Ready(void);

int getchar1(void);
int putchar1(int ch);
int kbhit1(void);
void getInput1(char *line, int maxlen);
void autoBaud(void);
```

REMOTE SOURCE CODE

```
#include "gModes.h"

#include <SI32_PBCFG_A_Type.h>
#include <SI32_PBSTD_A_Type.h>
#include <si32_device.h>

#include <cr_section_macros.h>
#include <stdio.h>

#include "main.h"
#include "myPB.h"
#include "myCPU.h"
```

```c
#include "myTIMER0.h"
#include "mySARADC1.h"
#include "myUART0.h"
#include "myFLASHCTRL0.h"
#include "myPCA0.h"

// Globals
fset_t fset = {0x5555, 100.0, 2.2};

//-----------------------------------------------------------------------
// My application.
// Thermal Sensor Wireless Remote
// Version 1.0
// 10/22/13
// Tom Russell - tcrussell@ieee.org  908-578-4615
//-----------------------------------------------------------------------
int main(void)
{

    //    SI32_UART_A_enable_rx_irda_mode(SI32_UART_1);
    //    SI32_UART_A_enable_rx_signal_inversion(SI32_UART_1);

    // Enter the default operating mode for this application
    enter_default_mode_from_reset();

    read_settings();

    led(GREEN);
    delay_ms(500);
    led(RED);
    delay_ms(500);
    led(GREEN);
    delay_ms(500);
    led(RED);

    printf("\r\nVivonics Wireless Remote v1.0\r\nR> ");
    delay_ms(1000);
    vboost(0);                      // don't use booster - too much noise
    vbusHighCurrent(1);
    setFreq(fset.freq);
 //   setFreq(140);
  // do_on("");
    while (1)
    {
        cmdHandler();
    }
}


#include <stdbool.h>
#include <stdint.h>

typedef struct
{
    uint16_t flashInit;         // set to 0x5555 if inited
    float freq;                 // oscillator frequency in kHz
    float senseRes;             // sense resistor
} fset_t;

extern fset_t fset;
// Copyright (c) 2013
```

```
#include "myCLKCTRL.h"
// Copyright (c) 2013

#ifndef __MYCLKCTRL_H__
#define __MYCLKCTRL_H__

#include "gCLKCTRL.h"


#endif //__MYCLKCTRL_H__
// Copyright (c) 2013

#include "myCMP0.h"

// Copyright (c) 2013

#ifndef __MYCMP0_H__
#define __MYCMP0_H__

#include <stdbool.h>

// INCLUDE GENERATED CONTENT
#include "gCMP0.h"


#endif //__MYCMP0_H__
// Copyright (c) 2013

#include "myCPU.h"
#include <si32_device.h>

#include <SI32_WDTIMER_A_Type.h>

void mySystemInit(void)
{
   SI32_WDTIMER_A_stop_counter(SI32_WDTIMER_0);
}


//------------------------------------------------------------------------------
// delay_ms:  Delays for <delay> milliseconds
//   Maximum delay = 65536 ms
//------------------------------------------------------------------------------
void delay_ms(uint32_t delay)
{
      uint32_t now;

      now = get_msTicks();
      while (get_msTicks() - now < delay);
}
// Copyright (c) 2013

#ifndef __MYCPU_H__
#define __MYCPU_H__

#include "gCPU.h"
void delay_ms(uint32_t delay);

#endif //__MYCPU_H__
// Copyright (c) 2012
```

```
//library
#include <stdbool.h>
#include <stdio.h>

// hal
#include <si32_device.h>
#include <SI32_FLASHCTRL_A_Type.h>
#include <SI32_PBSTD_A_Type.h>
#include <SI32_VMON_A_Type.h>
#include <SI32_RSTSRC_A_Type.h>

// application
#include "main.h"
#include "myFLASHCTRL0.h"
#include "myCpu.h"

#define FLASH_PAGE_ADDR  0x0F000              // user flash data address
(@60k)
#define FLASH_PAGES      1                             // 1 pages of 1024
bytes each

//--------------------------------------------------------------------------
// write_settings:  Writes settings to flash
//--------------------------------------------------------------------------
void write_settings(void)
{
        myFLASHCTRL0_erase_page(0, 0);
        myFLASHCTRL0_run_write_flash_mode(0, (uint16_t *)&fset,
((sizeof(fset))/2)+1);
}

//--------------------------------------------------------------------------
// read_settings:  Read settings from flash.  If flash not initialized,
//    write the default settings.
//--------------------------------------------------------------------------
void read_settings(void)
{
        fset_t *fptr = (fset_t *)FLASH_PAGE_ADDR;
        if (fptr->flashInit != 0x5555) write_settings();
        else fset = *fptr;
}

//==========================================================================
//MODE FUNCTIONS
//==========================================================================
//--------------------------------------------------------------------------
// myFLASHCTRL0_run_erase_page_mode:
//    This function erases the flash pages in the range of start_page to
//    end_page.  Each page is 1024 bytes and the first page is page 0 and
located
//    at FLASH_PAGE_ADDR
//--------------------------------------------------------------------------
bool myFLASHCTRL0_erase_page(uint16_t start_page, uint16_t end_page)
{
        uint32_t i;

        if (end_page < start_page || end_page >= FLASH_PAGES) return 0;

        // 1. Enable VDD Supply Monitor and set as a reset source
        SI32_VMON_A_enable_vdd_supply_monitor(SI32_VMON_0);
        SI32_RSTSRC_A_enable_vdd_monitor_reset_source(SI32_RSTSRC_0);
```

```
        for(i = start_page; i <= end_page; i++)
        {
            // 2. Write the address of the Flash page to WRADDR
            SI32_FLASHCTRL_A_write_wraddr(SI32_FLASHCTRL_0, FLASH_PAGE_ADDR +
(i*1024));

            // 3. Enter Flash Erase Mode
            SI32_FLASHCTRL_A_enter_flash_erase_mode(SI32_FLASHCTRL_0);

            // 4. Disable Interrupts
            __disable_irq();

            // 5. Write the inital unlock value to KEY
            SI32_FLASHCTRL_A_write_flash_key(SI32_FLASHCTRL_0, 0xA5);

            // 6. Write the single unlock value to KEY
            SI32_FLASHCTRL_A_write_flash_key(SI32_FLASHCTRL_0, 0xF1);

            // 7. Write any value to WRDATA in right-justified format to
            //     initiate the page erase
            SI32_FLASHCTRL_A_write_wrdata(SI32_FLASHCTRL_0, 0x0000);

            // 8. (optional) poll BUSYF if executing code from other than Flash
Memory
            // We are executing code from Flash, so no need to poll.

            // 9. Enable Interrupts
            __enable_irq();
        }
    /*
    #ifdef DEBUG
        // Debug Print message to indicate completion
        printf("\r\n%d flash page(s) at address 0x%x erased.\r\n", start_page,
FLASH_PAGE_ADDR );
    #endif
    */
        return 1;
    }

    //----------------------------------------------------------------------
    // myFLASHCTRL0_run_write_flash_mode:
    // This function writes an array of data of <num_bytes> located at *ptr
    //  to the specified page of flash data.  The start of the user flash area
    //  is given in FLASH_PAGE_ADDR and each page is 1024 bytes.
    //----------------------------------------------------------------------
    bool myFLASHCTRL0_run_write_flash_mode(uint16_t start_page,
                    uint16_t *ptr, uint32_t num_uint16)
    {
        uint32_t i;
        uint32_t start_addr;

        if (start_page >= FLASH_PAGES) return 0;
        start_addr = FLASH_PAGE_ADDR + (start_page*1024);

        // 1. Enable VDD Supply Monitor and set as a reset source
        SI32_VMON_A_enable_vdd_supply_monitor(SI32_VMON_0);
        SI32_RSTSRC_A_enable_vdd_monitor_reset_source(SI32_RSTSRC_0);

        // 2. Disable Flash Erase Operations
        SI32_FLASHCTRL_A_exit_flash_erase_mode(SI32_FLASHCTRL_0);
```

```
                // Write all the half-words in the array
                for(i = 0; i < num_uint16; i++)
                {
                    // 3. Write the address of the half-word to WRADDR
                    SI32_FLASHCTRL_A_write_wraddr(SI32_FLASHCTRL_0, start_addr + (2 * i));

                    // 4. Disable Interrupts
                    __disable_irq();

                    // 5. Write the inital unlock value to KEY
                    SI32_FLASHCTRL_A_write_flash_key(SI32_FLASHCTRL_0, 0xA5);

                    // 6. Write the single unlock value to KEY
                    SI32_FLASHCTRL_A_write_flash_key(SI32_FLASHCTRL_0, 0xF1);

                    // 7. Write the data to WRDATA in right-justified format to
                    //    initiate the write
                    SI32_FLASHCTRL_A_write_wrdata(SI32_FLASHCTRL_0, ptr[i] );

                    // 8. (optional) poll BUSYF if executing code from other than Flash
Memory
                    // We are executing code from Flash, so no need to poll.

                    // 9. Enable Interrupts
                    __enable_irq();

                }
        /*
        #ifdef DEBUG
            // Print message to indicate completion
            printf("\r\n%d bytes of data at address 0x%x written.\r\n", num_uint16*2,
FLASH_PAGE_ADDR );
        #endif
        */
            return 1;
        }
        // Copyright (c) 2012

        #ifndef __MYFLASHCTRL0_H__
        #define __MYFLASHCTRL0_H__


        // INCLUDE GENERATED CONTENT
        #include "gFLASHCTRL0.h"


        void write_settings(void);
        void read_settings(void);

        bool myFLASHCTRL0_run_write_flash_mode(uint16_t start_page,
                        uint16_t *ptr, uint32_t num_uint16);
        bool myFLASHCTRL0_erase_page(uint16_t start_page, uint16_t end_page);

        #endif //__MYFLASHCTRL0_H__

    // Copyright (c) 2013

        #include "myIDAC0.h"

        // Copyright (c) 2013
```

```
#ifndef __MYIDAC0_H__
#define __MYIDAC0_H__

#include <stdbool.h>

// INCLUDE GENERATED CONTENT
#include "gIDAC0.h"


#endif //__MYIDAC0_H__
// Copyright (c) 2013


// Include peripheral access modules used in this file
#include <SI32_PBCFG_A_Type.h>
#include <si32_device.h>
#include <SI32_PBSTD_A_Type.h>
#include <SI32_PBHD_A_Type.h>

#include "myPB.h"

//-----------------------------------------------------------------------------
// vboost:  Turn on or off the DC-DC boost converter (PB0.11)
//-----------------------------------------------------------------------------
void vboost(bool enable)
{
      if (enable) SI32_PBSTD_A_write_pins_high(SI32_PBSTD_0,1<<11);
      else SI32_PBSTD_A_write_pins_low(SI32_PBSTD_0,1<<11);
}


//-----------------------------------------------------------------------------
// vbusHighCurrent:  Turn on the PMOS transistor to allow high current to
//  flow from USB.  Off during startup to avoid current spike as capacitors
//  charge.  Uses high drive port 4 which is setup for 5V.  (PB4.3)
//-----------------------------------------------------------------------------
void vbusHighCurrent(bool enable)
{
      if (enable) SI32_PBHD_A_write_pins_low(SI32_PBHD_4,1<<3);
      else SI32_PBHD_A_write_pins_high(SI32_PBHD_4,1<<3);
}


//-----------------------------------------------------------------------------
// led:  Turn LED RED, GREEN or OFF (PB4.0, PB4.1)
//-----------------------------------------------------------------------------
void led(int color)
{
      if (color == RED)
      {
            SI32_PBHD_A_write_pins_high(SI32_PBHD_4,1<<1);
            SI32_PBHD_A_write_pins_low(SI32_PBHD_4,1<<0);
      }
      else if (color == GREEN)
      {
            SI32_PBHD_A_write_pins_low(SI32_PBHD_4,1<<1);
            SI32_PBHD_A_write_pins_high(SI32_PBHD_4,1<<0);
      }
      else
      {
            SI32_PBHD_A_write_pins_low(SI32_PBHD_4,1<<0);
            SI32_PBHD_A_write_pins_low(SI32_PBHD_4,1<<1);
```

```
        }
    }
    // Copyright (c) 2013

    #ifndef __MYPB_H__
    #define __MYPB_H__

    #include "gPB.h"

    #define OFF 0
    #define RED 1
    #define GREEN 2

    void vboost(bool enable);
    void vbusHighCurrent(bool enable);
    void led (int color);

    #endif //__MYPB_H__
    // Copyright (c) 2013

    // Include peripheral access modules used in this file
    #include <SI32_PCA_A_Type.h>
    #include <si32_device.h>
    #include <SI32_PCACH_A_Type.h>

    #include "myPCA0.h"
    #include "myCPU.h"


    //------------------------------------------------------------------------
    // setFreq:  Sets the output frequency in units of kHz.  Returns
    // frequency in kHz
    //------------------------------------------------------------------------
    float setFreq(float kHz)
    {
        uint32_t count;

        count = SystemPeripheralClock/(kHz*2000);
        if (count > 128) return 0.0;
        SI32_PCA_A_write_limit(SI32_PCA_0, count-1);
        return SystemPeripheralClock/count/2000;
    }
    // Copyright (c) 2013

    #ifndef __MYPCA0_H__
    #define __MYPCA0_H__

    #include <stdbool.h>

    // INCLUDE GENERATED CONTENT
    #include "gPCA0.h"

    float setFreq(float kHz);

    extern float oscFreq;

    #endif //__MYPCA0_H__
    //------------------------------------------------------------------------
    // mySARDAC1.c:  ADC routines for LC meter
    //
    // Notes on ADC:
    //  Interrupt is triggered on the end of any single measurement
```

```
//  Multiple channels can be scanned, end of scan indicated by 0x31 in timeslot
//  A single conversion can include a number of conversions which are summed
//  Burst mode track time is 64-count, so minimum is 64, not 0
//   This time is inserted in between every sample (x4 for 12 bit samples)
//  ADC has an internal REF (1.665V) but can also use VREF0 which is 2.4V
//    VREF0 is also tied to the external VREF pin when on
//--------------------------------------------------------------------------

//library
#include <stdbool.h>
#include <stdio.h>
#include <stdint.h>
// hal
#include <si32_device.h>
#include <SI32_SARADC_A_Type.h>
#include <SI32_PBSTD_A_Type.h>

// application
#include "mySARADC1.h"
#include "myTIMER0.h"
#include "myCpu.h"

// flag cleared inside conversion complete handler
uint32_t ADC_SCAN_DONE = 0;
uint8_t adc_chan = 0;

//=========================================================================
// 2nd Level Interrupt Handlers (Called from generated code)
//=========================================================================
void SARADC1_conv_complete_handler(void)
{
      SI32_SARADC_A_clear_single_conversion_complete_interrupt(SI32_SARADC_1);
//    SI32_SARADC_A_enable_burst_mode(SI32_SARADC_1);
      ADC_SCAN_DONE = 1;
}
void SARADC1_scan_done_handler(void)
{

SI32_SARADC_A_clear_single_conversion_complete_interrupt(SI32_SARADC_1);
      SI32_SARADC_A_clear_scan_done_interrupt(SI32_SARADC_1);
      SI32_SARADC_A_disable_autoscan(SI32_SARADC_1);
      SI32_SARADC_A_disable_accumulator(SI32_SARADC_1);
      ADC_SCAN_DONE = 1;

}

//--------------------------------------------------------------------------
// set_adc_chan:  Sets the adc channel for timeslot 0
//--------------------------------------------------------------------------
void set_adc_chan(uint8_t chan)
{
      SI32_SARADC_A_select_timeslot0_channel(SI32_SARADC_1, chan);
      adc_chan = chan;
}

//--------------------------------------------------------------------------
// read_adc:  Reads ADC channel previously selected by set_adc_chan()
//  The number of samples and the sample scan timing is set in gSARADC1.c
//  Current settings: 1us track time x 8 x 4 = 32 us tracking
//    8x4 readings of 1.3us each = 41.6us
//    total = 73.6us (measured 82 overall)
```

```
//     Values returned are 0 to 32767 based on 8 12 bit sample set in gSARDAC1.c
//-------------------------------------------------------------------------------
uint32_t read_adc(uint16_t samples)
{
        uint32_t adc_value = 0;
        uint16_t i;

        for (i = 0; i < samples; ++i)
        {
                ADC_SCAN_DONE = 0;
                // burst mode must be enabled before each conversion start
                // it is cleared by hardware after the burst mode conversion
completes
                SI32_SARADC_A_enable_burst_mode(SI32_SARADC_1);

                // a 1-to-0 transition on ACCMD bit will enable the accumulator for
the next conversion
                SI32_SARADC_A_enable_accumulator(SI32_SARADC_1);
                SI32_SARADC_A_clear_accumulator(SI32_SARADC_1);

                SI32_SARADC_A_start_conversion(SI32_SARADC_1);
                while (!ADC_SCAN_DONE);

                adc_value += SI32_SARADC_A_read_data(SI32_SARADC_1);
        }
        return (adc_value/samples);                          // maximum value 32767
}
// Copyright (c) 2013


#ifndef __MYSARADC1_H__
#define __MYSARADC1_H__

#include <stdbool.h>

// INCLUDE GENERATED CONTENT
#include "gSARADC1.h"

void set_adc_chan(uint8_t chan);
uint32_t read_adc(uint16_t samples);

// ADC channels on SiM3C144
#define REF_CHAN  4                      // reference level is ADC1.4 on pin
PB0.13
#define DATA_CHAN 5                      // data level is ADC1.5 on PB0.13

#endif //__MYSARADC1_H__
// Copyright (c) 2013

// hal
#include <si32_device.h>
#include <SI32_TIMER_A_Type.h>
#include <SI32_PBSTD_A_Type.h>

#include "myTIMER0.h"

//-------------------------------------------------------------------------------
// delay_us:  Delays for <delay> microseconds.
//    Timer 0 is clocked from prescaler AHB/2/10 = 1MHz.
//    It is configured as a 32 bit timer.
//    Maximum delay is 4e9 us = 4,294 sec = 71 min
//-------------------------------------------------------------------------------
```

```
void delay_us(uint32_t delay)
{
        uint32_t now;

//      delay *= 10;
        now = SI32_TIMER_A_read_count(SI32_TIMER_0);
        while (SI32_TIMER_A_read_count(SI32_TIMER_0) - now < delay);
}
// Copyright (c) 2013

#ifndef __MYTIMER0_H__
#define __MYTIMER0_H__

#include <stdbool.h>

// INCLUDE GENERATED CONTENT
#include "gTIMER0.h"

void delay_us(uint32_t delay);

#endif //__MYTIMER0_H__
// Copyright (c) 2013

// library
#include <stdio.h>
#include <stdlib.h>
#include <stdarg.h>
#include <string.h>

// hal
#include <si32_device.h>
#include <SI32_CLKCTRL_A_Type.h>
#include <SI32_UART_A_Type.h>
#include <SI32_PBSTD_A_Type.h>
#include <SI32_PCA_A_Type.h>
#include <SI32_PCACH_A_Type.h>

// application
#include "main.h"
#include "myUART0.h"
#include "myUART1.h"
#include "myPB.h"
#include "mySARADC1.h"
#include "myTIMER0.h"
#include "myFLASHCTRL0.h"
#include "myPCA0.h"
#include "myCPU.h"

// command table
static struct cmd_table commands[] =
{
        {"con", "connect to implant", do_echo},
        {"freq", "set oscillator freq (kHz)", do_freq},
        {"on", "implant power on", do_on},
        {"off","implant power off", do_off},
        {"baud","<baud rate> or blank for auto baud set", do_baud},
        {"adc", "report half bridge current", do_adc},
        {"res", "set sense resistor (ohms)", do_res},
        {"help", "this help screen", help},

        {"", "", NULL}
```

```
};

//------------------------------------------------------------------------------.
// cmdHandler:  Processes commands received from USB
//------------------------------------------------------------------------------.
void cmdHandler(void)
{
        char line[STRLEN+1];                      // input line
        char cmd[STRLEN+1] = "";                            // command
        int i;

        if (!kbhit()) return;

        getInput(line, STRLEN);
        if (sscanf(line,"%s", cmd) > 0)
        {
                for (i = 0; commands[i].func != NULL; ++i)
                {
                        if (strcmp(commands[i].cmd, cmd) == 0)
                        {
                                (commands[i].func)(line+strlen(commands[i].cmd));
                                break;
                        }
                }
                if (commands[i].func == NULL) printf("Invalid command\r\n");
        }
        printf("\r\nR> ");
}

//------------------------------------------------------------------------------
// help:  Display a list of all commands
//------------------------------------------------------------------------------
void help(char *line)
{
        int i, cnt;
        char cmd[STRLEN];

        cnt = sscanf(line, " %s", cmd);
        if (strcmp(cmd,".") == 0) cnt = 0;
        if (cnt <= 0) printf("\r\nCommands: \r\n");
        for (i = 0; commands[i].func != NULL; ++i)
        {
                if ((cnt == 1 && strcmp(commands[i].cmd,cmd) == 0) || cnt <= 0)
                {
                        if (cmd[0] == '.' || commands[i].cmd[0] != '.')
                                printf("%-8s %s\r\n", commands[i].cmd,
commands[i].desc);
                }
        }
}

//------------------------------------------------------------------------------
// do_echo:  Echo characters between UART0 and UART1
//------------------------------------------------------------------------------
void do_echo(char *line)
{
        char ch = 0;
//      char last = 0;

        printf("Press escape to exit\r\n");
        do_on(line);
```

```
        while (ch != 0x1b)                              // loop until ESC pressed
        {
                if (kbhit())
                {
                        ch = myUART0_get_char();
//                      if (ch == 0x1b && last == 0x1b) break;
//                      if (ch == 0x1b) escapeUART1();
                        myUART1_send_char(ch);
//                      last = ch;
//                      ch = 0;
                }
                else if (kbhit1())
                {
                        ch = myUART1_get_char();
                        myUART0_send_char(ch);
                }
        }
        do_off(line);
        printf("Implant power off\r\n");
}

//-----------------------------------------------------------------------
// do_freq:  Sets the frequency in kHz
//-----------------------------------------------------------------------
void do_freq(char *line)
{
        float newFreq;

        newFreq = setFreq(atof(line));

        if (newFreq != 0.0)
        {
                printf ("Oscillator set to %.1f kHz\r\n", newFreq);
                fset.freq = newFreq;
                write_settings();
        }
        else printf("Invalid frequency\r\n");

}

//-----------------------------------------------------------------------
// do_baud:
//-----------------------------------------------------------------------
void do_baud(char *line)
{
        uint16_t newBaud;

        newBaud = atoi(line);
        if (newBaud == 0)
        {
                // default baud rate
                SI32_UART_A_set_rx_baudrate(SI32_UART_1,
SystemPeripheralClock/16/1200 - 1);
                SI32_UART_A_set_tx_baudrate(SI32_UART_1,
SystemPeripheralClock/16/1200 - 1);
                do_off(line);           // turn off implant
                delay_ms(1000);         // wait one second
                do_on(line);            // turn on implant
                delay_ms(750);          // wait a bit since implant takes 1 sec to
start
```

```
        while (kbhit1()) myUART1_get_char();
        SI32_UART_A_enable_rx_autobaud(SI32_UART_1);
        delay_ms(500);
        while (kbhit1())  myUART1_get_char();
        do_off(line);            // turn off implant
    }
    else SI32_UART_A_set_rx_baudrate(SI32_UART_1,
SystemPeripheralClock/16/newBaud - 1);

    SI32_UART_A_set_tx_baudrate(SI32_UART_1, SI32_UART_1->BAUDRATE.RBAUD);
    printf("BAUD = %d\r\n", SystemPeripheralClock/16/(SI32_UART_1-
>BAUDRATE.RBAUD+1));
}


//---------------------------------------------------------------------
// do_on:  Enable power to implant
//---------------------------------------------------------------------
void do_on(char *line)
{
    SI32_PCA_A_start_counter_timer(SI32_PCA_0);
    led(GREEN);
}


//---------------------------------------------------------------------
// do_off:  Disable power to implant
//---------------------------------------------------------------------
void do_off(char *line)
{
    SI32_PCA_A_stop_counter_timer(SI32_PCA_0);
    led(RED);
}


//---------------------------------------------------------------------
// do_adc:  Report the current through the half-bridge
// VREF is VDD = 3.3V
//---------------------------------------------------------------------
void do_adc(char *line)
{
    float voltage;

    set_adc_chan(REF_CHAN);
    voltage = 3.3*read_adc(8)/32768.;
    printf("VREF = %.2f  Current = %.2f \r\n", voltage,
voltage/fset.senseRes);
}


//---------------------------------------------------------------------
// do_res:  Sets the value of the sense resistor
//---------------------------------------------------------------------
void do_res(char *line)
{
    float res;

    res = atof(line);
    if (res != 0.0)
    {
        fset.senseRes = res;
        write_settings();
    }
    printf("Sense resistor = %.2f ohms\r\n", fset.senseRes);
}
```

```c
//-----------------------------------------------------------------------
// getInput:  Reads a line from the serial port and fills a string
//   Implements backspace correctly.  Adds a CRLF to output.
//-----------------------------------------------------------------------
void getInput(char *line, int maxlen)
{
      char ch;
    int i = 0;

    while (1)
    {
        ch = myUART0_get_char();
        if (ch == '\r' || ch == '\n' || ch == '\0' )
        {
          putchar ('\r');
          putchar ('\n');
          line[i] = '\0';
          return;
        }
        if (ch == '\b')
        {
          if (i > 0)
          {
                putchar('\b');
                putchar(' ');
                putchar('\b');
                --i;
          }
        }
        else if (i < maxlen-3)
        {
          putchar(ch);
          line[i] = ch;
          ++i;
        }
    }
}


//-----------------------------------------------------------------------
// myUART0_printf: redirected to UART0
//-----------------------------------------------------------------------
int myUART0_printf(const char *fmt, ...)
{
      char buf[80], *p;
      va_list ap;
      va_start(ap, fmt);
      vsnprintf(buf, sizeof(buf), fmt, ap);
      for (p = buf; *p; ++p) myUART0_send_char(*p);
      va_end(ap);
      return 0;
}


//-----------------------------------------------------------------------
// myUART0_send_char:
// Outputs a single character to UART0.
//-----------------------------------------------------------------------
void myUART0_send_char(uint8_t val)
{
    // Block if the output buffer is full
    while (SI32_UART_A_read_tx_fifo_count(SI32_UART_0) >= 4);
```

```
      // Write character to the output buffer
      SI32_UART_A_write_data_u8(SI32_UART_0, val);
   }

   //-----------------------------------------------------------------------
   // myUART0_get_char:
   // Returns a single character received from UART0.
   // Note: This is a blocking function.
   //-----------------------------------------------------------------------
   uint8_t myUART0_get_char(void)
   {
      uint8_t val;

      // Block if input buffer is empty
      while (SI32_UART_A_read_rx_fifo_count(SI32_UART_0) == 0);

      // Read character from the input buffer
      val = SI32_UART_A_read_data_u8(SI32_UART_0);

      return val;
   }

   //-----------------------------------------------------------------------
   // kbhit:  Returns number of chars waiting in the RX FIFO
   //-----------------------------------------------------------------------
   uint8_t kbhit(void)
   {
         return SI32_UART_A_read_rx_fifo_count(SI32_UART_0);
   }


   // Copyright (c) 2013

   #ifndef __MYUART0_H__
   #define __MYUART0_H__

   #include <stdbool.h>

   // INCLUDE GENERATED CONTENT
   #include "gUART0.h"

   #define STRLEN    80                              // max input string
length

   struct cmd_table
   {
         char *cmd;                           // command name
         char *desc;                          // command description for help
         void (*func)(char *);        // pointer to command handler
   };


   void getInput(char *line, int maxlen);
   void myUART0_send_char(uint8_t val);
   uint8_t myUART0_get_char(void);
   uint8_t kbhit(void);
   int myUART0_printf(const char *fmt, ...);

   void cmdHandler(void);
   void help(char *line);
```

```
void do_echo(char *line);
void do_freq(char *line);
void do_on(char *line);
void do_off(char *line);
void do_adc(char *line);
void do_res(char *line);
void do_baud(char *line);


#endif //__MYUART0_H__
// Copyright (c) 2013

// library
#include <stdio.h>
#include <stdlib.h>
#include <stdarg.h>
#include <string.h>

// hal
#include <si32_device.h>
#include <SI32_CLKCTRL_A_Type.h>
#include <SI32_UART_A_Type.h>
#include <SI32_PBSTD_A_Type.h>
#include <SI32_PCA_A_Type.h>
#include <SI32_PCACH_A_Type.h>


#include "myUART1.h"
#include "myTimer0.h"
#include "myCPU.h"
#include "main.h"


//---------------------------------------------------------------------------
// escapeUART1:  Send escape sequence to implant
//---------------------------------------------------------------------------
void escapeUART1(void)
{
      SI32_PCA_A_stop_counter_timer(SI32_PCA_0);
      delay_ms(20);
      SI32_PCA_A_start_counter_timer(SI32_PCA_0);
      if (kbhit1()) myUART1_get_char();
}

//---------------------------------------------------------------------------
// getInput1:  Reads a line from UART1 and fills a string
//    Implements backspace correctly.  Adds a CRLF to output.
//    NO ECHO
//---------------------------------------------------------------------------
void getInput1(char *line, int maxlen)
{
      char ch;
   int i = 0;

   while (1)
   {
       ch = myUART1_get_char();
       if (ch == '\r' || ch == '\n' || ch == '\0' )
       {
//              putchar('\r');
//              putchar('\n');
```

```
//              line[i] = '\0';
          return;
        }
        if (ch == '\b')
        {
          if (i > 0)
          {
//                  putchar('\b');
//                  putchar(' ');
//                  putchar('\b');
              --i;
          }
        }
        else if (i < maxlen-3)
        {
//              putchar(ch);
          line[i] = ch;
          ++i;
        }
    }
}


//------------------------------------------------------------------
// myUART1_printf: redirected to UART1
//------------------------------------------------------------------
int myUART1_printf(const char *fmt, ...)
{
      char buf[80], *p;
      va_list ap;
      va_start(ap, fmt);
      vsnprintf(buf, sizeof(buf), fmt, ap);
      for (p = buf; *p; ++p) myUART1_send_char(*p);
      va_end(ap);
      return 0;
}


//-------------------------------------------------------------------
// myUART1_send_char:
// Outputs a single character to UART1.
//-------------------------------------------------------------------
void myUART1_send_char(uint8_t val)
{
  // Block if the output buffer is full
  while (SI32_UART_A_read_tx_fifo_count(SI32_UART_1) >= 4);

  // Write character to the output buffer
  SI32_UART_A_write_data_u8(SI32_UART_1, val);
}


//-------------------------------------------------------------------
// myUART1_get_char:
// Returns a single character received from UART1.
// Note: This is a blocking function.
//-------------------------------------------------------------------
uint8_t myUART1_get_char(void)
{
  uint8_t val;

  // Block if input buffer is empty
  while (SI32_UART_A_read_rx_fifo_count(SI32_UART_1) == 0);
```

```
    // Read character from the input buffer
    val = SI32_UART_A_read_data_u8(SI32_UART_1);

    return val;
}

//-----------------------------------------------------------------------
// kbhit1:  Returns number of chars waiting in the RX FIFO of UART1
//-----------------------------------------------------------------------
uint8_t kbhit1(void)
{
      return SI32_UART_A_read_rx_fifo_count(SI32_UART_1);
}
// Copyright (c) 2013

#ifndef __MYUART1_H__
#define __MYUART1_H__

#include <stdbool.h>

// INCLUDE GENERATED CONTENT
#include "gUART1.h"

int myUART1_printf(const char *fmt, ...);
void myUART1_send_char(uint8_t val);
uint8_t myUART1_get_char(void);
uint8_t kbhit1(void);
void escapeUART1(void);


#endif //__MYUART1_H__
// Copyright (c) 2013

#include "myVREF0.h"

// Copyright (c) 2013

#ifndef __MYVREF0_H__
#define __MYVREF0_H__

#include <stdbool.h>

// INCLUDE GENERATED CONTENT
#include "gVREF0.h"


#endif //__MYVREF0_H__
// Copyright (c) 2013

#include "myVREG0.h"

//========================================================================
// 2nd Level Interrupt Handlers (Called from generated code)
//========================================================================
void VREG0_vbus_invalid_handler(void)
{

}
// Copyright (c) 2013
```

```
#ifndef __MYVREG0_H__
#define __MYVREG0_H__

#include <stdbool.h>

// INCLUDE GENERATED CONTENT
#include "gVREG0.h"

void VREG0_vbus_invalid_handler(void);

#endif //__MYVREG0_H__
// Copyright (c) 2013

#include "myWDTIMER0.h"

// Copyright (c) 2013

#ifndef __MYWDTIMER0_H__
#define __MYWDTIMER0_H__

#include <stdbool.h>

// INCLUDE GENERATED CONTENT
#include "gWDTIMER0.h"


#endif //__MYWDTIMER0_H__
//-------------------------------------------------------------------------
// Copyright (c) 2010-2011 by Silicon Laboratories.  All rights reserved.
// The program contained in this listing is a proprietary trade secret of
// Silicon Laboratories, Austin, Texas, and is copyrighted under the
// United States Copyright Act of 1976 as an unpublished work, pursuant to
// Section 104 and Section 408 of Title XVII of the United States code.
// Unauthorized copying, adaptation, distribution, use, or display is
// prohibited by this law.
//
// Silicon Laboratories (si32) provides this software solely and exclusively
// for use on Silicon Laboratories' microcontroller products.
//
// This software is provided "as is".  No warranties, whether express, implied
// or statutory, including, but not limited to, implied warranties of
// merchantability and fitness for a particular purpose apply to this software.
// Silicon Laboratories shall not, in any circumstances, be liable for special,
// incidental, or consequential damages, for any reason whatsoever.
//-------------------------------------------------------------------------

// library
#include <stdio.h>
#include <stdlib.h>
#include <stdarg.h>
#include <string.h>
#include <si32_device.h>

//#include "globals.h"
#include "myUART0.h"

//-------------------------------------------------------------------------
// Precision32 Support Functions

int __sys_write(int iFileHandle, char *pcBuffer, int iLength)
{
```

```c
  int i;
  for (i = 0; i<iLength; i++)
  {
    myUART0_send_char(pcBuffer[i]); // print each character
  }
  return iLength;
}

int __sys_readc(void)
{
  int c;
  c = myUART0_get_char();
  return c;
}

//-eof----------------------------------------------------------------------


//--------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//--------------------------------------------------------------------------


//==========================================================================
// WARNING:
//
// This file is auto-generated by AppBuilder and should not be modified.
// Any hand modifications will be lost if the project is regenerated.
//==========================================================================

#include "gClkCtrl.h"
#include "gCPU.h"
#include <SI32_CLKCTRL_A_Type.h>
#include <si32_device.h>

//==========================================================================
// Configuration Functions
//==========================================================================

void CLKCTRL_enter_default_mode_from_reset(void)
{

   // Set system clock to AHB divider frequency
   SystemCoreClock = 20000000;

   // Set system peripheral clock to APB divider frequency
   SystemPeripheralClock = 20000000;
}

void CLKCTRL_setup_default_mode_clock_gates(void)
{
   SI32_CLKCTRL_A_enable_apb_to_modules_0(SI32_CLKCTRL_0,
                                   SI32_CLKCTRL_A_APBCLKG0_PB0 |
                                   SI32_CLKCTRL_A_APBCLKG0_UART0 |
                                   SI32_CLKCTRL_A_APBCLKG0_UART1 |
```

```
                                        SI32_CLKCTRL_A_APBCLKG0_PCA0  |
                                        SI32_CLKCTRL_A_APBCLKG0_TIMER0  |
                                        SI32_CLKCTRL_A_APBCLKG0_SARADC1  |
                                        SI32_CLKCTRL_A_APBCLKG0_CMP0  |
                                        SI32_CLKCTRL_A_APBCLKG0_IDAC0  |
                                        SI32_CLKCTRL_A_APBCLKG0_FLASHCTRL0);
   SI32_CLKCTRL_A_enable_apb_to_modules_1(SI32_CLKCTRL_0,
                                        SI32_CLKCTRL_A_APBCLKG1_MISC1  |
                                        SI32_CLKCTRL_A_APBCLKG1_MISC0);
}
//------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//------------------------------------------------------------------------


//========================================================================
// WARNING:
//
// This file is auto-generated by AppBuilder and should not be modified.
// Any hand modifications will be lost if the project is regenerated.
//========================================================================


#ifndef __GCLKCTRL_H__
#define __GCLKCTRL_H__


void CLKCTRL_enter_default_mode_from_reset(void);
void CLKCTRL_setup_default_mode_clock_gates(void);

#endif //__GCLKCTRL_H__
//------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//------------------------------------------------------------------------


//========================================================================
// WARNING:
//
// This file is auto-generated by AppBuilder and should not be modified.
// Any hand modifications will be lost if the project is regenerated.
//========================================================================

#include "gCMP0.h"
#include "gCPU.h"

// Include peripheral access modules used in this file
#include <SI32_CMP_A_Type.h>
#include <si32_device.h>
```

```
//================================================================
// Configuration Functions
//================================================================
void CMP0_enter_default_mode_from_reset(void)
{
    // No peripheral code is needed for this mode transition
}


//----------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//----------------------------------------------------------------


//================================================================
// WARNING:
//
// This file is auto-generated by AppBuilder and should not be modified.
// Any hand modifications will be lost if the project is regenerated.
//================================================================

#ifndef __GCMP0_H__
#define __GCMP0_H__

void CMP0_enter_default_mode_from_reset(void);


#endif //__GCMP0_H__
//----------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//----------------------------------------------------------------


//================================================================
// WARNING:
//
// This file is auto-generated by AppBuilder and should not be modified.
// Any hand modifications will be lost if the project is regenerated.
//================================================================

#include "gCPU.h"

#include <stdio.h>
#include <si32_device.h>

// MODULE VARIABLES
//----------------------------------------------------------------
//Variable that incrments 1000 times / second
volatile uint32_t msTicks;
```

```
// APB Clock Frequency in Hz
uint32_t SystemPeripheralClock;

//===========================================================================
// 2nd LEVEL  INTERRUPT HANDLERS
//===========================================================================
/*NONE*/

//===========================================================================
// 1st LEVEL  INTERRUPT HANDLERS
//===========================================================================
void SysTick_Handler(void)
{
    msTicks++;
    /*NO SECOND LEVEL HANDLER SPECIFIED*/
}

//---------------------------------------------------------------------------
void NMI_Handler(void)
{
    printf("NMI_Handler\n");
    /*NO SECOND LEVEL HANDLER SPECIFIED (halt USED)*/
    halt();
}

//---------------------------------------------------------------------------
void HardFault_Handler(void)
{
    printf("HardFault_Handler\n");
    /*NO SECOND LEVEL HANDLER SPECIFIED (halt USED)*/
    halt();
}

//---------------------------------------------------------------------------
void MemManage_Handler(void)
{
    printf("MemManage_Handler\n");
    /*NO SECOND LEVEL HANDLER SPECIFIED (halt USED)*/
    halt();
}

//---------------------------------------------------------------------------
void BusFault_Handler(void)
{
    printf("BusFault_Handler\n");
    /*NO SECOND LEVEL HANDLER SPECIFIED (halt USED)*/
    halt();
}

//---------------------------------------------------------------------------
void UsageFault_Handler(void)
{
    printf("UsageFault_Handler\n");
    /*NO SECOND LEVEL HANDLER SPECIFIED (halt USED)*/
    halt();
}


//===========================================================================
// CONFIGURATION FUNCTIONS
//===========================================================================
```

```
// Sets up systick
void cpu_update(void)
{
    set_ahb_clock(SystemCoreClock);

# if SI32_BASE_CPU_ARMV7M
    // set Priority for Cortex-M0 System Interrupts.
    NVIC_SetPriority(SysTick_IRQn, (1 << __NVIC_PRIO_BITS) - 1);
    // TBD the rest of them
# endif
}


//===================================================================================
// SUPPORT FUNCTIONS
//===================================================================================
void set_ahb_clock(uint32_t freq)
{
    // UPDATE SYSTICK
    if (SysTick_Config(freq / 1000))
    {
        printf("ERROR: SysTick_Config failed\n");
    }

    // UPDATE ITM DIVIDER
    *((uint32_t *) 0xE0040010) = ((50 * freq) / 20000000) - 1;
}// set_ahb_clock

//-----------------------------------------------------------------------------------
uint32_t get_msTicks(void)
{
    return msTicks;
}// get_msTicks

//-----------------------------------------------------------------------------------
void halt(void)
{
    printf("Halted\n");
    // Configurable Fault Status Register
    printf(" CFSR: 0x%08X\n", SCB->CFSR);
    // Hard Fault Status Register
    printf(" HFSR: 0x%08X\n", SCB->HFSR);
    // Debug Fault Status Register
    printf(" DFSR: 0x%08X\n", SCB->DFSR);
    // Memory Management Fault Address Register
    printf("MMFAR: 0x%08X\n", SCB->MMFAR);
    // Bus Fault Address register
    printf(" BFAR: 0x%08X\n", SCB->BFAR);
    //Auxiliary Fault Status Register
    printf(" AFSR: 0x%08X\n", SCB->AFSR);
    while(1);
}


//-eof----------------------------------------------------------------------------
//--------------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
```

```
// Original content and implementation provided by Silicon Laboratories
//---------------------------------------------------------------------------

//==========================================================================
// WARNING:
//
// This file is auto-generated by AppBuilder and should not be modified.
// Any hand modifications will be lost if the project is regenerated.
//==========================================================================

#ifndef __GCPU_H__
#define __GCPU_H__

#include <stdint.h>

// MODE FUNCTIONS
void cpu_update(void);

// SUPPORT FUNCTIONS
void set_ahb_clock(uint32_t freq);
uint32_t get_msTicks(void);
void halt(void);

// NVIC table location
# define si32McuOption_map_vectors_to_ram 0

// APB Clock Frequency in Hz
extern uint32_t SystemPeripheralClock;

# if si32McuOption_map_vectors_to_ram
void (*si32_nvic_table[SIM3U1XX_MCU_NVIC_COUNT])(void);
# define si32McuOption_vector_ram_base si32_nvic_table

# endif

#endif //__GCPU_H__
//---------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//---------------------------------------------------------------------------

//==========================================================================
// WARNING:
//
// This file is auto-generated by AppBuilder and should not be modified.
// Any hand modifications will be lost if the project is regenerated.
//==========================================================================

#include "gFLASHCTRL0.h"
#include "gCPU.h"

// Include peripheral access modules used in this file
#include <SI32_FLASHCTRL_A_Type.h>
#include <si32_device.h>
```

```
//=============================================================================
// Configuration Functions
//=============================================================================
void FLASHCTRL0_enter_default_mode_from_reset(void)
{
   // No peripheral code is needed for this mode transition
}


//-----------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//-----------------------------------------------------------------------------


//=============================================================================
// WARNING:
//
// This file is auto-generated by AppBuilder and should not be modified.
// Any hand modifications will be lost if the project is regenerated.
//=============================================================================

#ifndef __GFLASHCTRL0_H__
#define __GFLASHCTRL0_H__


void FLASHCTRL0_enter_default_mode_from_reset(void);


#endif //__GFLASHCTRL0_H__
//-----------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//-----------------------------------------------------------------------------


//=============================================================================
// WARNING:
//
// This file is auto-generated by AppBuilder and should not be modified.
// Any hand modifications will be lost if the project is regenerated.
//=============================================================================

#include "gIDAC0.h"
#include "gCPU.h"

// Include peripheral access modules used in this file
#include <SI32_IDAC_A_Type.h>
#include <si32_device.h>


//=============================================================================
// Configuration Functions
//=============================================================================
```

```
void IDAC0_enter_default_mode_from_reset(void)
{
    SI32_IDAC_A_set_external_trigger_channel(SI32_IDAC_0,
SI32_IDAC_A_CONTROL_ETRIG_DACNT7_VALUE);
    SI32_IDAC_A_enable_module(SI32_IDAC_0);
    SI32_IDAC_A_select_output_fullscale_1ma(SI32_IDAC_0);
    SI32_IDAC_A_disable_trigger(SI32_IDAC_0);
}


//-------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//-------------------------------------------------------------------------


//=========================================================================
// WARNING:
//
// This file is auto-generated by AppBuilder and should not be modified.
// Any hand modifications will be lost if the project is regenerated.
//=========================================================================

#ifndef __GIDAC0_H__
#define __GIDAC0_H__

void IDAC0_enter_default_mode_from_reset(void);


#endif //__GIDAC0_H__
//-------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//-------------------------------------------------------------------------


//=========================================================================
// WARNING:
//
// This file is auto-generated by AppBuilder and should not be modified.
// Any hand modifications will be lost if the project is regenerated.
//=========================================================================

#include "gCLKCTRL.h"
#include "gCMP0.h"
#include "gCPU.h"
#include "gFLASHCTRL0.h"
#include "gIDAC0.h"
#include "gPB.h"
#include "gPCA0.h"
#include "gSARADC1.h"
#include "gTIMER0.h"
```

```
#include "gUART0.h"
#include "gUART1.h"
#include "gVREF0.h"
#include "gVREG0.h"
#include "gWDTIMER0.h"

#include <si32_device.h>

void enter_default_mode_from_reset(void)
{
    // Setup clock gates
    CLKCTRL_setup_default_mode_clock_gates();

    // Init WDTIMER
    WDTIMER0_enter_default_mode_from_reset();

    // Setup ports
    pb_enter_default_mode_from_reset();

    // Initialize clock control
    SystemCoreClock = 20000000;
    cpu_update();
    CLKCTRL_enter_default_mode_from_reset();

    // Initialize peripherals
    cpu_update();
    UART0_enter_default_mode_from_reset();
    UART1_enter_default_mode_from_reset();
    FLASHCTRL0_enter_default_mode_from_reset();
    VREG0_enter_default_mode_from_reset();
    VREF0_enter_default_mode_from_reset();
    TIMER0_enter_default_mode_from_reset();
    CMP0_enter_default_mode_from_reset();
    IDAC0_enter_default_mode_from_reset();
    PCA0_enter_default_mode_from_reset();
    SARADC1_enter_default_mode_from_reset();
}


//-------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//-------------------------------------------------------------------------


//=========================================================================
// WARNING:
//
// This file is auto-generated by AppBuilder and should not be modified.
// Any hand modifications will be lost if the project is regenerated.
//=========================================================================

#ifndef __GMODES_H__
#define __GMODES_H__

// Default Mode initialization
```

```
void enter_default_mode_from_reset(void);

#endif //__GMODES_H__
//----------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//----------------------------------------------------------------------


//======================================================================
// WARNING:
//
// This file is auto-generated by AppBuilder and should not be modified.
// Any hand modifications will be lost if the project is regenerated.
//======================================================================

#include "gPB.h"

// Include peripheral access modules used in this file
#include <SI32_PBCFG_A_Type.h>
#include <si32_device.h>
#include <SI32_PBSTD_A_Type.h>
#include <SI32_PBHD_A_Type.h>


void pb_enter_default_mode_from_reset(void)
{
    // PB0 Setup
    SI32_PBSTD_A_set_pins_analog(SI32_PBSTD_0, 0x3018);
    SI32_PBSTD_A_set_pins_push_pull_output(SI32_PBSTD_0, 0x0843);
    SI32_PBSTD_A_write_pbskipen(SI32_PBSTD_0, 0x303C);
    SI32_PBSTD_A_write_pins_low(SI32_PBSTD_0, 0x0800);

    // PB1 Setup
    SI32_PBSTD_A_set_pins_analog(SI32_PBSTD_1, 0x000E);
    SI32_PBSTD_A_write_pbskipen(SI32_PBSTD_1, 0xFFFE);

    // Enable Crossbar0 signals & set properties
    SI32_PBCFG_A_enable_xbar0_signal(SI32_PBCFG_0, SI32_XBAR0_PCA0_CEX0);
    SI32_PBCFG_A_enable_xbar0l_peripherals(SI32_PBCFG_0,
SI32_PBCFG_A_XBAR0L_CMP0AEN);
    SI32_PBCFG_A_enable_xbar0h_peripherals(SI32_PBCFG_0,
SI32_PBCFG_A_XBAR0H_UART1EN);
    SI32_PBCFG_A_enable_crossbar_0(SI32_PBCFG_0);

    // PB2 Setup
    SI32_PBSTD_A_write_pbskipen(SI32_PBSTD_2, 0x7FFF);

    // PB3 Setup
    SI32_PBSTD_A_set_pins_digital_input(SI32_PBSTD_3, 0x0008);
    SI32_PBSTD_A_set_pins_push_pull_output(SI32_PBSTD_3, 0x0001);
    SI32_PBSTD_A_write_pbskipen(SI32_PBSTD_3, 0x0FF0);

    // Enable Crossbar1 signals & set properties
    SI32_PBCFG_A_enable_xbar1_peripherals(SI32_PBCFG_0,
```

```
SI32_PBCFG_A_XBAR1_UART0EN);
        SI32_PBCFG_A_enable_crossbar_1(SI32_PBCFG_0);


        // PB4 Setup
        SI32_PBCFG_A_unlock_ports(SI32_PBCFG_0);
        SI32_PBHD_A_write_pblock(SI32_PBHD_4, 0x0000);
        SI32_PBHD_A_select_nchannel_current_limit(SI32_PBHD_4, 6);
        SI32_PBHD_A_select_pchannel_current_limit(SI32_PBHD_4, 6);
        SI32_PBHD_A_enable_pullup_resistors(SI32_PBHD_4);
        SI32_PBHD_A_select_slew_rate(SI32_PBHD_4, SI32_PBHD_A_SLEW_SLOWEST);
        SI32_PBHD_A_enable_bias(SI32_PBHD_4);
        SI32_PBHD_A_enable_drivers(SI32_PBHD_4);
        SI32_PBHD_A_set_pins_push_pull_output(SI32_PBHD_4, 0x0003);
        SI32_PBHD_A_write_pins_low(SI32_PBHD_4, 0x0003);
        SI32_PBHD_A_enable_n_channel_drivers(SI32_PBHD_4, 0x000B);
        SI32_PBHD_A_enable_p_channel_drivers(SI32_PBHD_4, 0x0003);
        SI32_PBHD_A_enable_pin_current_limit(SI32_PBHD_4, 0x0003);
        SI32_PBHD_A_select_pin3_safe_state(SI32_PBHD_4,
SI32_PBHD_A_SAFE_STATE_HIGH);
    }


    //----------------------------------------------------------------------
    // Copyright (c) 2012 by Silicon Laboratories
    // All rights reserved. This program and the accompanying materials
    // are made available under the terms of the Silicon Laboratories End User
    // License Agreement which accompanies this distribution, and is available at
    // http://developer.silabs.com/legal/version/v10/License_Agreement_v10.htm
    //
    //
    // Original content and implementation provided by Silicon Laboratories
    //----------------------------------------------------------------------


    //======================================================================
    // WARNING:
    //
    // This file is auto-generated by AppBuilder and should not be modified.
    // Any hand modifications will be lost if the project is regenerated.
    //======================================================================

    #ifndef __GPB_H__
    #define __GPB_H__

    void pb_enter_default_mode_from_reset(void);

    #endif //__GPB_H__
    //----------------------------------------------------------------------
    // Copyright (c) 2012 by Silicon Laboratories
    // All rights reserved. This program and the accompanying materials
    // are made available under the terms of the Silicon Laboratories End User
    // License Agreement which accompanies this distribution, and is available at
    // http://developer.silabs.com/legal/version/v10/License_Agreement_v10.htm
    //
    //
    // Original content and implementation provided by Silicon Laboratories
    //----------------------------------------------------------------------


    //======================================================================
    // WARNING:
    //
    // This file is auto-generated by AppBuilder and should not be modified.
```

```
// Any hand modifications will be lost if the project is regenerated.
//==============================================================================

#include "gPCA0.h"
#include "gCPU.h"

// Include peripheral access modules used in this file
#include <SI32_PCA_A_Type.h>
#include <si32_device.h>
#include <SI32_PCACH_A_Type.h>

//==============================================================================
// Configuration Functions
//==============================================================================
void PCA0_enter_default_mode_from_reset(void)
{
.
SI32_PCACH_A_select_operating_mode_high_frequency_square_wave(SI32_PCA_0_CH0);
     SI32_PCA_A_write_limit(SI32_PCA_0, 99);
}

//----------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//----------------------------------------------------------------------------


//==============================================================================
// WARNING:
//
// This file is auto-generated by AppBuilder and should not be modified.
// Any hand modifications will be lost if the project is regenerated.
//==============================================================================

#ifndef __GPCA0_H__
#define __GPCA0_H__

void PCA0_enter_default_mode_from_reset(void);


#endif //__GPCA0_H__
//----------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//----------------------------------------------------------------------------


//==============================================================================
// WARNING:
//
// This file is auto-generated by AppBuilder and should not be modified.
```

```
// Any hand modifications will be lost if the project is regenerated.
//=============================================================================

#include "gSARADC1.h"
#include "gCPU.h"

// Include peripheral access modules used in this file
#include <SI32_SARADC_A_Type.h>
#include <si32_device.h>

//=============================================================================
// 2nd Level Interrupt Handlers
//=============================================================================
extern void SARADC1_conv_complete_handler(void);

//=============================================================================
// 1st Level Interrupt Handlers
//=============================================================================
void SARADC1_IRQHandler()
{
   if
(SI32_SARADC_A_is_single_conversion_complete_interrupt_pending(SI32_SARADC_1)
      &&
SI32_SARADC_A_is_single_conversion_complete_interrupt_enabled(SI32_SARADC_1))
      {
         SARADC1_conv_complete_handler();
      }
   }

//=============================================================================
// Configuration Functions
//=============================================================================
void SARADC1_enter_default_mode_from_reset(void)
   {
      SI32_SARADC_A_select_timeslot0_channel(SI32_SARADC_1, 4);
      SI32_SARADC_A_select_timeslot1_channel(SI32_SARADC_1, 4);
      SI32_SARADC_A_select_timeslot2_channel(SI32_SARADC_1, 4);
      SI32_SARADC_A_select_timeslot3_channel(SI32_SARADC_1, 4);
      SI32_SARADC_A_select_timeslot4_channel(SI32_SARADC_1, 4);
      SI32_SARADC_A_select_timeslot5_channel(SI32_SARADC_1, 4);
      SI32_SARADC_A_select_timeslot6_channel(SI32_SARADC_1, 4);
      SI32_SARADC_A_select_timeslot7_channel(SI32_SARADC_1, 4);
      SI32_SARADC_A_select_output_packing_mode_lower_halfword_only(SI32_SARADC_1);
      SI32_SARADC_A_select_burst_mode_clock_apb_clock(SI32_SARADC_1);
      SI32_SARADC_A_select_burst_mode_track_time(SI32_SARADC_1, 54);
      SI32_SARADC_A_select_bias_power(SI32_SARADC_1,
SI32_SARADC_A_BIAS_POWER_HIGH);
      SI32_SARADC_A_select_vref_external(SI32_SARADC_1);

SI32_SARADC_A_select_channel_characteristic0_burst_mode_repeat_count(SI32_SARADC
_1, SI32_SARADC_A_BURST_MODE_REPEAT_COUNT_SAMPLE_8_TIMES);
      SI32_SARADC_A_select_channel_characteristic0_12bit_mode(SI32_SARADC_1);
      SI32_SARADC_A_enable_module(SI32_SARADC_1);
      SI32_SARADC_A_enable_burst_mode(SI32_SARADC_1);

SI32_SARADC_A_enable_single_conversion_complete_interrupt(SI32_SARADC_1);
      NVIC_ClearPendingIRQ(SARADC1_IRQn);
      NVIC_EnableIRQ(SARADC1_IRQn);
   }

//-----------------------------------------------------------------------------
```

```
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//------------------------------------------------------------------------


//=============================================================================
// WARNING:
//
// This file is auto-generated by AppBuilder and should not be modified.
// Any hand modifications will be lost if the project is regenerated.
//=============================================================================

#ifndef __GSARADC1_H__
#define __GSARADC1_H__

void SARADC1_IRQHandler(void);
void SARADC1_enter_default_mode_from_reset(void);


#endif //__GSARADC1_H__
//------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//------------------------------------------------------------------------


//=============================================================================
// WARNING:
//
// This file is auto-generated by AppBuilder and should not be modified.
// Any hand modifications will be lost if the project is regenerated.
//=============================================================================

#include "gTIMER0.h"
#include "gCPU.h"

// Include peripheral access modules used in this file
#include <SI32_TIMER_A_Type.h>
#include <si32_device.h>

//=============================================================================
// Configuration Functions
//=============================================================================
void TIMER0_enter_default_mode_from_reset(void)
{
    SI32_TIMER_A_set_clock_divider_counter(SI32_TIMER_0, 236);
    SI32_TIMER_A_set_clock_divider_reload(SI32_TIMER_0, 236);
    SI32_TIMER_A_start_high_timer(SI32_TIMER_0);
}

//------------------------------------------------------------------------
```

```
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//-----------------------------------------------------------------------

//=======================================================================
// WARNING:
//
// This file is auto-generated by AppBuilder and should not be modified.
// Any hand modifications will be lost if the project is regenerated.
//=======================================================================

#ifndef __GTIMER0_H__
#define __GTIMER0_H__

void TIMER0_enter_default_mode_from_reset(void);


#endif //__GTIMER0_H__
//-----------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//-----------------------------------------------------------------------

//=======================================================================
// WARNING:
//
// This file is auto-generated by AppBuilder and should not be modified.
// Any hand modifications will be lost if the project is regenerated.
//=======================================================================

#include "gUART0.h"
#include "gCPU.h"

// Include peripheral access modules used in this file
#include <SI32_UART_A_Type.h>
#include <si32_device.h>

//=======================================================================
// Configuration Functions
//=======================================================================
void UART0_enter_default_mode_from_reset(void)
{
    SI32_UART_A_set_rx_baudrate(SI32_UART_0, SystemPeripheralClock/2/115200 -
1);
    SI32_UART_A_set_tx_baudrate(SI32_UART_0, SystemPeripheralClock/2/115200 -
1);
    SI32_UART_A_enable_rx(SI32_UART_0);
    SI32_UART_A_enable_tx(SI32_UART_0);
}
```

```
//----------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//----------------------------------------------------------------------


//======================================================================
// WARNING:
//
// This file is auto-generated by AppBuilder and should not be modified.
// Any hand modifications will be lost if the project is regenerated.
//======================================================================

#ifndef __GUART0_H__
#define __GUART0_H__

void UART0_enter_default_mode_from_reset(void);


#endif //__GUART0_H__
//----------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//----------------------------------------------------------------------


//======================================================================
// WARNING:
//
// This file is auto-generated by AppBuilder and should not be modified.
// Any hand modifications will be lost if the project is regenerated.
//======================================================================

#include "gUART1.h"
#include "gCPU.h"

// Include peripheral access modules used in this file
#include <SI32_UART_A_Type.h>
#include <si32_device.h>

//======================================================================
// Configuration Functions
//======================================================================
void UART1_enter_default_mode_from_reset(void)
{
  SI32_UART_A_set_rx_baudrate(SI32_UART_1, SystemPeripheralClock/16/1200 - 1);
  SI32_UART_A_set_tx_baudrate(SI32_UART_1, SystemPeripheralClock/16/1200 - 1);
  SI32_UART_A_enable_rx_irda_mode(SI32_UART_1);
  SI32_UART_A_enable_tx_irda_mode(SI32_UART_1);
  SI32_UART_A_select_tx_irda_pulse_width(SI32_UART_1,
```

```
SI32_UART_A_IRDA_PULSE_WIDTH_1_4TH);
        SI32_UART_A_enter_half_duplex_mode(SI32_UART_1);
        SI32_UART_A_enable_rx(SI32_UART_1);
        SI32_UART_A_enable_tx(SI32_UART_1);
}

//-------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//-------------------------------------------------------------------

//===================================================================
// WARNING:
//
// This file is auto-generated by AppBuilder and should not be modified.
// Any hand modifications will be lost if the project is regenerated.
//===================================================================

#ifndef __GUART1_H__
#define __GUART1_H__

void UART1_enter_default_mode_from_reset(void);


#endif //__GUART1_H__
//-------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//-------------------------------------------------------------------

//===================================================================
// WARNING:
//
// This file is auto-generated by AppBuilder and should not be modified.
// Any hand modifications will be lost if the project is regenerated.
//===================================================================

#include "gVREF0.h"
#include "gCPU.h"

// Include peripheral access modules used in this file
#include <SI32_VREF_A_Type.h>
#include <si32_device.h>

//===================================================================
// Configuration Functions
//===================================================================
void VREF0_enter_default_mode_from_reset(void)
{
```

```
   SI32_VREF_A_select_2p4_volts(SI32_VREF_0);
}

//-----------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//-----------------------------------------------------------------------


//=======================================================================
// WARNING:
//
// This file is auto-generated by AppBuilder and should not be modified.
// Any hand modifications will be lost if the project is regenerated.
//=======================================================================

#ifndef __GVREF0_H__
#define __GVREF0_H__

void VREF0_enter_default_mode_from_reset(void);


#endif //__GVREF0_H__
//-----------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//-----------------------------------------------------------------------


//=======================================================================
// WARNING:
//
// This file is auto-generated by AppBuilder and should not be modified.
// Any hand modifications will be lost if the project is regenerated.
//=======================================================================

#include "gVREG0.h"
#include "gCPU.h"

// Include peripheral access modules used in this file
#include <SI32_VREG_A_Type.h>
#include <si32_device.h>


//=======================================================================
// 2nd Level Interrupt Handlers
//=======================================================================
extern void VREG0_vbus_invalid_handler(void);

//=======================================================================
// 1st Level Interrupt Handlers
//=======================================================================
```

```
void VBUSINVALID_IRQHandler()
{
   if (SI32_VREG_A_is_vbus_invalid_interrupt_pending(SI32_VREG_0)
      && SI32_VREG_A_is_vbus_invalid_interrupt_enabled(SI32_VREG_0))
   {
     VREG0_vbus_invalid_handler();
   }
}

//===============================================================================
// Configuration Functions
//===============================================================================
void VREG0_enter_default_mode_from_reset(void)
{
   // No peripheral code is needed for this mode transition
}


//-------------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//-------------------------------------------------------------------------------


//===============================================================================
// WARNING:
//
// This file is auto-generated by AppBuilder and should not be modified.
// Any hand modifications will be lost if the project is regenerated.
//===============================================================================

#ifndef __GVREG0_H__
#define __GVREG0_H__

void VBUSINVALID_IRQHandler(void);
void VREG0_enter_default_mode_from_reset(void);


#endif //__GVREG0_H__
//-------------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//-------------------------------------------------------------------------------


//===============================================================================
// WARNING:
//
// This file is auto-generated by AppBuilder and should not be modified.
// Any hand modifications will be lost if the project is regenerated.
//===============================================================================
```

```
#include "gWDTIMER0.h"
#include "gCPU.h"

// Include peripheral access modules used in this file
#include <SI32_WDTIMER_A_Type.h>
#include <si32_device.h>

//===========================================================================
// Configuration Functions
//===========================================================================
void WDTIMER0_enter_default_mode_from_reset(void)
{
    SI32_WDTIMER_A_stop_counter(SI32_WDTIMER_0);
}

//---------------------------------------------------------------------------
// Copyright (c) 2012 by Silicon Laboratories
// All rights reserved. This program and the accompanying materials
// are made available under the terms of the Silicon Laboratories End User
// License Agreement which accompanies this distribution, and is available at
// http://developer.silabs.com/legal/version/v10/License_Agreement_v10.htm
//
//
// Original content and implementation provided by Silicon Laboratories
//---------------------------------------------------------------------------

//===========================================================================
// WARNING:
//
// This file is auto-generated by AppBuilder and should not be modified.
// Any hand modifications will be lost if the project is regenerated.
//===========================================================================

#ifndef __GWDTIMER0_H__
#define __GWDTIMER0_H__

void WDTIMER0_enter_default_mode_from_reset(void);


#endif //__GWDTIMER0_H__
// Copyright (c) 2013
```

Although specific features of the invention are shown in some drawings and not in others, this is for convenience only as each feature may be combined with any or all of the other features in accordance with the invention. The words "including", "comprising", "having", and "with" as used herein are to be interpreted broadly and comprehensively and are not limited to any physical interconnection. Moreover, any embodiments disclosed in the subject application are not to be taken as the only possible embodiments. Other embodiments will occur to those skilled in the art and are within the following claims.

Other embodiments will occur to those skilled in the art and are within the following claims.

**Claims**

1.  A flow rate sensor system (10) for measuring the flow rate of a bodily fluid, the system comprising:

    an encapsulated implant (12) including:

        a flow tube (22) having an inlet (24) and an outlet (26) configured to receive a flow of a bodily fluid (28), a heating element (32) externally coupled to the flow tube (22) configured to dissipate heat at a predetermined rate over a predetermined amount of time,

a single thermistor 34' externally and directly coupled to the heating element (32) configured to measure a temperature rise of the heating element (32) over the predetermined amount of time,

an implant microcontroller (35) coupled to the single thermistor 34' configured to determine the flow rate of the bodily fluid in the flow tube (22) from the measured temperature rise of the heating element (32) over the predetermined amount of time and from a curve fit applied to a stored set of previously obtained calibration measurements, and

an implant power and communication subsystem (50) coupled to the implant microcontroller (35) configured to wirelessly receive power and wirelessly transmit and receive data; and

an external device (14) including:

an external microcontroller (56), and

an external power and communication subsystem (58) coupled to the external microcontroller (56) configured to wirelessly deliver power to the implant power and communication subsystem (50) and transmit and receive data to and from the implant power and communication subsystem (50).

2. The system of claim 1 in which the heating element (32) includes a surface mount resistor, a coil (106) of electrically conductive wire, or a printed circuit heater (108).

3. The system of claim 1 further including a thermal insulator (70) configured to thermally isolate the heating element (32) and the single thermistor 34' from cooling paths other than the direct cooling path to the bodily fluid in the flow tube (22).

4. The system of claim 3 in which the thermal insulator (70) includes an insulation layer (70") over the heating element (32) and the single thermistor 34', or a sealed volume of air surrounding (70") the heating element (32) and the single thermistor 34'.

5. The system of claim 1 in which the flow through flow tube (22) is comprised of a thin wall of polymer material with low thermal conductivity configured to limit heat transfer along a length and a circumference of the tube (22) while maintaining heat transfer in a radial direction to the fluid.

6. The system of claim 1 in which the bodily fluid (28) includes one or more of: cerebrospinal fluid (CSF), bile, blood, and urine.

7. The system of claim 1 in which the encapsulated implant (12) is coupled to a shunt, tube, vessel or catheter implanted in a human body or an animal.

8. The system of claim 7 in which the shunt includes one or more of: a ventriculo-peritoneal (VP) shunt (18), ventroarterial shunt, and lumboperitoneal shunt.

9. The system of claim 8 in which the encapsulated implant is coupled to a distal catheter (20) of the shunt (18), or to a proximal catheter (16) of the shunt.

10. The system of claim 1 in which the external power and communication subsystem (58) includes an external coil (90) coupled to the external microcontroller (56) and the implant power and communication subsystem (50) includes an implant coil (52) coupled to the microcontroller (35).

11. The system of claim 10 in which the implant coil (52) is integrated with the encapsulated implant (12).

12. The system of claim 10 in which the external power and communication subsystem (58) includes a resonant circuit comprised of the external coil (90) and a capacitor (74), and a source of low-level voltage pulses, the external device resonant circuit configured to provide sinusoidal current in the external coil of sufficient amplitude to induce sufficient sinusoidal voltage in the implant coil.

13. The system of claim 12 in which the implant power and communication subsystem (50) includes an implant resonant circuit comprised of the implant coil (52) and a capacitor (202) having a resonance frequency closely matched to the resonance frequency of the external resonant circuit to maintain sufficient AC voltage amplitude to power the implant power and communication subsystem (50) and to enable communication between the external power and communication subsystem (58) and implant power and communication subsystem (50).

14. The system of claim 13 in which the implant power and communication subsystem (50) is configured to convert induced sinusoidal voltages in the implant coil (52) to a highly regulated DC voltage over the range of loading conditions to power the heating element (32), the temperature sensor (34), the microcontroller (35), and components of the implant power and communication subsystem (50).

15. The system of claim 13 in which the external power communication subsystem (58) is configured to enable the external microcontroller (56) to communicate data to the implant power and communication subsystem (50) by changing the voltage supplied to the resonant circuit of the external power and communication subsystem (58) to modulate the amplitude of the voltage induced in the implant coil (52) and use that change in voltage to represent different binary states.

16. The system of claim 15 in which the implant power and communication subsystem (50) transmits binary values serially to the external power and communication subsystem (58) by sequentially applying and removing an electrical load from the implant coil (52) to induce changes in voltage in the external coil (90) that are decoded into data by the external microcontroller (56).

17. The system of claim 16 in which the external power and communication subsystem (58) includes a sense resistor (98) configured to measure change in the amplitude of the current in external power and communication subsystem (58) resulting from changes in the induced voltage in the external coil (90).

18. The system of claim 17 in which the external microcontroller (56) is coupled to the series resistor (98) and is configured to decode changes in the current of the external power and communication subsystem (58) into data.

19. The system of claim 1 in which the implant microcontroller (35) is configured to store the set of previously obtained calibration measurements relating heating element (32) temperature rise to flow rate.

20. The system of claim 1 in which the implant microcontroller (35) is configured to determine the flow rate from the measured temperature rise when temperature of the heating element (32) is determined to be no longer rising to minimize the length of time needed to determine the flow rate, the amount of heat generated by the heating device (32), and the amount of heat delivered to a patient.

21. The system of claim 1 in which the implant microcontroller (35) is configured to store identification information associated with the encapsulated implant (12).

22. The system of claim 1 in which the implant microcontroller (35) is configured to use a mean value of a set of temperature rise samples obtained over the predetermined amount of time as the temperature rises to determine the flow rate of the bodily fluid in order to increase the signal to noise ratio.

23. The system of claim 1 in which the implant microcontroller (35) is configured to use a weighted average of a set of temperature rise samples obtained over the predetermined amount of time as the temperature rises to determine the flow rate of the bodily fluid in order to increase the signal to noise ratio.

24. The system of claim 1 in which the external device (14) includes a smart device including a flow sensor App and a tethered external coil.

25. The system of claim 1 in which the external device (14) includes a display (290) for displaying one or more of: the measured flow rate, the predetermined amount of time, induced voltage on the implant coil, and identification information associated with the encapsulated implant (12).

**Patentansprüche**

1. Durchflussratensensorsystem (10) zum Messen der Durchflussrate einer Körperflüssigkeit, wobei das System umfasst:
ein gekapseltes Implantat (12), aufweisend:

ein Durchflussrohr (22) mit einem Einlass (24) und einem Auslass (26), das dafür ausgelegt ist, einen Fluss einer Körperflüssigkeit (28) aufzunehmen,

ein Heizelement (32), das extern mit dem Durchflussrohr (22) verbunden ist und dafür ausgelegt ist, Wärme mit einer vorbestimmten Geschwindigkeit über eine vorbestimmte Zeitdauer abzuleiten,

einen einzelnen Thermistor 34', der extern und direkt mit dem Heizelement (32) verbunden ist und dafür ausgelegt ist, einen Temperaturanstieg des Heizelements (32) über die vorbestimmte Zeitdauer zu messen,

einen mit dem einzelnen Thermistor 34' verbundenen Implantat-Mikrocontroller (35), der dafür ausgelegt ist, die Durchflussrate der Körperflüssigkeit im Durchflussrohr (22) anhand des gemessenen Temperaturanstiegs des Heizelements (32) über die vorbestimmte Zeitdauer und anhand einer Kurvenanpassung, die auf einen gespeicherten Satz von zuvor erhaltenen Kalibriermessungen angewendet wird, zu bestimmen, und

ein Implantat-Stromversorgungs- und Kommunikationssubsystem (50), das mit dem Implantat-Mikrocontroller (35) verbunden ist und dafür ausgelegt ist, drahtlos Energie aufzunehmen und drahtlos Daten zu senden und zu empfangen; und

eine externe Vorrichtung (14), aufweisend:

einen externen Mikrocontroller (56), und

ein externes Stromversorgungs- und Kommunikationssubsystem (58), das mit dem externen Mikrocontroller (56) verbunden ist und dafür ausgelegt ist, dem Implantat-Stromversorgungs- und Kommunikationssubsystem (50) drahtlos Energie zuzuführen und Daten an das Implantat-Stromversorgungs- und Kommunikationssubsystem (50) zu übertragen bzw. von diesem zu empfangen.

2. System nach Anspruch 1, wobei das Heizelement (32) einen oberflächenmontierbaren Widerstand, eine Spule (106) aus elektrisch leitendem Draht oder eine gedruckte Heizerschaltung (108) aufweist.

3. System nach Anspruch 1, ferner einen Wärmeisolator (70) umfassend, der dafür ausgelegt ist, das Heizelement (32) und den einzelnen Thermistor 34' von anderen Kühlwegen als dem direkten Kühlweg zur Körperflüssigkeit im Durchflussrohr (22) thermisch zu isolieren.

4. System nach Anspruch 3, wobei der Wärmeisolator (70) eine Isolierschicht (70") über dem Heizelement (32) und dem einzelnen Thermistor 34' oder ein geschlossenes Luftvolumen (70"), das das Heizelement (32) und den einzelnen Thermistor 34' umgibt, beinhaltet.

5. System nach Anspruch 1, wobei das Durchflussrohr (22) aus einer dünnen Wand aus Polymermaterial mit niedriger Wärmeleitfähigkeit besteht, die dafür ausgelegt ist, die Wärmeübertragung entlang einer Länge und eines Umfangs des Rohres (22) zu begrenzen, während die Wärmeübertragung in radialer Richtung zur Flüssigkeit aufrechterhalten wird.

6. System nach Anspruch 1, wobei die Körperflüssigkeit (28) eines oder mehrere von Cerebrospinalflüssigkeit (CSF), Gallenflüssigkeit, Blut und Urin beinhaltet.

7. System nach Anspruch 1, wobei das gekapselte Implantat (12) mit einem Shunt, Schlauch, Gefäß oder Katheter verbunden ist, der oder das in einen menschlichen Körper oder ein Tier implantiert ist.

8. System nach Anspruch 7, wobei der Shunt einen oder mehrere der folgenden beinhaltet: einen ventrikuloperitonealen (VP) Shunt (18), einen ventro-atrialen Shunt und einen lumbo-peritonealen Shunt.

9. System nach Anspruch 8, wobei das gekapselte Implantat mit einem distalen Katheter (20) des Shunts (18) oder mit einem proximalen Katheter (16) des Shunts verbunden ist.

10. System nach Anspruch 1, wobei das externe Stromversorgungs- und Kommunikationssubsystem (58) eine externe Spule (90) aufweist, die mit dem externen Mikrocontroller (56) verbunden ist, und das Implantat-Stromversorgungs- und Kommunikationssubsystem (50) eine Implantat-Spule (52) aufweist, die mit dem Mikrocontroller (35) verbunden ist.

11. System nach Anspruch 10, wobei die Implantat-Spule (52) in das gekapselte Implantat (12) integriert ist.

12. System nach Anspruch 10, wobei das externe Stromversorgungs- und Kommunikationssubsystem (58) einen Resonanzkreis, bestehend aus der externen Spule (90) und einem Kondensator (74), und eine Quelle für Niederspannungsimpulse aufweist, wobei der Resonanzkreis der externen Vorrichtung dafür ausgelegt ist, in der externen Spule einen sinusförmigen Strom mit ausreichender Amplitude bereitzustellen, um in der Implantat-Spule eine ausreichende sinusförmige Spannung zu induzieren.

13. System nach Anspruch 12, wobei das Implantat-Stromversorgungs- und Kommunikationssubsystem (50) einen Implantat-Resonanzkreis aufweist, der aus der Implantat-Spule (52) und einem Kondensator (202) mit einer Resonanzfrequenz besteht, die eng an die Resonanzfrequenz des externen Resonanzkreises angepasst ist, um eine ausreichende Wechselspannungs (AC) - Amplitude aufrechtzuerhalten, um das Implantat-Stromversorgungs- und Kommunikationssubsystem (50) mit Strom zu versorgen und um eine Kommunikation zwischen dem externen Stromversorgungs- und Kommunikationssubsystem (58) und dem Implantat-Stromversorgungs- und Kommunikationssubsystem (50) zu ermöglichen.

14. System nach Anspruch 13, wobei das Implantat-Stromversorgungs- und Kommunikationssubsystem (50) dafür ausgelegt ist, induzierte sinusförmige Spannungen in der Implantat-Spule (52) in eine stark regulierte Gleichspannung (DC) über den Bereich der Belastungsbedingungen umzuwandeln, um das Heizelement (32), den Temperatursensor (34), den Mikrocontroller (35) und die Komponenten des Implantat-Stromversorgungs- und Kommunikationssubsystems (50) mit Strom zu versorgen.

15. System nach Anspruch 13, wobei das externe Stromversorgungs- und Kommunikationssubsystem (58) dafür ausgelegt ist, den externen Mikrocontroller (56) in die Lage zu versetzen, Daten an das Implantat-Stromversorgungs- und Kommunikationssubsystem (50) zu übertragen, indem die dem Schwingkreis des externen Stromversorgungs- und Kommunikationssubsystems (58) zugeführte Spannung geändert wird, um die Amplitude der in der Implantat-Spule (52) induzierten Spannung zu modulieren, und diese Spannungsänderung zu nutzen, um verschiedene binäre Zustände darzustellen.

16. System nach Anspruch 15, wobei das Implantat-Stromversorgungs- und Kommunikationssubsystem (50) binäre Werte seriell an das externe Stromversorgungs- und Kommunikationssubsystem (58) überträgt, indem es sequentiell eine elektrische Last an die Implantat-Spule (52) anlegt und von dieser entfernt, um Änderungen der Spannung in der externen Spule (90) zu induzieren, die von dem externen Mikrocontroller (56) in Daten decodiert werden.

17. System nach Anspruch 16, wobei das externe Stromversorgungs- und Kommunikationssubsystem (58) einen Messwiderstand (98) aufweist, der dafür ausgelegt ist, Änderungen der Amplitude des Stroms im externen Stromversorgungs- und Kommunikationssubsystem (58) zu messen, die sich aus Änderungen der induzierten Spannung in der externen Spule (90) ergeben.

18. System nach Anspruch 17, wobei der externe Mikrocontroller (56) mit dem Reihenwiderstand (98) verbunden ist und dafür ausgelegt ist, Änderungen des Stroms des externen Stromversorgungs- und Kommunikationssubsystems (58) in Daten zu decodieren.

19. System nach Anspruch 1, wobei der Implantat-Mikrocontroller (35) dafür ausgelegt ist, den Satz von zuvor erhaltenen Kalibriermessungen, die den Temperaturanstieg des Heizelements (32) mit der Durchflussrate in Beziehung setzen, zu speichern.

20. System nach Anspruch 1, wobei der Implantat-Mikrocontroller (35) dafür ausgelegt ist, die Durchflussrate anhand des gemessenen Temperaturanstiegs zu bestimmen, wenn bestimmt wird, dass die Temperatur des Heizelements (32) nicht mehr ansteigt, um die zum Bestimmen der Durchflussrate benötigte Zeitdauer, die von der Heizvorrichtung (32) erzeugte Wärmemenge und die an einen Patienten abgegebene Wärmemenge zu minimieren.

21. System nach Anspruch 1, wobei der Implantat-Mikrocontroller (35) dafür ausgelegt ist, Identifikationsinformationen, die mit dem gekapselten Implantat (12) verknüpft sind, zu speichern.

22. System nach Anspruch 1, wobei der Implantat-Mikrocontroller (35) dafür ausgelegt ist, einen Mittelwert eines Satzes von Temperaturanstiegsmessungen, die über die vorbestimmte Zeitdauer erhalten werden, in der die Temperatur ansteigt, zu nutzen, um die Durchflussrate der Körperflüssigkeit zu bestimmen, um das Signal-Rausch-Verhältnis zu erhöhen.

23. System nach Anspruch 1, wobei der Implantat-Mikrocontroller (35) dafür ausgelegt ist, einen gewichteten Mittelwert eines Satzes von Temperaturanstiegsmessungen, die über die vorbestimmte Zeitdauer erhalten werden, in der die Temperatur ansteigt, zu nutzen, um die Durchflussrate der Körperflüssigkeit zu bestimmen, um das Signal-Rausch-Verhältnis zu erhöhen.

24. System nach Anspruch 1, wobei die externe Vorrichtung (14) eine intelligente Vorrichtung mit einer Durchflusssensor-App und einer verbundenen externen Spule umfasst.

25. System nach Anspruch 1, wobei die externe Vorrichtung (14) eine Anzeige (290) zum Anzeigen eines oder mehrerer der folgenden aufweist: der gemessenen Durchflussrate, der vorbestimmten Zeitdauer, der in der Implantat-Spule induzierten Spannung und Identifizierungsinformationen, die mit dem gekapselten Implantat (12) verknüpft sind.

**Revendications**

1. Système de capteur de débit (10) destiné à mesurer le débit d'un fluide corporel, le système comprenant :
   un implant encapsulé (12) comprenant :

   un tube d'écoulement (22) ayant une entrée (24) et une sortie (26), configuré pour recevoir un écoulement d'un fluide corporel (28),
   un élément chauffant (32) couplé extérieurement au tube d'écoulement (22), configuré pour dissiper la chaleur à un rythme prédéterminé pendant une durée prédéterminée,
   une thermistance unique 34' couplée extérieurement et directement à l'élément chauffant (32) configurée pour mesurer une élévation de température de l'élément chauffant (32) pendant la durée prédéterminée,
   un microcontrôleur d'implant (35) couplé à la thermistance unique 34', configuré pour déterminer le débit du fluide corporel dans le tube d'écoulement (22) à partir de l'élévation de température mesurée de l'élément chauffant (32) pendant la durée prédéterminée, et d'un ajustement de courbe appliqué à un ensemble stocké de mesures d'étalonnage obtenues précédemment, et
   un sous-système d'alimentation et de communication d'implant (50) couplé au microcontrôleur d'implant (35) configuré pour recevoir sans fil de l'énergie et pour transmettre et recevoir sans fil des données ; et
   un dispositif externe (14) comprenant :

   un microcontrôleur externe (56), et
   un sous-système externe d'alimentation et de communication (58) couplé au microcontrôleur externe (56) configuré pour fournir sans fil l'alimentation au sous-système d'alimentation et de communication d'implant (50) et pour transmettre et recevoir des données vers et depuis le sous-système d'alimentation et de communication d'implant (50).

2. Système selon la revendication 1, l'élément chauffant (32) comprenant une résistance montée en surface, une bobine (106) de fil électriquement conducteur ou un dispositif de chauffage de circuit imprimé (108).

3. Système selon la revendication 1, comprenant en outre un isolant thermique (70) configuré pour isoler thermiquement l'élément chauffant (32) et la thermistance unique 34' des voies de refroidissement autres que la voie de refroidissement directe vers le fluide corporel dans le tube d'écoulement (22).

4. Système selon la revendication 3, l'isolant thermique (70) comprenant une couche d'isolation (70") sur l'élément chauffant (32) et la thermistance unique 34', ou un volume étanche d'air entourant (70") l'élément chauffant (32) et la thermistance unique 34'.

5. Système selon la revendication 1, le tube d'écoulement (22) étant constitué d'une paroi mince en matériau polymère à faible conductivité thermique, configuré pour limiter le transfert de chaleur sur une longueur et une circonférence du tube (22) tout en maintenant le transfert de chaleur dans une direction radiale vers le fluide.

6. Système selon la revendication 1, le fluide corporel (28) comprenant un ou plusieurs éléments parmi : du liquide céphalorachidien (LCR), de la bile, du sang et de l'urine.

7. Système selon la revendication 1, l'implant encapsulé (12) étant couplé à une dérivation, un tube, un vaisseau ou un cathéter implanté dans un corps humain ou un animal.

8. Système selon la revendication 7, la dérivation comprenant un ou plusieurs des éléments parmi : une dérivation ventriculo-péritonéale (VP) (18), une dérivation ventro-artérielle et une dérivation lombo-péritonéale.

9. Système selon la revendication 8, l'implant encapsulé étant couplé à un cathéter distal (20) de la dérivation (18), ou à un cathéter proximal (16) de la dérivation.

10. Système selon la revendication 1, le sous-système externe d'alimentation et de communication (58) comprenant une bobine externe (90) couplée au microcontrôleur externe (56) et le sous-système d'alimentation et de communication d'implant (50) comprenant une bobine d'implant (52) couplée au microcontrôleur (35).

11. Système selon la revendication 10, la bobine d'implant (52) étant intégrée à l'implant encapsulé (12).

12. Système selon la revendication 10, le sous-système externe d'alimentation et de communication (58) comprenant un circuit résonant constitué de la bobine externe (90) et d'un condensateur (74), et une source d'impulsions de tension de faible niveau, le circuit résonant du dispositif externe étant configuré pour fournir un courant sinusoïdal dans la bobine externe d'amplitude suffisante pour induire une tension sinusoïdale suffisante dans la bobine d'implant.

13. Système selon la revendication 12, le sous-système d'alimentation et de communication d'implant (50) comprenant un circuit résonant d'implant constitué de la bobine d'implant (52) et d'un condensateur (202) ayant une fréquence de résonance correspondant étroitement à la fréquence de résonance du circuit résonant externe pour maintenir une amplitude de tension alternative suffisante pour alimenter le sous-système d'alimentation et de communication d'implant (50) et pour permettre la communication entre le sous-système externe d'alimentation et de communication (58) et le sous-système d'alimentation et de communication d'implant (50).

14. Système selon la revendication 13, le sous-système d'alimentation et de communication d'implant (50) étant configuré pour convertir des tensions sinusoïdales induites dans la bobine d'implant (52) en une tension continue hautement régulée sur la plage de conditions de charge pour alimenter l'élément chauffant (32), le capteur de température (34), le microcontrôleur (35) et des composants du sous-système d'alimentation et de communication d'implant (50).

15. Système selon la revendication 13, le sous-système externe d'alimentation et de communication (58) étant configuré pour permettre au microcontrôleur externe (56) de communiquer des données au sous-système d'alimentation et de communication d'implant (50) en modifiant la tension fournie au circuit résonant du sous-système externe d'alimentation et de communication (58) pour moduler l'amplitude de la tension induite dans la bobine d'implant (52) et utiliser ce changement de tension pour représenter différents états binaires.

16. Système selon la revendication 15, le sous-système d'alimentation et de communication d'implant (50) transmettant des valeurs binaires en série au sous-système externe d'alimentation et de communication (58) en appliquant et en retirant séquentiellement une charge électrique de la bobine d'implant (52) pour induire des changements de tension dans la bobine externe (90) qui sont décodés en données par le microcontrôleur externe (56).

17. Système selon la revendication 16, le sous-système externe d'alimentation et de communication (58) comprenant une résistance de détection (98) configurée pour mesurer un changement d'amplitude du courant dans le sous-système externe d'alimentation et de communication (58) résultant de changements de tension induite dans la bobine externe (90).

18. Système selon la revendication 17, le microcontrôleur externe (56) étant couplé à la résistance en série (98) et étant configuré pour décoder les changements de courant du sous-système externe d'alimentation et de communication (58) en données.

19. Système selon la revendication 1, le microcontrôleur d'implant (35) étant configuré pour stocker l'ensemble de mesures d'étalonnage obtenues précédemment mettant en relation l'élévation de température de l'élément chauffant (32) et le débit.

20. Système selon la revendication 1, le microcontrôleur d'implant (35) étant configuré pour déterminer le débit à partir de l'élévation de température mesurée lorsque la température de l'élément chauffant (32) est déterminée comme n'augmentant plus afin de minimiser le temps nécessaire pour déterminer le débit, la quantité de chaleur générée par l'élément chauffant (32) et la quantité de chaleur administrée à un patient.

**21.** Système selon la revendication 1, le microcontrôleur d'implant (35) étant configuré pour stocker des informations d'identification associées à l'implant encapsulé (12).

**22.** Système selon la revendication 1, le microcontrôleur d'implant (35) étant configuré pour utiliser une valeur moyenne d'un ensemble d'échantillons d'élévation de température obtenus pendant la durée prédéterminée lorsque la température s'élève pour déterminer le débit du fluide corporel afin d'augmenter le rapport signal/bruit.

**23.** Système selon la revendication 1, le microcontrôleur d'implant (35) étant configuré pour utiliser une moyenne pondérée d'un ensemble d'échantillons d'élévation de température obtenus sur une période de temps prédéterminée lorsque la température s'élève pour déterminer le débit du fluide corporel afin d'augmenter le rapport signal/bruit.

**24.** Système selon la revendication 1, le dispositif externe (14) comprenant un dispositif intelligent comprenant un capteur d'écoulement App et une bobine externe attachée.

**25.** Système selon la revendication 1, le dispositif externe (14) comprenant un dispositif d'affichage (290) pour afficher un ou plusieurs des éléments parmi : le débit mesuré, la durée prédéterminée, la tension induite sur la bobine d'implant et les informations d'identification associées à l'implant encapsulé (12).

**FIG. 1**

*FIG. 2*

*FIG. 3*

**FIG. 4**

FIG. 5

Runtime

*FIG. 6*

Average Heater Temperature Rise (°C)

Syringe Pump Set Flowrate (ml/hr)

41

*FIG. 7*

EP 3 131 460 B1

*FIG. 8*

EP 3 131 460 B1

FIG. 9

FIG. 10

EP 3 131 460 B1

FIG. 11

EP 3 131 460 B1

102

34

**FIG. 12**

104

34

Thermocouple

Metal A

Metal B

Wiring to Signal
Conditioning
Circuitry

Measurement
Junction

Reference
Junction

**FIG. 13**

**FIG. 14**

**FIG. 15**

32 →

110

*FIG. 16*

112

32

112

112

112

*FIG. 17*

**FIG. 18**

**FIG. 19**

**FIG. 20**

**FIG. 21**

**FIG. 22**

EP 3 131 460 B1

**FIG. 23**

**FIG. 24**

FIG. 25

**EP 3 131 460 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090204019 A1, Giuggen